# EUROPEAN PATENT APPLICATION

(11) **EP 4 755 889 A1**
(43) Date of publication of application: **10.06.2026**
(21) Application number: 25816543.0
(22) Date of filing: 30.05.2025
(51) Int. Cl.: C07D 471/04, G01N 33/68, G01N 33/53

(54) **COMPOUND FOR DETECTING AMYLOID-BETA OLIGOMER AND METHOD FOR DIAGNOSING DEGENERATIVE BRAIN DISEASE USING SAME**

(30) Priority: 31.05.2024 KR 20240072019
(71) Applicant: Amyloid Solution Inc., Seongnam-si, Gyeonggi-do 13486 (KR); University-Industry Foundation, Yonsei University, Seoul 03722 (KR)
(72) Inventor: KIM, Youngsoo, Incheon 21983 (KR); KIM, Ikyon, Incheon 21983 (KR); CHO, Matthew Illhwan, Incheon 21983 (KR); LEE, Sunhee, Incheon 21983 (KR); CHANG, Hyekyung, Seongnam-si, Gyeonggi-do 13486 (KR); LEE, Saeyeon, Seongnam-si, Gyeonggi-do 13599 (KR); KIM, Hyeyun, Incheon 21983 (KR)
(74) Representative: Vossius & Partner Patentanwälte Rechtsanwälte mbB
(86) International application number: PCT/KR2025/007502
(87) International publication number: WO 2025/249975

(57) **Abstract**

Disclosed are a novel compound for detecting amyloid-beta, and a method of diagnosing a neurodegenerative disease using the same, wherein a compound represented by Formula 1 or a salt thereof according to the present disclosure may specifically bind to amyloid-beta. Due to its high selectivity, the compound or a salt thereof can detect amyloid-beta in plasma with high sensitivity despite interference in detection caused by the presence of other proteins in plasma, and can be utilized for the diagnosis of a neurodegenerative disease or screening for therapeutic agents for a neurodegenerative disease.

## Description

### Technical Field

The present disclosure relates to a novel compound for detecting amyloid-beta oligomers and a method of diagnosing neurodegenerative diseases using the same.

### Background Art

Alzheimer's disease (AD) is a progressive neurodegenerative disease characterized by the deposition of amyloid-beta (Aβ) peptides in the brain. Aβ peptides are produced when the amyloid precursor protein is cleaved by enzymes, and the generated peptides aggregate into oligomers and plaques in AD patients. Although there is no distinct correlation between Aβ plaque burden and cognitive dysfunction, Aβ oligomers are known to be involved in the symptomatic onset of AD. Furthermore, in the brains of AD patients, Aβ oligomers induce neurotoxicity, neuronal cell death, and synaptic dysfunction. Therefore, the detection of Aβ oligomers is essential for the development of AD therapeutics and diagnostics.

Considering that the major Aβ species in plasma are soluble Aβ oligomers, detecting plasma Aβ can be utilized to monitor changes in Aβ oligomer levels and predict the onset of AD. Moreover, blood Aβ testing in clinical trials is a non-invasive and cost-effective method for detecting Aβ oligomers, which can be a beneficial approach for AD patients. However, accurately measuring Aβ oligomer levels in the bloodstream has long been a challenge in terms of the accuracy and sensitivity of plasma Aβ detection. Controversy regarding accurate plasma Aβ measurement is known to stem from the heterogeneous and polymorphic properties of Aβ oligomers. Specifically, unlike Aβ plaques which possess a β-sheet-rich structure, Aβ oligomers are unstable intermediates with diverse shapes, structures, and compositions. The transient and polymorphic nature of Aβ oligomers causes masking of Aβ oligomer-targeting compounds and creates steric hindrance, resulting in low diagnostic accuracy and sensitivity. Consequently, there is an urgent need to develop new imaging agents to selectively target Aβ oligomers.

Accordingly, the present inventor intends to provide a compound that not only significantly improves detection efficiency through interaction with Aβ aggregates, but also maintains consistent detection efficiency without diminishment even for modified Aβ complex forms.

### Disclosure of Invention

### Technical Problem

One aspect is to provide a compound represented by Formula 1 or a salt thereof:

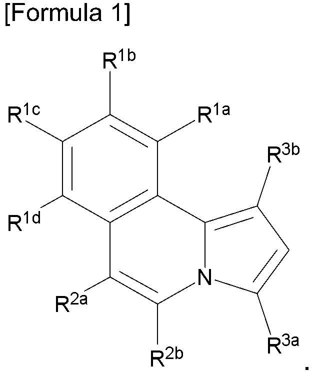

Another aspect is to provide a method of preparing the compound represented by Formula 1.

Another aspect is to provide a composition for detecting amyloid-beta (Aβ) including the compound represented by Formula 1 or a pharmaceutically acceptable salt thereof.

Another aspect is to provide a method of detecting amyloid-beta (Aβ), including contacting an isolated biological sample with the compound represented by Formula 1 or a pharmaceutically acceptable salt thereof.

Another aspect is to provide a composition for diagnosing a neurodegenerative disease, including the compound represented by Formula 1 or a pharmaceutically acceptable salt thereof.

Another aspect is to provide a method of providing information regarding the diagnosis of a neurodegenerative disease, the method including contacting an isolated biological sample with the compound represented by Formula 1 or a pharmaceutically acceptable salt thereof.

Another aspect is to provide a method of diagnosing a neurodegenerative disease, including administering the compound represented by Formula 1 or a pharmaceutically acceptable salt thereof to a biological sample.

### Solution to Problem

One aspect is to provide a compound represented by Formula 1 or a salt thereof:

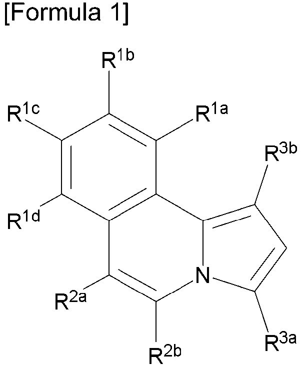

**In** Formula 1,
R^{1a} and R^{1b} are each independently hydrogen, deuterium, halogen, amino, hydroxy, substituted or unsubstituted C₁₋₁₀ alkyl, substituted or unsubstituted C₂₋₁₀ alkenyl, substituted or unsubstituted C₂₋₁₀ alkynyl, substituted or unsubstituted C₁₋₁₀ alkoxy, substituted or unsubstituted 6- to 10-membered aryl, or substituted or unsubstituted 5- to 10-membered heteroaryl;
R^{1c} and R^{1d} are each independently hydrogen, deuterium, halogen, amino, hydroxy, substituted or unsubstituted C₁₋₁₀ alkyl, substituted or unsubstituted C₂₋₁₀ alkenyl, substituted or unsubstituted C₂₋₁₀ alkynyl, substituted or unsubstituted C₁₋₁₀ alkoxy, substituted or unsubstituted 6- to 10-membered aryl, or substituted or unsubstituted 5- to 10-membered heteroaryl, or
R^{1c} and R^{1d} are connected to each other to form a compound represented by Formula 2;

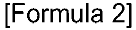

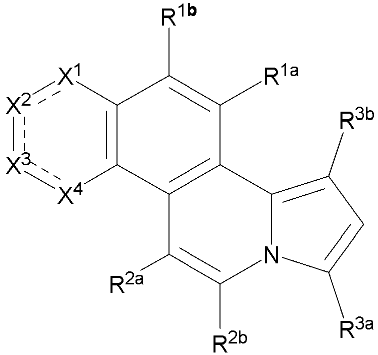

In Formula 2, X¹, X², X³, and X⁴ are each independently C(R⁴), nitrogen, oxygen, sulfur, or phosphorus;
R⁴ is hydrogen, deuterium, halogen, amino, hydroxy, substituted or unsubstituted C₁₋₁₀ alkyl, substituted or unsubstituted C₂₋₁₀ alkenyl, substituted or unsubstituted C₂₋₁₀ alkynyl, substituted or unsubstituted C₁₋₁₀ alkoxy, substituted or unsubstituted 6- to 10-membered aryl, or substituted or unsubstituted 5- to 10-membered heteroaryl;
R^{2a} and R^{2b} are each independently hydrogen, deuterium, halogen, amino, hydroxy, substituted or unsubstituted C₁₋₁₀ alkyl, substituted or unsubstituted C₂₋₁₀ alkenyl, substituted or unsubstituted C₂₋₁₀ alkynyl, substituted or unsubstituted C₁₋₁₀ alkoxy, substituted or unsubstituted 6- to 10-membered aryl, or substituted or unsubstituted 5- to 10-membered heteroaryl; and
R^{3a} and R^{3b} are each independently hydrogen, deuterium, halogen, amino, hydroxy, -CN, -C(O)NH₂, -C(O)NH₂, -C(O)O(C₁₋₁₀ alkyl), substituted or unsubstituted C₁₋₁₀ alkyl, substituted or unsubstituted 6- to 10-membered aryl, or substituted or unsubstituted 5- to 10-membered heteroaryl.

In an embodiment, R^{1a} and R^{1b} are each independently hydrogen, deuterium, or halogen;
R^{1c} and R^{1d} are each independently hydrogen, deuterium, halogen, amino, hydroxy, substituted or unsubstituted C₁₋₅ alkyl, substituted or unsubstituted C₂₋₅ alkenyl, substituted or unsubstituted C₂₋₅ alkynyl, substituted or unsubstituted C₁₋₅ alkoxy, substituted or unsubstituted 6-membered aryl, or substituted or unsubstituted 6-membered heteroaryl, or
R^{1c} and R^{1d} are connected to each other to form the compound represented by Formula 2, and
X¹, X², X³, and X⁴ are each independently C(R⁴), nitrogen, oxygen, sulfur, or phosphorus, and
R⁴ is hydrogen, deuterium, or halogen;
R^{2a} and R^{2b} are each independently hydrogen, deuterium, halogen, substituted or unsubstituted C₁₋₅ alkyl, substituted or unsubstituted C₂₋₅ alkenyl, substituted or unsubstituted C₂₋₅ alkynyl, substituted or unsubstituted 6- to 10-membered aryl, or substituted or unsubstituted 5- to 10-membered heteroaryl; and
R^{3a} and R^{3b} are each independently hydrogen, deuterium, halogen, amino, hydroxy, -CN, -C(O)NH₂, -C(O)NH₂, -C(O)O(C₁₋₅ alkyl), substituted or unsubstituted C₁₋₅ alkyl, or substituted or unsubstituted 6- to 10-membered aryl.

In another embodiment, R^{1a} and R^{1b} are each independently hydrogen or halogen;
R^{1c} and R^{1d} are each independently hydrogen, halogen, substituted or unsubstituted C₁₋₅ alkyl, substituted or unsubstituted C₁₋₅ alkoxy, or substituted or unsubstituted 6-membered aryl, or
R^{1c} and R^{1d} are connected to each other to form the compound represented by Formula 2, and
X¹, X², X³, and X⁴ are each independently C(R⁴), and
R⁴ is hydrogen;
R^{2a} and R^{2b} are each independently hydrogen, halogen, substituted or unsubstituted C₁₋₅ alkyl, substituted or unsubstituted 6- to 10-membered aryl, or substituted or unsubstituted 5- to 6-membered heteroaryl; and
R^{3a} and R^{3b} may be each independently hydrogen, -CN, -C(O)NH₂, - C(O)O(C₁₋₅ alkyl), substituted or unsubstituted C₁₋₅ alkyl, or substituted or unsubstituted 6-membered aryl.

The term "alkyl" refers to a functional group derived by removing one hydrogen atom from one carbon atom in a saturated hydrocarbon connected by single bonds between carbon atoms. The alkyl may be a linear or branched hydrocarbon containing 1 to 20 carbon atoms (C₁₋₂₀), 1 to 10 carbon atoms (C₁₋₁₀), or 1 to 5 carbon atoms (C₁₋₅). For example, the alkyl may be methyl, ethyl, n-propyl, i-propyl, n-butyl, i-butyl, t-butyl, and the like.

The term "Cₘ₋ₙ" (where m and n are each independently an integer of 1 or more) means having m to n carbon atoms.

The term "alkenyl" refers to a functional group derived by removing one hydrogen atom from one carbon atom in an unsaturated hydrocarbon containing one or more double bonds between carbon atoms. The alkenyl may be a linear or branched hydrocarbon containing 2 to 20 carbon atoms (C₂₋₂₀), 2 to 10 carbon atoms (C₂₋₁₀), or 2 to 5 carbon atoms (C₂₋₅). For example, the alkenyl may be vinyl, propenyl, butenyl, and the like.

The term "alkynyl" refers to a functional group derived by removing one hydrogen atom from one carbon atom in an unsaturated hydrocarbon containing one or more triple bonds between carbon atoms. The alkynyl may be a linear or branched hydrocarbon containing 2 to 20 carbon atoms (C₂₋₂₀), 2 to 10 carbon atoms (C₂₋₁₀), or 2 to 5 carbon atoms (C₂₋₅). For example, the alkynyl may be ethynyl, propynyl, butynyl, and the like.

The term "alkoxy" refers to an alkyl bonded to oxygen (-O-alkyl). The alkoxy may contain 2 to 20 carbon atoms (C₂₋₂₀), 2 to 10 carbon atoms (C₂₋₁₀), or 2 to 5 carbon atoms (C₂₋₅). For example, the alkoxy may be methoxy, ethoxy, propoxy, butoxy, and the like.

The term "aryl" refers to a functional group derived by removing one hydrogen atom from one carbon atom in a parent aromatic hydrocarbon. The aryl may be 5- to 18-membered, 5- to 14-membered, 5- to 10-membered, or 6- to 10-membered depending on the number of atoms constituting the ring. For example, the aryl may be phenyl, biphenyl, naphthalenyl, fluorenyl, indenyl, indanyl, and the like.

The term "heteroaryl" refers to aryl in which one or more carbon atoms constituting the aromatic ring are replaced by an atom selected from the group consisting of nitrogen, oxygen, and sulfur. The heteroaryl may be 5- to 18-membered, 5- to 14-membered, or 5- to 10-membered depending on the number of atoms constituting the ring. For example, the heteroaryl may be thiophenyl, pyrrolyl, pyrazolyl, pyridinyl, imidazolyl, triazolyl, oxazolyl, and the like.

The alkyl, alkenyl, alkynyl, alkoxy, aryl, or heteroaryl may each be substituted or unsubstituted. The term "substituted" refers to the replacement of one or more hydrogen atoms bonded to carbon with another element or functional group, and "unsubstituted" refers to the state where all hydrogen atoms bonded to carbon are not replaced with other atoms or functional groups. Examples of other atoms that may be substituted include fluorine, chlorine, bromine, or iodine; and examples of functional groups that may be substituted include hydroxyl, thiol, nitro, oxo, substituted or unsubstituted alkyl, substituted or unsubstituted alkenyl, substituted or unsubstituted alkynyl, substituted or unsubstituted cycloalkyl, substituted or unsubstituted heterocycloalkyl, substituted or unsubstituted alkoxy, substituted or unsubstituted cycloalkoxy, substituted or unsubstituted alkylthio, substituted or unsubstituted aryloxy, substituted or unsubstituted arylthio, substituted or unsubstituted heteroaryloxy, substituted or unsubstituted heteroarylthio, and the like.

For example, when the alkyl is substituted, it may be trifluoromethyl (-CF₃) or (2-(2-(2-fluoroethoxy)ethoxy)ethoxy)methyl; -CH₂OC₂H₄OC₂H₄OC₂H₅F), and the like.

For example, when the aryl is substituted, it may be methoxyphenyl, fluorophenyl, chlorophenyl, or tolyl, and the like.

In an embodiment, the compound represented by Formula 1 may be a compound represented by one chemical formula selected from the group consisting of Formulas 3 to 43: 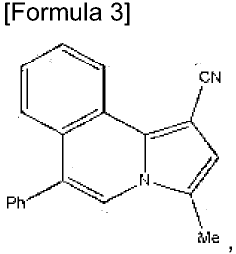 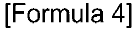 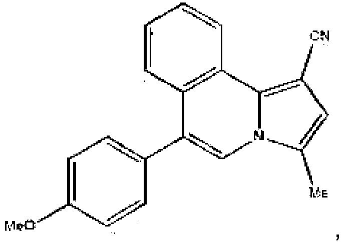 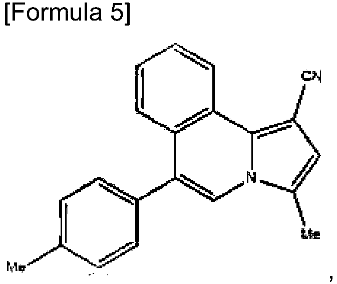 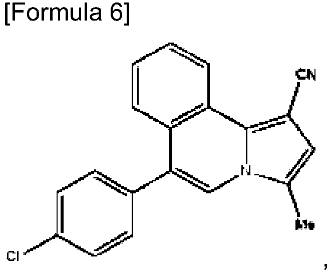 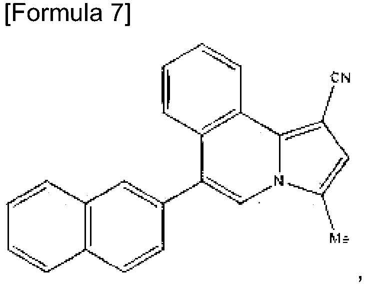 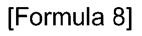 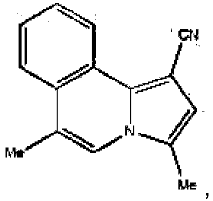 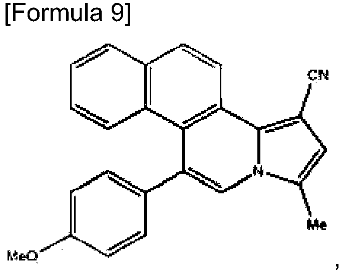 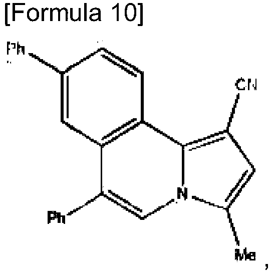 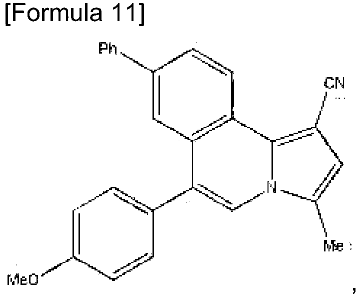 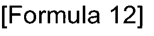 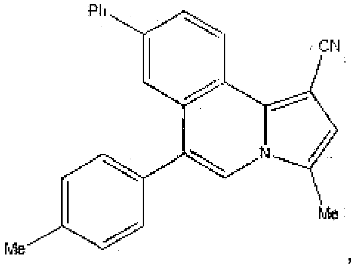 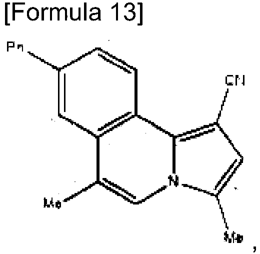 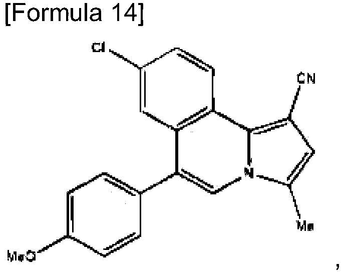 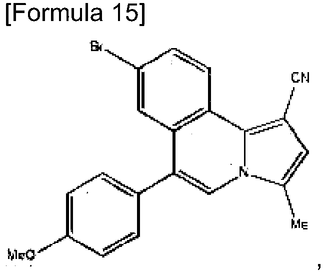 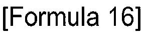 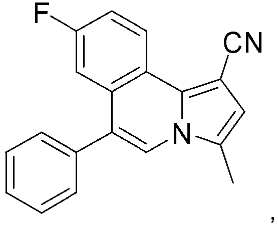 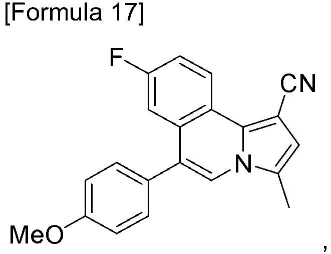 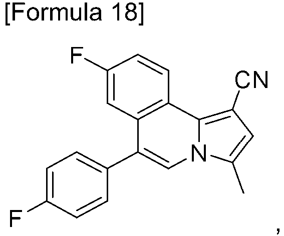 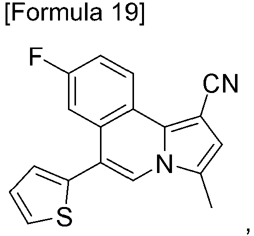 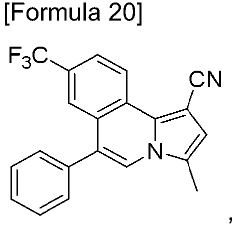 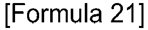 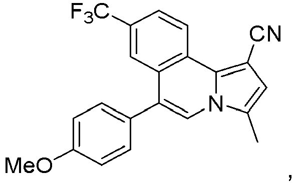 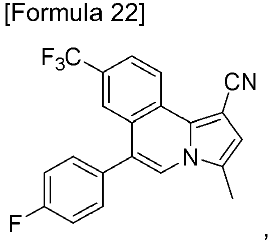 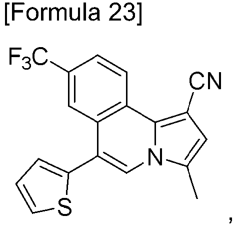 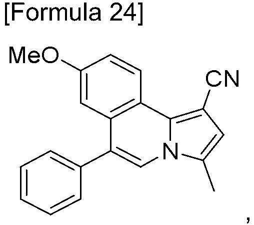 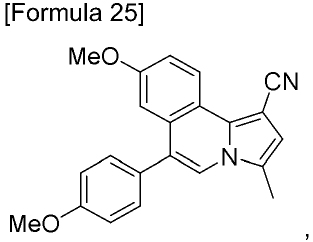 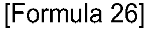 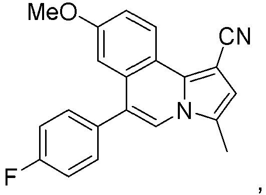 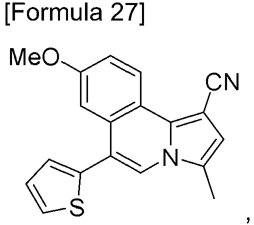 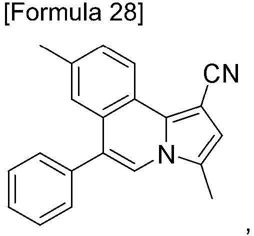 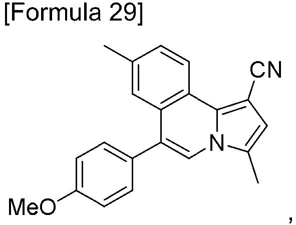 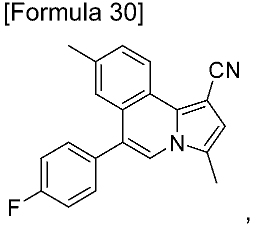 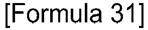 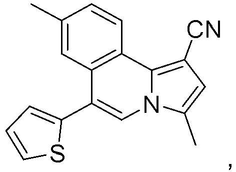 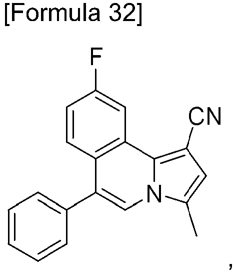 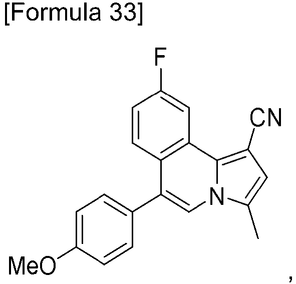 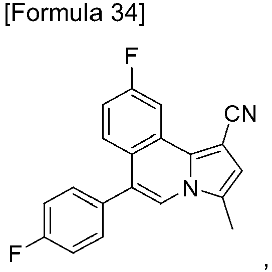 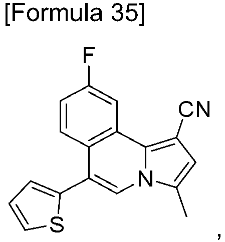 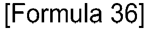 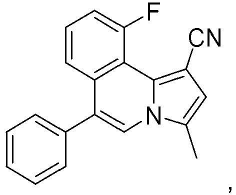 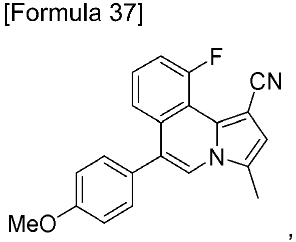 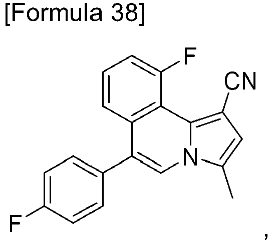 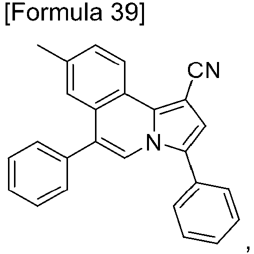 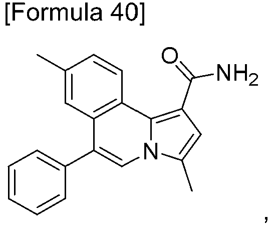 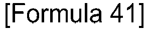 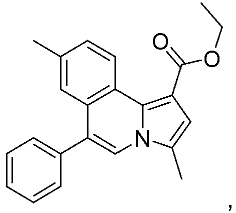 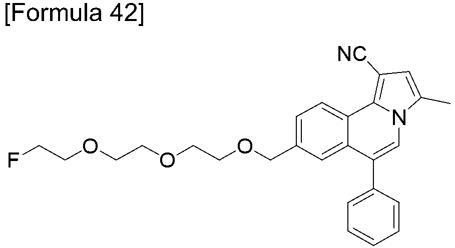 and

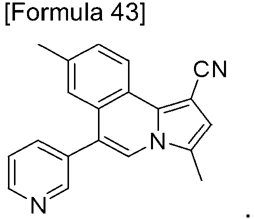

The salt may be an acid addition salt formed by a free acid. When used for the purpose of diagnosing a disease, the compound may be in the form of a pharmaceutically acceptable salt or a diagnostically acceptable salt. The expression "pharmaceutically or diagnostically acceptable salt" refers to any organic or inorganic addition salt of the base compound of the active substance, which is relatively non-toxic and harmless to a patient at a concentration having an effective action, and whose side effects caused by the salt do not degrade the beneficial efficacy of the base compound of the active substance. As the free acid forming the acid addition salt, inorganic acids and organic acids may be used. As the inorganic acid, hydrochloric acid, hydrobromic acid, nitric acid, sulfuric acid, perchloric acid, phosphoric acid, and the like may be used. As the organic acid, citric acid, acetic acid, lactic acid, maleic acid, fumaric acid, gluconic acid, methanesulfonic acid, glycolic acid, succinic acid, tartaric acid, galacturonic acid, embonic acid, glutamic acid, aspartic acid, oxalic acid, (D) or (L) malic acid, maleic acid, methanesulfonic acid, ethanesulfonic acid, 4-toluenesulfonic acid, salicylic acid, citric acid, benzoic acid, or malonic acid, and the like may be used. In addition, the salt includes alkali metal salts (sodium salt, potassium salt, etc.) and alkaline earth metal salts (calcium salt, magnesium salt, etc.). For example, the acid addition salt may include acetate, aspartate, benzoate, besylate, bicarbonate/carbonate, bisulfate/sulfate, borate, camsylate, citrate, edisylate, esylate, formate, fumarate, gluceptate, gluconate, glucuronate, hexafluorophosphate, hibenzate, hydrochloride/chloride, hydrobromide/bromide, hydroiodide/iodide, isethionate, lactate, malate, maleate, malonate, mesylate, methylsulfate, naphthylate, 2-napsylate, nicotinate, nitrate, orotate, oxalate, palmitate, pamoate, phosphate/hydrogen phosphate/dihydrogen phosphate, saccharate, stearate, succinate, tartrate, tosylate, trifluoroacetate, aluminum, arginine, benzathine, calcium, choline, diethylamine, diolamine, glycine, lysine, magnesium, meglumine, olamine, potassium, sodium, tromethamine, zinc salt, and the like, and specifically, may be hydrochloride or trifluoroacetate.

The acid addition salt may be prepared by conventional methods, for example, by dissolving the compound in an organic solvent, such as methanol, ethanol, acetone, methylene chloride, acetonitrile, and the like, adding an organic acid or inorganic acid, and filtering and drying the resulting precipitate; or by distilling the solvent and excess acid under reduced pressure and then drying; or by crystallizing in an organic solvent.

Additionally, a metal salt may be prepared using a base. The alkali metal or alkaline earth metal salt is obtained, for example, by dissolving the compound in an excess amount of alkali metal hydroxide or alkaline earth metal hydroxide solution, filtering the undissolved compound salts, and evaporating and drying the filtrate. In this case, it is pharmaceutically suitable to prepare sodium, potassium, or calcium salts as the metal salts. Furthermore, the corresponding silver salt is obtained by reacting the alkali metal or alkaline earth metal salt with a suitable silver salt (e.g., silver nitrate).

Furthermore, the compound represented by Formula 1 or a salt thereof may include all solvates, hydrates, isomers, and the like that can be prepared therefrom.

The term "isomer" refers to compounds having the same chemical formula or molecular formula but differing structurally or sterically. Examples of the isomers may include structural isomers such as tautomers, stereoisomers such as R or S isomers having an asymmetric carbon center, geometric isomers (trans, cis), and enantiomers.

Another aspect is to provide a method for preparing a compound represented by Formula 1 below, including, as shown in Reaction Scheme 1 below:
obtaining Compound 1003 by reacting Compound 1001 and Compound 1002 in an organic solvent (Step 1);
obtaining Compound 1004 by reacting Compound 1003 and NH₄OAc in an organic solvent (Step 2);
obtaining Compound 1006 by reacting Compound 1004 with Compound 1005-1, Compound 1005-2, or Compound 1005-3 in an organic solvent (Step 3); and
obtaining a compound represented by Formula 1 below (Compound 1007) by reacting Compound 1006 and a catalyst in an organic solvent (Step 4):

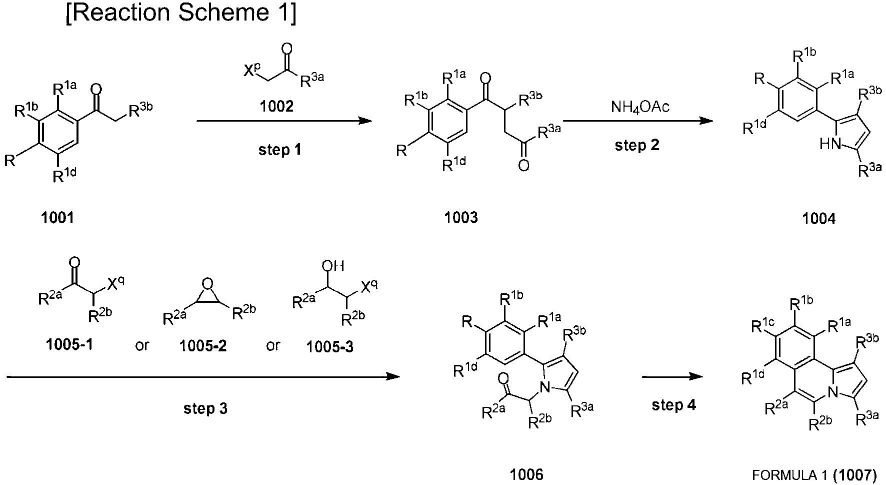

In Reaction Scheme 1,
R is R^{1c} or X^{q};
X^{p} and X^{q} are each independently hydrogen, deuterium, or halogen;
R^{1a} and R^{1b} are each independently hydrogen, deuterium, halogen, amino, hydroxy, substituted or unsubstituted C₁₋₁₀ alkyl, substituted or unsubstituted C₂₋₁₀ alkenyl, substituted or unsubstituted C₂₋₁₀ alkynyl, substituted or unsubstituted C₁₋₁₀ alkoxy, substituted or unsubstituted 6- to 10-membered aryl, or substituted or unsubstituted 5- to 10-membered heteroaryl;
R^{1c} and R^{1d} are each independently hydrogen, deuterium, halogen, amino, hydroxy, substituted or unsubstituted C₁₋₁₀ alkyl, substituted or unsubstituted C₂₋₁₀ alkenyl, substituted or unsubstituted C₂₋₁₀ alkynyl, substituted or unsubstituted C₁₋₁₀ alkoxy, substituted or unsubstituted 6- to 10-membered aryl, or substituted or unsubstituted 5- to 10-membered heteroaryl, or
R^{1c} and R^{1d} are connected to each other to form a compound represented by Formula 2;

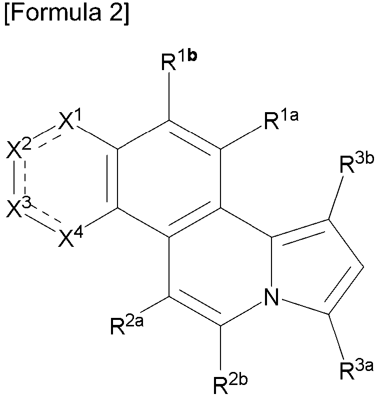

In Formula 2, X¹, X², X³, and X⁴ are each independently C(R⁴), nitrogen, oxygen, sulfur, or phosphorus;
R⁴ is hydrogen, deuterium, halogen, amino, hydroxy, substituted or unsubstituted C₁₋₁₀ alkyl, substituted or unsubstituted C₂₋₁₀ alkenyl, substituted or unsubstituted C₂₋₁₀ alkynyl, substituted or unsubstituted C₁₋₁₀ alkoxy, substituted or unsubstituted 6- to 10-membered aryl, or substituted or unsubstituted 5- to 10-membered heteroaryl;
R^{2a} and R^{2b} are each independently hydrogen, deuterium, halogen, amino, hydroxy, substituted or unsubstituted C₁₋₁₀ alkyl, substituted or unsubstituted C₂₋₁₀ alkenyl, substituted or unsubstituted C₂₋₁₀ alkynyl, substituted or unsubstituted C₁₋₁₀ alkoxy, substituted or unsubstituted 6- to 10-membered aryl, or substituted or unsubstituted 5- to 10-membered heteroaryl; and
R^{3a} and R^{3b} are each independently hydrogen, deuterium, halogen, amino, hydroxy, -CN, -C(O)NH₂, -C(O)NH₂, -C(O)O(C₁₋₁₀ alkyl), substituted or unsubstituted C₁₋₁₀ alkyl, substituted or unsubstituted 6- to 10-membered aryl, or substituted or unsubstituted 5- to 10-membered heteroaryl.

The terms "alkyl", "alkoxy", "aryl", "heteroaryl", "salt", and the like may be within the ranges described above.

In an embodiment, the preparation method may further include reacting the compound of Formula 1 with sulfuric acid after Step 4. For example, a reaction such as Reaction Scheme 2 may be performed:

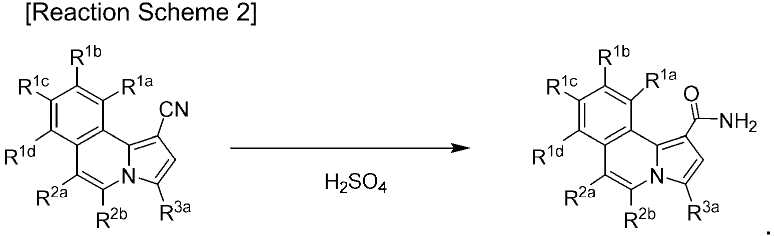

In an embodiment, when reacting Compound 1004 and Compound 1005-2 in an organic solvent in Step 3, the preparation method may further include reacting an intermediate generated after reacting Compound 1004 and Compound 1005-2 in an organic solvent but before obtaining Compound 1006 with an oxidizing agent. For example, a reaction such as Reaction Scheme 3 may be performed:

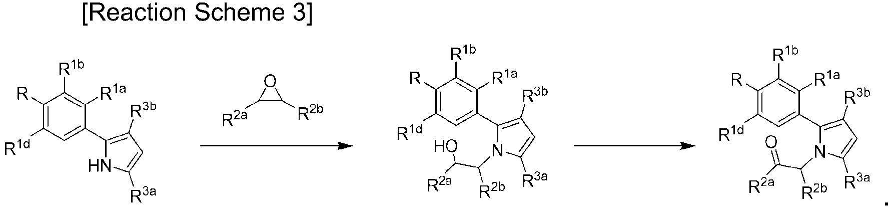

In an embodiment, when reacting Compound 1004 and Compound 1005-3 in an organic solvent in Step 3, the preparation method may further include reacting an intermediate generated after reacting Compound 1004 and Compound 1005-3 in an organic solvent but before obtaining Compound 1006 with an oxidizing agent. For example, a reaction such as Reaction Scheme 4 may be performed:

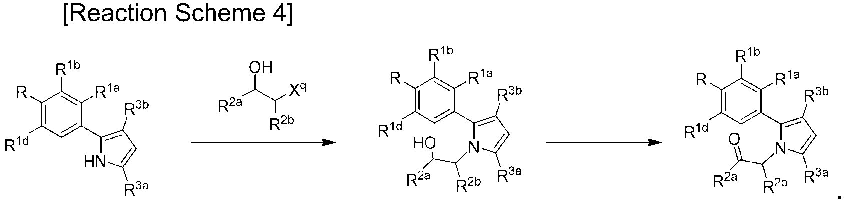

In an embodiment, the oxidizing agent may be at least one selected from the group consisting of PCC (pyridinium chlorochromate), PDC (pyridinium dichromate), Dess-Martin periodinane (DMP), IBX (2-iodoxybenzoic acid), Swern oxidant, and Parikh-Doering.

In an embodiment, when R is X^{q}, the preparation method may further include reacting an intermediate, which is generated after reacting Compound 1006 and a catalyst in an organic solvent in Step 4 but before obtaining the compound represented by Formula 1, with a Stille coupling starting material. Specifically, the Stille coupling starting material may be (tributylstannyl)methanol.

In an embodiment, when R is X^{q}, the preparation method may further include reacting with R^{1c}(OTs) after the reaction with the Stille coupling starting material. For example, a reaction such as Reaction Scheme 5 may be performed:

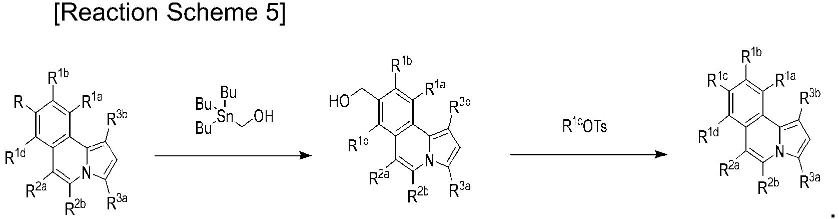

In an embodiment, the organic solvents in Steps 1 to 4 may be each independently at least one selected from the group consisting of methanol, ethanol, propanol, acetonitrile, dichloroethane (DCE), ethyl acetate, dimethyl sulfoxide, and dimethylformamide.

In an embodiment, the catalyst in Step 4 may be at least one selected from the group consisting of trifluoromethanesulfonic acid (TfOH), methanesulfonic acid (MsOH), p-toluenesulfonic acid (p-TsOH), and scandium triflate (Sc(OTf)₃), but is not limited thereto.

Another aspect is to provide a composition for detecting amyloid-beta (Aβ) including the compound represented by Formula 1 or a pharmaceutically acceptable salt thereof.

The term "amyloid-beta" or "amyloid-beta protein" refers to a protein or a fragment thereof capable of forming amyloid deposition, which aggregates by entangling with each other in the form of a beta-sheet structure. In the present disclosure, the amyloid-beta protein may refer to an individual protein constituting an amyloid-beta protein aggregate.

In an embodiment, the amyloid-beta may include oligomers, protofibrils, fibrils, plaques, protein monomers bound to homologous proteins, and aggregates, which are aggregates of amyloid-beta proteins. Specifically, the amyloid-beta may be at least one selected from the group consisting of Aβ oligomers, Aβ protofibrils, Aβ fibrils, and Aβ plaques. More specifically, the amyloid-beta may be an Aβ oligomer.

In an embodiment, the amyloid-beta may be a variant thereof.

In an embodiment, the compound represented by Formula 1 or a pharmaceutically acceptable salt thereof may interact with one or more domains selected from the group consisting of a domain including the amino acid sequence of SEQ ID NO: 1, a domain including the amino acid sequence of SEQ ID NO: 2, and a domain including the amino acid sequence of SEQ ID NO: 3.

The term "domain" refers to an amino acid sequence or a structural region to which a specific compound binds within the amino acid sequence constituting the amyloid beta (Aβ₁₋₄₂) protein.

Specifically, the "domain including the amino acid sequence of SEQ ID NO: n (wherein n is 1, 2, or 3)" may further include 0 to 5 (0, 1, 2, 3, 4, or 5) amino acids from the N-terminal amino acid and/or C-terminal amino acid sequence of said amino acid.

For example, in the case of the domain including the amino acid sequence of SEQ ID NO: 1, it may be a domain consisting of SEQ ID NO: 1, a domain consisting of a sequence in which 1 to 5 amino acids are further added to the N-terminal position of the amino acids of SEQ ID NO: 1, a domain consisting of a sequence in which 1 to 5 amino acids are further added to the C-terminal position of the amino acids of SEQ ID NO: 1, or a domain consisting of a sequence in which 1 to 5 amino acids are further added to the N-terminal and C-terminal positions of the amino acids of SEQ ID NO: 1.

If it is the domain including the amino acid sequence of SEQ ID NO: 2, it may be a domain consisting of SEQ ID NO: 2 or a domain consisting of a sequence in which 1 to 5 amino acids are further added to the N-terminal position of the amino acids of SEQ ID NO: 2.

Furthermore, in the case of the domain including the amino acid sequence of SEQ ID NO: 3, it may be a domain consisting of SEQ ID NO: 3 or a domain consisting of a sequence in which 1 to 5 amino acids are further added to the C-terminal position of the amino acids of SEQ ID NO: 3.

The amino acid sequences of SEQ ID NOs 1 to 3 are as follows:
SEQ ID NO: 1: FFAEDVG, SEQ ID NO: 2: MVGGVVIA, SEQ ID NO: 3: DAEFRHDS.

In an embodiment, the compound represented by Formula 1 or a pharmaceutically acceptable salt thereof may interact with one or more domains selected from the group consisting of: a domain consisting of an amino acid sequence having a sequence homology of about 70 % or more, about 75 % or more, about 80 % or more, about 85 % or more, about 90 % or more, about 92 % or more, about 95 % or more, about 97 % or more, about 98 % or more, or about 99 % or more with the amino acid sequence of SEQ ID NO: 1; a domain consisting of an amino acid sequence having a sequence homology of about 70 % or more, about 75 % or more, about 80 % or more, about 85 % or more, about 90 % or more, about 92 % or more, about 95 % or more, about 97 % or more, about 98 % or more, or about 99 % or more with the amino acid sequence of SEQ ID NO: 2; and a domain consisting of an amino acid sequence having a sequence homology of about 70 % or more, about 75 % or more, about 80 % or more, about 85 % or more, about 90 % or more, about 92 % or more, about 95 % or more, about 97 % or more, about 98 % or more, or about 99 % or more with the amino acid sequence of SEQ ID NO: 3.

In an embodiment, the compound represented by Formula 1 or a pharmaceutically acceptable salt thereof may interact with one or more domains selected from the group consisting of a domain consisting of the amino acid sequence of SEQ ID NO: 1, a domain consisting of the amino acid sequence of SEQ ID NO: 2, and a domain consisting of the amino acid sequence of SEQ ID NO: 3.

The composition for detection may detect amyloid-beta by combining the compound represented by Formula 1 or a pharmaceutically acceptable salt thereof with a labeling substance. The labeling substance may be for measuring the degree of binding between amyloid-beta and the compound represented by Formula 1 or a pharmaceutically acceptable salt thereof, and examples of the labeling substance may be a fluorescent substance or a fluorescent protein fragment.

The fluorescent substance may be a fluorescent dye having a basic skeleton of rhodamine, coumarin, EvoBlue, oxazine, carbopyronine, naphthalene, biphenyl, anthracene, phenanthrene, pyrene, or carbazole, or a derivative of said fluorescent dye. Specifically, the fluorescent substance may be one selected from the group consisting of Fluorescein, CR110: Carboxyrhodamine 110: Rhodamine Green (trade name), TAMRA: Carboxytetramethylrhodamine: TMR, Carboxyrhodamine 6G: CR6G, BODIPY FL (trade name): 4,4-difluoro-5,7-dimethyl-4-bora-3a,4a-diaza-s-indacene-3-propionic acid, BODIPY 493/503 (trade name): 4,4-difluoro-1,3,5,7-tetramethyl-4-bora-3a,4a-diaza-s-indacene-8-propionic acid, BODIPY R6G (trade name): 4,4-difluoro-5-(4-phenyl-1,3-butadienyl)-4-bora-3a,4a-diaza-s-indacene-3-propionic acid, BODIPY 558/568 (trade name): 4,4-difluoro-5-(2-thienyl)-4-bora-3a,4a-diaza-s-indacene-3-propionic acid, BODIPY 564/570 (trade name): 4,4-difluoro-5-styryl-4-bora-3a,4a-diaza-s-indacene-3-propionic acid, BODIPY 576/589 (trade name): 4,4-difluoro-5-(2-pyrrolyl)-4-bora-3a,4a-diaza-s-indacene-3-propionic acid, BODIPY 581/591 (trade name): 4,4-difluoro-5-(4-phenyl-1,3-butadienyl)-4-bora-3a,4a-diaza-s-indacene-3-propionic acid, EvoBlue10 (trade name), EvoBlue30 (trade name), MR121, ATTO 655 (trade name), ATTO 680 (trade name), ATTO 700 (trade name), ATTO MB2 (trade name), Alexa Fluor 350 (trade name), Alexa Fluor 405 (trade name), Alexa Fluor 430 (trade name), Alexa Fluor 488 (trade name), Alexa Fluor 532 (trade name), Alexa Fluor 546 (trade name), Alexa Fluor 555 (trade name), Alexa Fluor 568 (trade name), Alexa Fluor 594 (trade name), Alexa Fluor 633 (trade name), Alexa Fluor 680 (trade name), Alexa Fluor 700 (trade name), Alexa Fluor 750 (trade name), Alexa Fluor 790 (trade name), Flamma 496 (trade name), Flamma 507 (trade name), Flamma 530 (trade name), Flamma 552 (trade name), Flamma 560 (trade name), Flamma 575 (trade name), Flamma 581 (trade name), Flamma 648 (trade name), Flamma 675 (trade name), Flamma 749 (trade name), Flamma 774 (trade name), Flamma 775 (trade name), Rhodamine Red-X (trade name), Texas Red-X (trade name), 5(6)-TAMRA-X (trade name), 5TAMRA (trade name), Indocyanine green (ICG), and 2-((E)-2-((E)-2-(4-(2-carboxyethyl)phenoxy)-3-((E)-2-(3,3-dimethyl-5-sulfonato-1-(3-(trimethyl ammonio)-propyl)indolin-2-ylidene)ethylidene)cyclohex-1-enyl)vinyl)-3,3-dimethyl-1-(3-(trimethyl ammonio)-propyl)-3H-indolium-5-sulfonate disodium bromide (ZW800-1).

The fluorescent protein fragments include Venus, Cerulean, Citrine, and mKate, and may be fluorescent proteins exhibiting different colors or parts thereof.

In an embodiment, the composition for detection may be for confirming the detection of amyloid-beta protein from a sample isolated from a subject, and the isolated sample may be a biologically isolated sample.

The subject may be a mammal, for example, a mouse, human, pig, cow, horse, or sheep, specifically a human, and more specifically a human suspected of having a disease mediated by amyloid-beta accumulation.

The biologically isolated sample may be isolated from a tissue where amyloid-beta is distributed, and specifically, may be at least one selected from the group consisting of blood, plasma, cerebrospinal fluid, brain tissue lysate, cortical lysate, and hippocampal lysate.

Another aspect is to provide a method for detecting amyloid-beta (Aβ) including contacting an isolated biological sample with the compound represented by Formula 1 or a pharmaceutically acceptable salt thereof.

The method for detecting amyloid-beta (Aβ) may further include confirming the presence or absence of amyloid-beta or quantifying the amount of amyloid-beta in the biological sample treated with the compound represented by Formula 1 or a pharmaceutically acceptable salt thereof.

The confirming of the presence or absence of amyloid-beta or quantifying the amount of amyloid-beta may include a process of confirming the binding between the compound represented by Formula 1 or a pharmaceutically acceptable salt thereof and the amyloid-beta, and the confirmation of the binding may be performed by measuring an emission spectrum emitted by the bound compound represented by Formula 1 or a pharmaceutically acceptable salt thereof and the amyloid-beta. Specifically, it may be analyzing the presence or absence of amyloid-beta or quantifying the amount thereof by measuring fluorescence generated from the compound represented by Formula 1 or a salt thereof bound to the amyloid-beta when an excitation source is irradiated into a biological sample treated with the compound represented by Formula 1 or a pharmaceutically acceptable salt thereof.

In an embodiment, the measurement of the fluorescence signal may be performed using a microplate reader, a spectrophotometer, a microscope-based fluorescence detector, or a flow cytometer. In addition, the measurement of the fluorescence signal may be performed through a commercially available or known fluorescence detection system, but is not limited thereto.

The terms "pharmaceutically acceptable salt", "amyloid-beta", and the like are within the ranges described above.

Another aspect is to provide a composition for diagnosing a neurodegenerative disease including the compound represented by Formula 1 or a pharmaceutically acceptable salt thereof.

The term "neurodegenerative disease" refers to all diseases associated with degenerative changes in the brain, and in particular, refers to all brain diseases that can be caused by factors such as aggregation and accumulation of amyloid-beta in the brain and/or neurons. In addition, prodromal symptoms that are not generally recognized as diseases are also included in the amyloid-beta-related diseases. Examples of the neurodegenerative disease may be a disease selected from the group consisting of mild cognitive impairment, dementia, Alzheimer's disease, hereditary cerebral hemorrhage with amyloidosis-Dutch type (HCHWA-D), preclinical Alzheimer's disease, Parkinson's disease, Huntington's disease, mild cognitive impairment, mild cognitive impairment due to Alzheimer's disease, cerebral amyloid angiopathy, Down's syndrome, tauopathy, amyloid stroke, Lou Gehrig's disease, systemic amyloidosis, Dutch-type amyloidosis, Niemann-Pick disease, senile dementia, amyotrophic lateral sclerosis, spinocerebellar ataxia, Tourette's syndrome, Friedreich's ataxia, Machado-Joseph disease, Lewy body dementia, dystonia, progressive supranuclear palsy, and frontotemporal dementia.

The term "Alzheimer's disease" is used interchangeably with senile dementia and refers to a disease accompanied by mental deterioration associated with a specific neurodegenerative disease characterized by senile plaques, neurofibrillary tangles, and progressive neuronal loss.

The term "Parkinson's disease" refers to a chronic and progressive degenerative disease of the central nervous system that often exhibits symptoms impairing motor function and speech ability.

The term "Huntington's disease" refers to a neurodegenerative disease caused by a trinucleotide repeat expansion within the gene encoding the Huntingtin protein, accompanied by chorea, psychiatric disturbances, dementia, and the like.

The term "Lou Gehrig's disease" refers to a disease in which only motor neurons selectively die, exhibiting symptoms where both upper motor neurons in the cerebral cortex and lower motor neurons in the brainstem and spinal cord are progressively destroyed.

The term "Niemann-Pick disease" refers to a disease exhibiting symptoms indicating progressive destruction of neurons in the brain.

The term "tauopathy" may refer to a neurodegenerative disease in which neurons are damaged due to abnormal accumulation of tau protein (a family closely related to intracellular microtubule-associated proteins) in brain tissue.

The neurodegenerative disease may be characterized by higher amyloid-beta levels in affected individuals compared to unaffected individuals. Specifically, the amyloid-beta may be at least one selected from the group consisting of Aβ oligomers, Aβ protofibrils, Aβ fibrils, and Aβ plaques. More specifically, the amyloid-beta may be an Aβ oligomer. In addition, the high amyloid-beta level may appear in one or more selected from the group consisting of plasma, blood, cerebrospinal fluid, cortex, and hippocampus.

The terms "pharmaceutically acceptable salt", "amyloid-beta", and the like are within the ranges described above.

Another aspect is to provide a method of diagnosing a neurodegenerative disease including contacting a biological sample with the compound represented by Formula 1 or a pharmaceutically acceptable salt thereof.

The terms "pharmaceutically acceptable salt" and "neurodegenerative disease" and the like are within the ranges described above.

Another aspect is to provide a method of providing information regarding the diagnosis of an amyloid-beta-related disease, for example, a neurodegenerative disease, the method including contacting an isolated biological sample with the compound represented by Formula 1 or a pharmaceutically acceptable salt thereof.

The method of providing information may include confirming the presence or absence of amyloid-beta or quantifying the amount of amyloid-beta in the biological sample contacted with the compound represented by Formula 1 or a pharmaceutically acceptable salt thereof.

Furthermore, the method of providing information may further include comparing the quantified amount of amyloid-beta with the amount of amyloid-beta in a biological sample isolated from a subject not having said neurodegenerative disease (hereinafter, normal control group), and determining that the subject has the neurodegenerative disease when the quantified amount of amyloid-beta is greater than the amount of amyloid-beta of the normal control group.

The terms "pharmaceutically acceptable salt", "amyloid-beta-related disease", "neurodegenerative disease", "amyloid-beta", and the like are within the ranges described above.

Another aspect is to provide a method for screening an amyloid-beta protein aggregation inhibitor or an aggregate-dissolving agent, including contacting a sample containing amyloid-beta protein with the compound represented by Formula 1 or a pharmaceutically acceptable salt thereof.

The sample containing amyloid-beta protein may be, for example, plasma, blood, cerebrospinal fluid, cortical lysate, or hippocampal lysate derived from a neurodegenerative disease animal model; may be a culture of cells derived from a neurodegenerative disease animal model; or may be a solution containing purified amyloid-beta protein.

The screening method may be used for screening a therapeutic agent for the amyloid-beta-related disease or a therapeutic agent for a neurodegenerative disease. For example, it may be used in a manner of: administering a test substance and the compound represented by Formula 1 or a pharmaceutically acceptable salt thereof to an Alzheimer's disease animal model (in vivo); or treating a sample derived from an Alzheimer's disease animal model (ex vivo) or an in vitro system containing amyloid-beta protein with a test substance and the compound represented by Formula 1 or a pharmaceutically acceptable salt thereof; quantifying the amount of amyloid-beta protein; and selecting the test substance as a therapeutic agent for the disease if the quantitative value of amyloid-beta in an experimental group administered or treated with the specific test substance is lower than that of a control group not administered or treated.

The terms "amyloid-beta", "pharmaceutically acceptable salt", and the like are within the ranges described above.

### Advantageous Effects of Invention

The compound represented by Formula 1 or a salt thereof according to the present disclosure may specifically bind to amyloid-beta (Aβ). Due to its high selectivity, the compound or a pharmaceutically acceptable salt thereof can detect amyloid-beta in plasma with high sensitivity despite interference in detection caused by the presence of other proteins in plasma, and can be utilized for the diagnosis of a degenerative brain disease or screening for therapeutic agents for a degenerative brain disease.

### Brief Description of Drawings

FIGS. 1A to 1F are graphs showing fluorescence intensity measured upon binding of compounds according to an aspect to Aβ monomers (mAβ), oligomers (oAβ), or fibrils (fAβ) (FIGS. 1A and 1B: IK15 compounds; FIGS. 1C to 1F: AMD compounds).
FIGS. 2A and 2B show fluorescence intensity measured over time after binding of compounds according to an aspect with Aβ monomers (mAβ), oligomers (oAβ), or fibrils (fAβ).
FIG. 3A shows fluorescence intensity measured upon binding of a compound according to an aspect to Aβ monomers (mAβ), oligomers (oAβ), and fibrils (fAβ).
FIG. 3B shows fluorescence intensity measured over time after binding of a compound according to an aspect with Aβ monomers (mAβ), oligomers (oAβ), or fibrils (fAβ).
FIG. 3C shows the binding of a compound according to an aspect to Aβ oligomers (oAβ).
FIG. 3D shows the identification of the Aβ binding site of a compound according to an aspect.
FIG. 3E shows the binding of a compound according to an aspect to tau aggregates or Aβ oligomers (Aβ₁₋₄₂).
FIG. 3F is an experimental schematic for identifying the Aβ oligomer targeting site of a compound according to an aspect.
FIGS. 3G to 3J show the binding affinity of a compound according to an aspect for Aβ fragments or Aβ complexes.
FIG. 4A is an experimental schematic for verifying the Aβ detection efficacy of a compound according to an aspect in a 5XFAD transgenic mouse model.
FIG. 4B shows the detection of Aβ in cerebral cortex lysates of a 5XFAD transgenic mouse model using the IK15j compound according to an aspect.
FIG. 4C shows the detection of Aβ in hippocampal lysates of a 5XFAD transgenic mouse model using the IK15j compound according to an aspect.
FIG. 4D shows the detection of Aβ in the cerebrospinal fluid (hereinafter, CSF) of a 5XFAD transgenic mouse model using the IK15j compound according to an aspect.
FIG. 4E shows the detection of Aβ in the plasma of a 5XFAD transgenic mouse model using the IK15j compound according to an aspect.
FIG. 4F is a schematic diagram showing mouse brain regions for performing immunofluorescence with a compound according to an aspect and an anti-Aβ antibody.
FIG. 4G shows immunofluorescence performed in the brain of a 5XFAD transgenic mouse model using a compound according to an aspect and an anti-Aβ antibody.
FIGS. 5A and 5B show the detection of Aβ in cerebral cortex lysates of a 5XFAD transgenic mouse model using compounds according to an aspect.
FIGS. 6A and 6B show the detection of Aβ in hippocampal lysates of a 5XFAD transgenic mouse model using compounds according to an aspect.
FIGS. 7A and 7B show the detection of Aβ in the cerebrospinal fluid (hereinafter, CSF) of a 5XFAD transgenic mouse model using compounds according to an aspect.
FIG. 8 shows the detection of Aβ in the plasma of a 5XFAD transgenic mouse model using compounds according to an aspect.
FIG. 9A shows the sensitivity of a compound according to an aspect to Aβ oligomers.
FIG. 9B shows the binding of a compound according to an aspect to fibrinogen or albumin.
FIG. 9C shows the binding of a compound according to an aspect to Aβ oligomers in the presence of albumin.
FIG. 9D is an experimental schematic for verifying the binding of a compound according to an aspect to Aβ oligomers in the presence of albumin.
FIG. 9E shows the binding of a compound according to an aspect to Aβ oligomers in the presence of albumin.

### Mode for the Invention

Hereinafter, the present disclosure will be described in more detail by the following Examples. However, the following Examples are merely for illustrating the present disclosure, and the scope of the present disclosure is not limited thereto.

### <Reference Examples>

### <Reference Example 1> Fluorescence Spectral Scanning

Fluorescence spectral scanning of IK15 was performed using a microplate reader (SpectraMax M5, Molecular Devices). For the excitation scan, the compound of the present disclosure was dissolved in DMSO to prepare a 1 mM stock solution, followed by serial dilution with deionized water. Then, 150 µL of the sample (100 µM) was aliquoted into a 96-well clear round-bottom plate, and the compound's excitation spectrum was measured from 200 nm to 900 nm in 5 nm increments. The peak wavelength of each compound was recorded as its excitation wavelength. For the emission scan, 150 µL of the same sample prepared above was transferred to a 96-well opaque round-bottom plate. The excitation wavelength was set based on each compound, and the emission spectrum was scanned across various spectral ranges in 5 nm increments. Next, the emission spectra of candidate compounds mixed with Aβ were measured to determine whether these compounds exhibit a fluorescence shift in the presence of Aβ.

### <Reference Example 2> Preparation of MAP

A Mapping Amyloid Plate (MAP) for investigating the target site of the compound of the present disclosure on Aβ₁₋₄₂ fragments or Aβ₁₋₄₂ oligomers was prepared with reference to Korean Patent Application No. 10-2023-0133625, which is incorporated herein by reference in its entirety.

Briefly, to remove the bovine serum albumin coating from the plate, each well of the plate was washed three times with 200 µL of wash buffer (0.1 M sodium phosphate, 0.15 M sodium chloride, 0.05 % Tween-20, pH 7.2). Full-length and fragmented peptides of Aβ₁₋₄₂ were individually dissolved in DMSO to prepare 1.0 M stock solutions, which were then diluted with binding buffer (0.1 M sodium phosphate, 0.15 M sodium chloride, 10 mM EDTA, pH 7.2) to prepare 50 µg/mL peptide solutions (5 % DMSO). 100 µL of the peptide solution was added to each well and allowed to react with the maleimide at room temperature overnight. After peptide immobilization, unbound peptides were removed by washing three times with 200 µL of wash buffer. To inactivate residual maleimide groups, 200 µL of a cysteine solution (10 µg/mL in binding buffer) was added to each well and incubated at room temperature for 1 hour. After cysteine capping, all wells of the plate were washed three times with 200 µL of wash buffer.

### <Reference Example 3> Investigation of the Targeting Site of IK15 on Aβ₁₋₄₂ Oligomers

Two conditions were prepared to confirm whether the compound of the present disclosure targets the structure or the sequence of Aβ oligomers. First, the compound of the present disclosure (50 µM) was prepared in binding buffer (10 % DMSO), and 100 µL of the compound was added to the MAP and treated for 24 hours at room temperature. After incubation, all wells were washed three times with 200 µL of wash buffer. Second, full-length Aβ₁₋₄₂ peptide was added to the MAP and incubated at 37 °C for 8 hours to form complexes between full-length Aβ₁₋₄₂ and fragmented Aβ₁₋₄₂. After incubation, all wells were washed three times with 200 µL of wash buffer, and the MAP was treated with 100 µL of the compound of the present disclosure (50 µM) for 24 hours at room temperature. After treatment, all wells were washed three times with 200 µL of wash buffer. The fluorescence intensity of both plates was measured using a microplate reader (λex = 285 nm/λem = 475 nm).

### <Reference Example 4> Animals

To investigate the interaction between the compound of the present disclosure and Aβ oligomers in brain lysates, cerebrospinal fluid (CSF), and plasma of AD mouse models, 6-month-old female 5XFAD transgenic mice (Strain name: B6SJL-Tg(APPSwFILon,PSEN1*M146L*L286V)6799Vas/-Mmjax, which express Swedish (K670N/M671L), Florida (I716V), and London (V717I) mutations of APP and M146L and L286V mutations of PSEN1) and wild-type mice (C57BL/6 x SJL) were obtained from The Jackson Laboratory (Bar Harbor, ME, USA). All animal experiments were performed in accordance with the National Institutes of Health (NIH) Guide for the Care and Use of Laboratory Animals. The study protocols were approved by the Institutional Animal Care and Use Committee (IACUC) of Yonsei University (Seoul, Republic of Korea; IACUC-202103-1221-01). All animal studies followed the ARRIVE reporting guidelines.

### <Reference Example 5> Brain Lysate Analysis

To prepare brain lysates, mice were sacrificed, and the hippocampal and cortical regions of the mouse brain were dissected separately. Each brain region was homogenized in ice-cold RIPA buffer (20 mM Tris-HCl, pH 7.5, 50 mM NaCl, 0.5 % NP-40, 4 mM EDTA, 0.1 % SDS, 0.5 % sodium deoxycholate) containing 1X protease inhibitor cocktail (Roche Diagnostics, Switzerland). The homogenized brain tissue was incubated on ice for 20 minutes and then centrifuged at 14,000 rpm at 4 °C for 30 minutes, and the supernatant (soluble fraction) of the brain lysate was collected. To analyze the interaction between the compound of the present disclosure and the mouse brain lysate samples, the diluted brain lysate samples were mixed with the compound of the present disclosure (50 µM). The lysate samples containing the compound were loaded into the wells of a 96-well half-area black microplate (Corning, USA). The samples were analyzed and scanned at λex = 285 nm / λem = 475 nm.

### <Reference Example 6> Cerebrospinal Fluid (CSF) Analysis

To analyze the interaction between the compound of the present disclosure and mouse CSF, CSF samples from sacrificed mice were used. Each CSF sample was treated with the compound of the present disclosure (50 µM). The individual samples and the compound of the present disclosure were added to the wells of a 96-well half-area black microplate. The total volume of each sample was 50 µL. The signal of each sample was detected using a microplate reader (λex = 285 nm / λem = 475 nm).

### <Reference Example 7> Plasma Analysis

To analyze the interaction between the compound of the present disclosure and mouse plasma, plasma samples from sacrificed mice were used. The individual samples and the compound of the present disclosure were added to the wells of a 96-well half-area black microplate. The total volume of each sample was 50 µL. The signal of each sample was detected using a microplate reader (λex = 285 nm / λem = 475 nm).

### <Reference Example 8> Brain Staining of AD Mouse Models

Mouse brain tissues were fixed in 4 % paraformaldehyde (Biosesang, Korea) at 4 °C overnight and then cryoprotected by immersion in 30 % sucrose for 48 hours. Brain sections (25 µm) were cut using a cryostat (CM1860, Leica) and mounted on slides. The brain tissue sections were treated with the compound of the present disclosure (500 µM) overnight at room temperature. After washing with 1X PBS, antigen retrieval for the fixed brain sections was performed in 1X PBS (Gibco, Korea) containing 1 % SDS (Biosesang, Korea) for 10 minutes, followed by blocking with 20 % horse serum in 1X PBS. After blocking for 1 hour, the slides were incubated with mouse monoclonal antibody 6E10 (1:200, BioLegend, USA) at room temperature for 1 hour. After washing with 1X PBS, the slides were incubated with goat anti-mouse IgG conjugated with Alexa Fluor 555 (1:200, Invitrogen, USA) at room temperature for 1 hour. As a positional marker, the brain slides were stained with Hoechst 33342 (10 µg/mL in PBS) at room temperature for 3 minutes. After staining the cortical and hippocampal regions of the fixed brain, each brain slide was visualized using a fluorescence microscope (DM500, Leica) equipped with filter cubes containing excitation and emission filters: N2.1 filter cube for detecting 6E10 staining (Excitation filter: BP 515-560; Dichromatic mirror: 580; Emission filter: LP 590) and L5 filter cube for detecting the compound of the present disclosure (Excitation filter: BP 480/40; Dichromatic mirror: 505; Emission filter: BP 527/30).

### <Reference Example 9> Detection of Plasma Aβ Oligomers

Aβ₁₋₄₂ peptide (1,000, 100, 10, 1, 0.1, 0.01, and 0.001 pg/mL) was aggregated at 37 °C for 8 hours and added to human plasma (Sigma-Aldrich, USA). Then, each plasma sample was treated with the compound of the present disclosure (100 µM) for 8 hours at room temperature. The signal of each sample was detected using a microplate reader (λex = 285 nm / λem = 475 nm).

### <Reference Example 10> Investigation of IK15 Selectivity for Blood Proteins

Aβ₁₋₄₂ peptide was aggregated at 37 °C for 8 hours, and 25 µM of Aβ₁₋₄₂ oligomers, albumin (Sigma-Aldrich, USA), and fibrinogen (Sigma-Aldrich, USA) were seeded into a black microplate. Each sample was treated with IK15j (100 µM) for 8 hours at room temperature. The signal of each sample was detected using a microplate reader (λex = 285 nm / λem = 475 nm).

### <Reference Example 11> Investigation of IK15 Selectivity for Albumin-Aβ Oligomer Complexes

Full-length Aβ₁₋₄₂ peptide was immobilized on a maleimide-activated microplate. Then, the plate was treated with additional Aβ₁₋₄₂ peptide (10 µM) at 37°C for 8 hours. After peptide immobilization, unbound peptides were removed by washing three times with 200 µL of wash buffer. To form albumin-Aβ oligomer complexes, the plate was treated with albumin (10 µM, Sigma-Aldrich, USA) at room temperature for 24 hours, and unbound residues were removed by washing three times with 200 µL of wash buffer. The control Aβ oligomer wells were not treated with albumin. Each well was treated with the compound of the present disclosure (100 µM), and the signal of each sample was detected using a microplate reader (λex = 285 nm / λem = 475 nm).

### <Reference Example 12> Statistical Analysis

All graphical data were analyzed using GraphPad Prism 9.0 software, and statistical analyses were performed using one-way analysis of variance (ANOVA) followed by Bonferroni's post-hoc comparisons or Student's unpaired t-test. Error bars represent the standard error of the mean (S.E.M.).

### <Preparation Examples>

### <Preparation Example 1> Preparation of Examples 1 to 13

Examples of the compound according to one aspect (hereinafter, Examples 1 to 13) were synthesized according to Reaction Scheme 6 below.

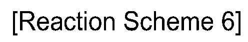

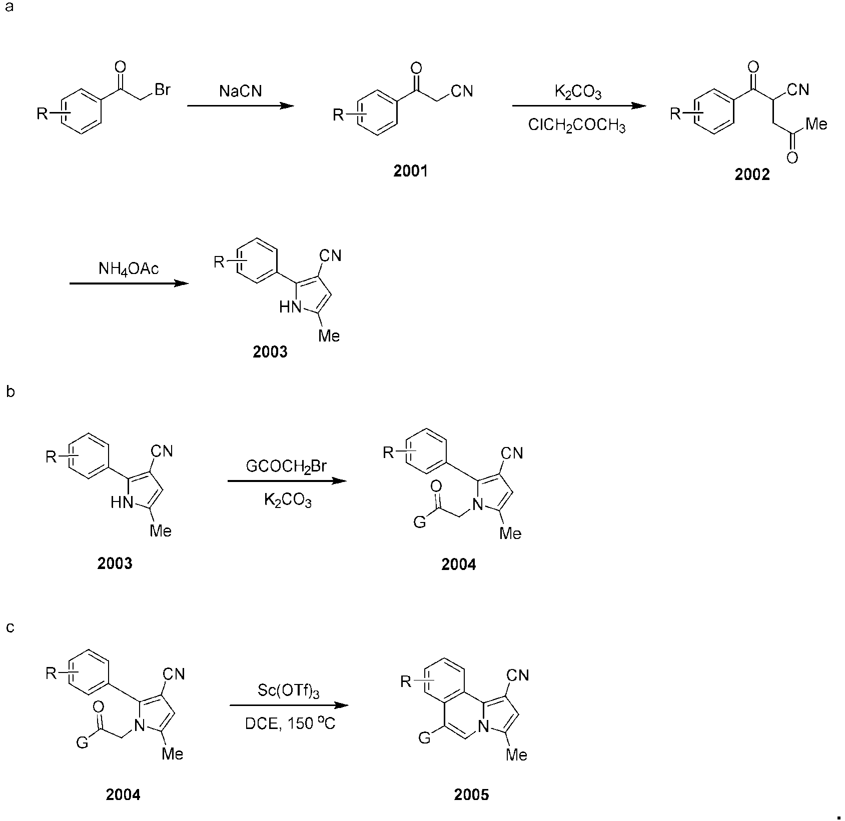

Commercially available various α-bromoketones were treated with NaCN to obtain Compound 2001, and then the β-ketonitrile was subjected to base-mediated alkylation with 2-chloroacetone to obtain the 1,4-diketo Compound 2002, which was treated with NH₄OAc to convert it into the corresponding 2-arylpyrrole Compound 2003. Subsequently, N-alkylation of various α-haloketones to the 2-arylpyrrole Compound 2003 generated Compound 2004. Then, under optimized conditions, Compound 2004, which is a various N-substituted 2-arylpyrrole derivative compound, was synthesized into the pyrrolo[2,1-a]isoquinoline Compound 2005. By varying the α-bromoketone or the α-haloketone, 13 example compounds (Example 1 (Compound represented by Formula 3; IK15a), Example 2 (Compound represented by Formula 4; IK15b), Example 3 (Compound represented by Formula 5; IK15c), Example 4 (Compound represented by Formula 6; IK15d), Example 5 (Compound represented by Formula 7; IK15e), Example 6 (Compound represented by Formula 8; IK15f), Example 7 (Compound represented by Formula 9; IK15I), Example 8 (Compound represented by Formula 10; IK15n), Example 9 (Compound represented by Formula 11; IK15o), Example 10 (Compound represented by Formula 12; IK15p), Example 11 (Compound represented by Formula 13; IK15q), Example 12 (Compound represented by Formula 14; IK15r), Example 13 (Compound represented by Formula 15; IK15s)) were prepared.

For the synthesized compounds of Examples 1 to 13, H-NMR (400 MHz, CDCl₃) and C-NMR (100 MHz, CDCl₃) were performed according to known NMR experimental methods to analyze the structures of the substances, and the results are as follows:

### [Example 1 (Compound represented by Formula 3; IK15a; 3-methyl-6-phenylpyrrolo[2,1-a]isoquinoline-1-carbonitrile)]

¹H NMR (400 MHz, CDCl₃) δ 8.91(d, *J* = 8.0 Hz, 1H), 7.62 (s, 3H), 7.54-7.46 (m, 6H), 6.76 (s, 1H), 2.49 (s, 3H),

¹³C{1H} NMR (100 MHz, CDCl₃) δ 136.5, 133.3, 130.2, 128.7, 128.3, 128.2, 127.6, 127.5, 126.7, 125.9, 125.0, 123.6, 123.1, 120.1, 118.9, 114.3, 82.4, 11.5; HRMS (ESI-QTOF) m/z [M+H]⁺ calcd for C₂₀H₁₅N₂ 283.1230, found 280.1239.

### [Example 2 (Compound represented by Formula 4; IK15b; 6-(4-methoxyphenyl)-3-methylpyrrolo[2,1-a]isoquinoline-1-carbonitrile)]

¹H NMR (400 MHz, CDCl₃) δ 8.89 (d, J = 7.6 Hz, 1H), 7.67-7.62 (m, 2H), 7.59 (s, 1H), 7.50-7.45 (m, 1H), 7.40 (d, J = 7.6 Hz, 2H), 7.06 (d, J = 7.6 Hz, 2H), 6.75 (s, 1H), 3.91 (s, 3H), 2.48 (s, 3H),

¹³C{1H} NMR (100 MHz, CDCl₃) δ 159.6, 133.2, 131.3, 128.6, 128.2, 127.8, 127.6, 126.3, 125.9, 125.0, 123.5, 123.1, 120.0, 119.0, 114.2, 114.1, 82.3, 55.4, 11.5; HRMS (ESI-QTOF) m/z [M+H]⁺ calcd for C₂₁H₁₇N₂O 313.1336, found 313.1340.

### [Example 3 (Compound represented by Formula 5; IK15c; 3-methyl-6-(p-tolyl)pyrrolo[2,1-a]isoquinoline-1-carbonitrile)]

¹H NMR (400 MHz, CDCl₃) δ 8.90 (d, J = 7.6 Hz, 1H), 7.66-7.62 (m, 2H), 7.60 (s, 1H), 7.50-7.43 (m, 1H), 7.39-7.33 (m, 4H), 6.75 (s, 1H), 2.47 (s, 6H),

¹³C{1H} NMR (100 MHz, CDCl₃) δ 138.1, 133.5, 133.2, 130.0, 129.4, 128.3, 127.7, 127.6, 126.6, 125.9, 125.0, 123.6, 123.1, 120.0, 119.0, 114.2, 82.4, 21.3, 11.5; HRMS (ESI-QTOF) m/z [M+H]⁺ calcd for C₂₁H₁₇N₂ 297.1386, found 297.1392.

### [Example 4 (Compound represented by Formula 6; IK15d; 6-(4-chlorophenyl)-3-methylpyrrolo[2,1-a]isoquinoline-1-carbonitrile)]

¹H NMR (400 MHz, CDCl₃) δ 8.89 (d, J = 8.0 Hz, 1H), 7.65-7.60 (m, 1H), 7.59 (s, 1H), 7.56 (s, 1H), 7.52-7.47 (m, 3H), 7.42 (d, J = 8.4 Hz, 2H), 6.76 (s, 1H), 2.49 (s, 3H),

¹³C{1H} NMR (100 MHz, CDCl₃) δ 134.9, 134.4, 133.2, 131.5, 129.0, 128.5, 127.7, 127.2, 125.6, 125.5, 125.0, 123.7, 123.2, 120.2, 118.8, 114.4, 82.7, 11.5; HRMS (ESI-QTOF) m/z [M+H]⁺ calcd for C₂₀H₁₄N₂Cl 317.0840, found 317.0828.

### [Example 5 (Compound represented by Formula 7; IK15e; 3-methyl-6-(naphthalen-2-yl)pyrrolo[2,1-a]isoquinoline-1-carbonitrile)]

¹H NMR (400 MHz, CDCl₃) δ 8.93 (d, J = 8.0 Hz, 1H), 8.01-7.91 (m, 4H), 7.71 (s, 1H), 7.68-7.63 (m, 2H), 7.59 (s, 3H), 7.50-7.43 (m, 1H), 6.78 (s, 1H), 2.51 (s, 3H),

¹³C{1H} NMR (100 MHz, CDCl₃) δ 134.0, 133.4, 133.3, 132.9, 129.0, 128.4, 128.2, 128.1, 128.0, 127.8, 127.7, 127.6, 126.69, 126.65, 126.6, 126.0, 125.1, 123.7, 123.1, 120.4, 118.9, 114.3, 82.5, 11.6; HRMS (ESI-QTOF) m/z [M+H]⁺ calcd for C₂₄H₁₇N₂ 333.1386, found 333.1396.

### [Example 6 (Compound represented by Formula 8; IK15f; 3,6-dimethylpyrrolo[2,1-a]isoquinoline-1-carbonitrile)]

¹H NMR (400 MHz, CDCl₃) δ 8.83 (d, *J = 7.2* Hz, 1H), 7.77 (d, *J* = 6.8 Hz, 1H), 7.63-7.56 (m, 2H), 7.51 (s, 1H), 6.67 (s, 1H), 2.50 (s, 3H), 2.46 (s, 3H),

¹³C{1H} NMR (100 MHz, CDCl₃) δ 133.2, 128.3, 128.0, 127.6, 124.8, 123.8, 123.1, 123.0, 119.5, 119.2, 119.1, 113.6, 82.0, 16.7, 11.5; HRMS (ESI-QTOF) m/z [M+H]⁺ calcd for C₁₅H₁₃N₂ 221.1073, found 221.1084.

### [Example 7 (Compound represented by Formula 9; IK15I; 5-(4-methoxyphenyl)-8-methylbenzo[f]pyrrolo[2,1-a]isoquinoline-10-carbonitrile)]

¹H NMR (400 MHz, CDCl₃) δ 8.95 (d, J = 7.2 Hz, 1H), 8.80 (d, J = 7.6 Hz, 1H), 7.89 (d, J = 7.2 Hz, 1H), 7.65 (s, 2H), 7.45 (s, 1H), 7.34 (d, J = 6.4 Hz, 2H), 7.15 (s, 1H), 7.04 (d, J = 6.4 Hz, 2H), 6.85 (s, 1H), 3.93 (s, 3H), 2.50 (s, 3H),

¹³C{1H} NMR (100 MHz, CDCl₃) δ 159.4, 133.6, 133.2, 130.4, 130.1, 129.5, 128.8, 127.5, 126.2, 125.5, 124.5, 123.6, 122.6, 120.7, 119.3, 115.7, 114.5, 81.7, 55.4, 11.5; HRMS (ESI-QTOF) m/z [M+H]⁺ calcd for C₂₅H₁₉N₂O 363.1492, found 363.1502.

### [Example 8 (Compound represented by Formula 10; IK15n; 3-methyl-6,8-diphenylpyrrolo[2,1-a]isoquinoline-1-carbonitrile)]

¹H NMR (400 MHz, CDCl₃) δ 8.97 (d, J = 8.4 Hz, 1H), 7.88 (d, J = 8.4 Hz, 1H), 7.84 (s, 1H), 7.64 (s, 1H), 7.59-7.52 (m, 7H), 7.43 (t, J = 7.2 Hz, 2H), 7.36 (d, J = 6.0 Hz, 1H), 6.78 (s, 1H), 2.50 (s, 3H),

¹³C{1H} NMR (100 MHz, CDCl₃) δ 140.3, 135.4, 133.2, 130.1, 128.9, 128.8, 128.3, 128.0, 127.7, 127.5, 127.2, 126.8, 124.1, 123.9, 123.8, 123.6, 120.5, 118.9, 114.4, 93.3, 82.5, 11.6; HRMS (ESI-QTOF) m/z [M+H]⁺ calcd for C₂₆H₁₉N₂ 359.1543, found 359.2311.

### [Example 9 (Compound represented by Formula 11; IK15o; 6-(4-methoxyphenyl)-3-methyl-8-phenylpyrrolo[2,1-a]isoquinoline-1-carbonitrile)]

¹H NMR (400 MHz, CDCl₃) δ 8.94 (d, J = 8.4 Hz, 1H), 7.88-7.84 (m, 2H), 7.61 (s, 1H), 7.57 (d, J = 7.6 Hz, 2H), 7.46-7.41 (m, 4H), 7.37 (d, J = 6.8 Hz, 1H), 7.06 (d, J = 8.4 Hz, 2H), 6.75 (s, 1H), 3.91 (s, 3H), 2.49 (s, 3H),

¹³C{1H} NMR (100 MHz, CDCl₃) δ 159.6, 140.3, 130.2, 133.1, 131.3, 128.9, 128.6, 128.3, 127.7, 127.3, 127.2, 126.4, 124.03, 123.96, 123.7, 123.6, 120.4, 119.0, 114.3, 114.2, 82.4, 55.4, 11.6; HRMS (ESI-QTOF) m/z [M+H]⁺ calcd for C₂₇H₂₁N₂O 389.1649, found 389.1658.

### [Example 10 (Compound represented by Formula 12; IK15p; 3-methyl-8-phenyl-6-(p-tolyl)pyrrolo[2,1-a]isoquinoline-1-carbonitrile)]

¹H NMR (400 MHz, CDCl₃) δ 8.96 (d, J = 8.8 Hz, 1H), 7.89-7.84 (m, 2H), 7.62 (s, 1H), 7.57 (d, J = 7.6 Hz, 2H), 7.46-7.41 (m, 3H), 7.40 (s, 1H), 7.37-7.33 (m, 3H), 6.77 (s, 1H), 2.49 (s, 3H), 2.48 (s, 3H),

¹³C{1H} NMR (100 MHz, CDCl₃) δ 140.4, 140.3, 138.1, 133.4, 133.1, 130.0, 129.5, 128.9, 128.1, 127.7, 127.4, 127.3, 126.7, 124.1, 124.0, 123.7, 123.6, 120.4, 119.0, 114.3, 82.4, 21.3, 11.6; HRMS (ESI-QTOF) m/z [M+H]⁺ calcd for C₂₇H₂₁N₂ 373.1699, found 373.1693.

### [Example 11 (Compound represented by Formula 13; IK15q; 3,6-dimethyl-8-phenylpyrrolo[2,1-a]isoquinoline-1-carbonitrile)]

¹H NMR (400 MHz, CDCl₃) δ 8.87 (d, J = 8.4 Hz, 1H), 7.93 (s, 1H), 7.85 (d, J = 6.8 Hz, 1H), 7.70 (d, J = 6.8 Hz, 2H), 7.52 (d, J = 6.0 Hz, 2H), 7.49 (s, 1H), 7.42 (d, J = 6.4 Hz, 1H), 6.67 (s, 1H), 2.54 (s, 3H), 2.47 (s, 3H),

¹³C{1H} NMR (100 MHz, CDCl₃) δ 140.5, 140.3, 133.1, 129.0, 128.7, 127.8, 127.3, 127.2, 123.9, 123.6, 131.2, 122.0, 119.7, 119.5, 119.1, 113.8, 82.1, 16.8, 11.5; HRMS (ESI-QTOF) m/z [M+H]⁺ calcd for C₂₁H₁₇N₂ 297.1386, found 297.1391.

### [Example 12 (Compound represented by Formula 14; IK15r; 8-chloro-6-(4-methoxyphenyl)-3-methylpyrrolo[2,1-a]isoquinoline-1-carbonitrile)]

¹H NMR (400 MHz, CDCl₃) δ 8.80 (d, J = 6.4 Hz, 1H), 7.60 (d, J = 4.8 Hz, 2H), 7.55 (d, J = 7.6 Hz, 1H), 7.38 (d, J = 6.0 Hz, 2H), 7.07 (d, J = 6.0 Hz, 2H), 6.75 (s, 1H), 3.91 (s, 3H), 2.48 (s, 3H),

¹³C{1H} NMR (100 MHz, CDCl₃) δ 159.8, 133.5, 132.5, 131.2, 129.3, 128.6, 127.8, 125.4, 125.3, 124.5, 123.9, 123.4, 121.0, 118.6, 114.4, 114.3, 82.7, 55.4, 11.5; HRMS (ESI-QTOF) m/z [M+H]⁺ calcd for C₂₁H₁₆ClN₂O 347.0946, found 347.0952.

### [Example 13 (Compound represented by Formula 15; IK15s; 8-bromo-6-(4-methoxyphenyl)-3-methylpyrrolo[2,1-a]isoquinoline-1-carbonitrile)]

¹H NMR (400 MHz, CDCl₃) δ 8.74 (d, J = 8.8 Hz, 1H), 7.75 (s, 1H), 7.69 (d, J = 7.2 Hz, 1H), 7.60 (s, 1H), 7.37 (d, J = 8.4 Hz, 2H), 7.07 (d, J = 8.0 Hz, 2H), 6.76 (s, 1H), 3.91 (s, 3H), 2.48 (s, 3H),

¹³C{1H} NMR (100 MHz, CDCl₃) δ 159.8, 132.6, 131.3, 131.2, 129.5, 128.4, 127.8, 125.3, 123.6, 123.9, 123.7, 121.7, 121.0, 118.6, 114.5, 114.3, 82.9, 55.4, 11.5; HRMS (ESI-QTOF) m/z [M+H]⁺ calcd for C₂₁H₁₆BrN₂O 391.0441, found 391.0444.

### <Preparation Example 2> Preparation of Example 14 (compound represented by Formula 16; AMD-D-253)

The compound of Example 14 was synthesized according to Reaction Scheme 7 below.

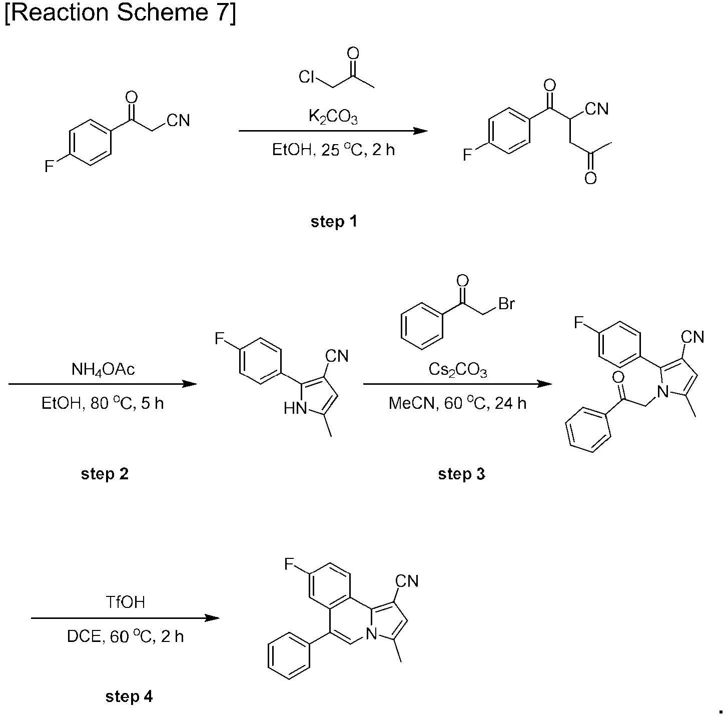

### Step 1. Preparation of 2-(4-fluorobenzoyl)-4-oxopentanenitrile

3-(4-Fluorophenyl)-3-oxopropanenitrile (5 g, 30.60 mmol) and 1-chloropropan-2-one (3.1 g, 33.66 mmol) were mixed in ethanol (80 mL), and potassium carbonate (10.5 g, 76.50 mmol) was added thereto. The mixture was stirred at 25 °C under nitrogen for 2 hours. Water (50 mL) was added to the reaction mixture, followed by extraction with ethyl acetate (120 mL × 3). The organic layers were combined, washed with saturated sodium chloride solution, dried over sodium sulfate, and concentrated. The concentrate was purified by silica gel column chromatography (ethyl acetate/petroleum ether, volume ratio = 5:95) to obtain 2-(4-fluorobenzoyl)-4-oxopentanenitrile (3 g, yield 44 %).

### Step 2. Preparation of 2-(4-fluorophenyl)-5-methyl-1H-pyrrole-3-carbonitrile

2-(4-Fluorobenzoyl)-4-oxopentanenitrile (3 g, 13.70 mmol) and ammonium acetate (2.1 g, 27.40 mmol) were mixed in ethanol (50 mL), and the mixture was stirred at 80 °C for 5 hours. Water (50 mL) was added to the reaction mixture, followed by extraction with dichloromethane (100 mL × 3). The organic layers were combined, washed with saturated sodium chloride solution, dried over sodium sulfate, and concentrated. The concentrate was purified by silica gel column chromatography (ethyl acetate/petroleum ether, volume ratio = 12:88) to obtain 2-(4-fluorophenyl)-5-methyl-1 H-pyrrole-3-carbonitrile (2.2 g, yield 80 %) as a yellow solid.

### Step 3. Preparation of 2-(4-fluorophenyl)-5-methyl-1-(2-oxo-2-phenylethyl)-1H-pyrrole-3-carbonitrile

2-(4-Fluorophenyl)-5-methyl-1H-pyrrole-3-carbonitrile (200 mg, 0.99 mmol) and 2-bromo-1-phenylethan-1-one (238 mg, 1.20 mmol) were mixed in acetonitrile (60 mL), and cesium carbonate (651 mg, 1.99 mmol) was added thereto. The mixture was stirred at 60 °C for 24 hours. Water (40 mL) was added to the reaction mixture, followed by extraction with dichloromethane (80 mL × 3). The organic layers were combined, washed with saturated sodium chloride solution, dried over sodium sulfate, and concentrated. The concentrate was purified by silica gel column chromatography (ethyl acetate/petroleum ether, volume ratio = 10:90) to obtain 2-(4-fluorophenyl)-5-methyl-1-(2-oxo-2-phenylethyl)-1H-pyrrole-3-carbonitrile (220 mg, yield 69 %) as a white solid.

### Step 4. Preparation of 8-fluoro-3-methyl-6-phenylpyrrolo[2,1-a]isoquinoline-1-carbonitrile (Example 14)

2-(4-Fluorophenyl)-5-methyl-1-(2-oxo-2-phenylethyl)-1H-pyrrole-3-carbonitrile (200 mg, 0.63 mmol), trifluoromethanesulfonic acid (471 mg, 3.14 mmol), and 1,2-dichloroethane (30 mL) were mixed, and the mixture was stirred at 60 °C for 2 hours. Water (25 mL) was added to the reaction mixture, followed by extraction with dichloromethane (50 mL × 3). The organic layers were combined, washed with saturated sodium chloride solution, dried over sodium sulfate, and concentrated. The concentrate was purified by Prep-HPLC (25 % - 95 % acetonitrile/0.1 % aqueous ammonia) to obtain 8-fluoro-3-methyl-6-phenylpyrrolo[2,1-a]isoquinoline-1-carbonitrile (19.8 mg, yield 10 %) as a white solid.

LCMS (ESI-MS): mass calcd C₂₀H₁₃FN₂ 300.1 m/z, found 301.1 [M+H]⁺.

¹H NMR (400 MHz, CDCl₃) δ 8.91 (dd, J = 9.2, 5.6 Hz, 1H), 7.65 (s, 1H), 7.57-7.50 (m, 3H), 7.49-7.45 (m, 2H), 7.39-7.33 (m, 1H), 7.30-7.27 (m, 1H), 6.76 (s, 1H), 2.49 (s, 3H).

### <Preparation Example 3> Preparation of Example 15 (compound represented by Formula 17; AMD-D-254)

The compound of Example 15 was synthesized according to Reaction Scheme 8 below.

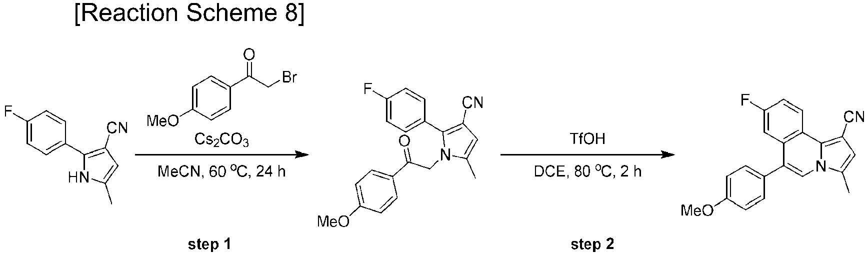

### Step 1. Preparation of 2-(4-fluorophenyl)-1-(2-(4-methoxyphenyl)-2-oxoethyl)-5-methyl-1H-pyrrole-3-carbonitrile

2-(4-Fluorophenyl)-5-methyl-1H-pyrrole-3-carbonitrile (200 mg, 0.99 mmol), which is the intermediate of Step 2 of Example 14, and 2-bromo-1-(4-methoxyphenyl)ethan-1-one (275 mg, 1.20 mmol) were mixed in acetonitrile (60 mL), and cesium carbonate (651 mg, 1.99 mmol) was added thereto. The mixture was stirred at 60 °C for 24 hours. Water (40 mL) was added to the reaction mixture, followed by extraction with dichloromethane (80 mL × 3). The organic layers were combined, washed with saturated sodium chloride solution, dried over sodium sulfate, and concentrated. The concentrate was purified by silica gel column chromatography (ethyl acetate/petroleum ether, volume ratio = 10:90) to obtain 2-(4-fluorophenyl)-1-(2-(4-methoxyphenyl)-2-oxoethyl)-5-methyl-1H-pyrrole-3-carbonitrile (220 mg, yield 63 %) as a white solid.

### Step 2. Preparation of 8-fluoro-6-(4-methoxyphenyl)-3-methylpyrrolo[2,1-a]isoquinoline-1-carbonitrile (Example 15)

2-(4-Fluorophenyl)-1-(2-(4-methoxyphenyl)-2-oxoethyl)-5-methyl-1H-pyrrole-3-carbonitrile (100 mg, 0.29 mmol), trifluoromethanesulfonic acid (430 mg, 2.87 mmol), and 1,2-dichloroethane (30 mL) were mixed, and the mixture was stirred at 80 °C for 2 hours. Water (25 mL) was added to the reaction mixture, followed by extraction with dichloromethane (50 mL × 3). The organic layers were combined, washed with saturated sodium chloride solution, dried over sodium sulfate, and concentrated. The concentrate was purified by Prep-HPLC (25 % - 95 % acetonitrile/0.1 % aqueous ammonia) to obtain 8-fluoro-6-(4-methoxyphenyl)-3-methylpyrrolo[2,1-a]isoquinoline-1-carbonitrile (25.9 mg, yield 27 %) as a white solid.

LCMS (ESI-MS): mass calcd C₂₁H₁₅FN₂O 330.1 m/z, found 330.9 [M+H]⁺.

¹H NMR (400 MHz, CDCl₃) δ 8.89 (dd, J = 8.8, 5.2 Hz, 1H), 7.62 (s, 1H), 7.40-7.37 (m, 2H), 7.36-7.27 (m, 2H), 7.08-7.05 (m, 2H), 6.75 (s, 1H), 3.91 (s, 3H), 2.48 (s, 3H).

### <Preparation Example 4> Preparation of Example 16 (compound represented by Formula 18; AMD-D-255)

The compound of Example 16 was synthesized according to Reaction Scheme 9 below.

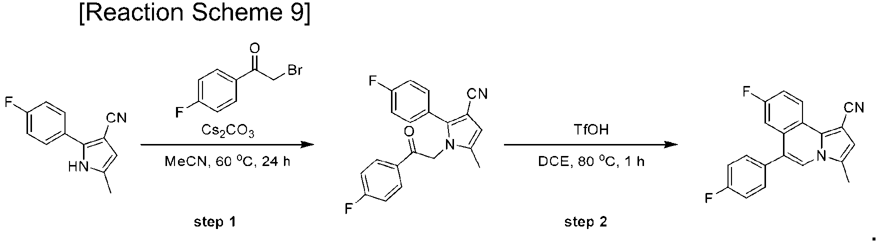

### Step 1. Preparation of 2-(4-fluorophenyl)-1-(2-(4-fluorophenyl)-2-oxoethyl)-5-methyl-1H-pyrrole-3-carbonitrile

2-(4-Fluorophenyl)-5-methyl-1H-pyrrole-3-carbonitrile (300 mg, 1.50 mmol), which is the intermediate of Step 2 of Example 14, and 2-bromo-1-(4-fluorophenyl)ethan-1-one (390 mg, 1.80 mmol) were mixed in acetonitrile (40 mL), and cesium carbonate (976 mg, 3.00 mmol) was added thereto. The mixture was stirred at 60 °C for 24 hours. Water (40 mL) was added to the reaction mixture, followed by extraction with dichloromethane (80 mL × 3). The organic layers were combined, washed with saturated sodium chloride solution, dried over sodium sulfate, and concentrated. The concentrate was purified by silica gel column chromatography (ethyl acetate/petroleum ether, volume ratio = 10:90) to obtain 2-(4-fluorophenyl)-1-(2-(4-fluorophenyl)-2-oxoethyl)-5-methyl-1H-pyrrole-3-carbonitrile (150 mg, yield 29 %) as a white solid.

### Step 2. Preparation of 8-fluoro-6-(4-fluorophenyl)-3-methylpyrrolo[2,1-a]isoquinoline-1-carbonitrile (Example 16)

2-(4-Fluorophenyl)-1-(2-(4-fluorophenyl)-2-oxoethyl)-5-methyl-1H-pyrrole-3-carbonitrile (100 mg, 0.29 mmol), trifluoromethanesulfonic acid (223 mg, 1.49 mmol), and 1,2-dichloroethane (30 mL) were mixed, and the mixture was stirred at 80 °C for 1 hour. Water (25 mL) was added to the reaction mixture, followed by extraction with dichloromethane (50 mL × 3). The organic layers were combined, washed with saturated sodium chloride solution, dried over sodium sulfate, and concentrated. The concentrate was purified by Prep-HPLC (25 % - 65 % acetonitrile/0.1 % aqueous ammonia) to obtain 8-fluoro-6-(4-fluorophenyl)-3-methylpyrrolo[2,1-a]isoquinoline-1-carbonitrile (20.1 mg, yield 21 %) as a white solid.

LCMS (ESI-MS): mass calcd C₂₀H₁₂F₂N₂ 318.1 m/z, found 318.6 [M+H]⁺.

¹H NMR (400 MHz, CDCl₃) δ 8.91 (dd, J = 9.2, 5.6 Hz, 1H), 7.63 (s, 1H), 7.46-7.42 (m, 2H), 7.39-7.34 (m, 1H), 7.25-7.19 (m, 3H), 6.77 (s, 1H), 2.49 (s, 3H).

### <Preparation Example 5> Preparation of Example 17 (compound represented by Formula 19; AMD-D-257)

The compound of Example 17 was synthesized according to Reaction Scheme 10 below.

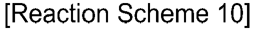

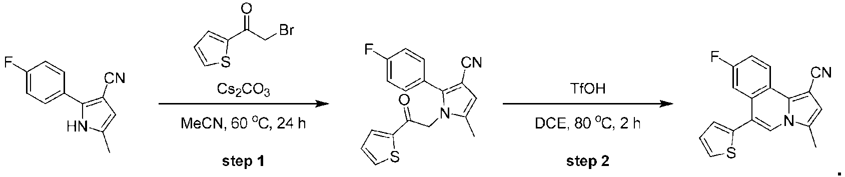

### Step 1. Preparation of 2-(4-fluorophenyl)-5-methyl-1-(2-oxo-2-(thiophen-2-yl) ethyl)-1H-pyrrole-3-carbonitrile

2-(4-Fluorophenyl)-5-methyl-1H-pyrrole-3-carbonitrile (300 mg, 1.50 mmol), which is the intermediate of Step 2 of Example 14, and 2-bromo-1-(thiophen-2-yl)ethan-1-one (369 mg, 1.80 mmol) were mixed in acetonitrile (50 mL), and cesium carbonate (976 mg, 3.00 mmol) was added thereto. The mixture was stirred at 60 °C for 24 hours. Water (40 mL) was added to the reaction mixture, followed by extraction with dichloromethane (80 mL × 3). The organic layers were combined, washed with saturated sodium chloride solution, dried over sodium sulfate, and concentrated. The concentrate was purified by silica gel column chromatography (ethyl acetate/petroleum ether, volume ratio = 10:90) to obtain 2-(4-fluorophenyl)-5-methyl-1-(2-oxo-2-(thiophen-2-yl)ethyl)-1H-pyrrole-3-carbonitrile (160 mg, yield 33 %) as a white solid.

### Step 2. Preparation of 8-fluoro-3-methyl-6-(thiophen-2-yl) pyrrolo[2, 1-a]isoquinoline-1-carbonitrile (Example 17)

2-(4-Fluorophenyl)-5-methyl-1-(2-oxo-2-(thiophen-2-yl)ethyl)-1H-pyrrole-3-carbonitrile (100 mg, 0.31 mmol), trifluoromethanesulfonic acid (231 mg, 1.54 mmol), and 1,2-dichloroethane (30 mL) were mixed, and the mixture was stirred at 80 °C for 2 hours. Water (25 mL) was added to the reaction mixture, followed by extraction with dichloromethane (50 mL × 3). The organic layers were combined, washed with saturated sodium chloride solution, dried over sodium sulfate, and concentrated. The concentrate was purified by Prep-HPLC (25 % - 70 % acetonitrile /0.1 % aqueous ammonia) to obtain 8-fluoro-3-methyl-6-(thiophen-2-yl)pyrrolo[2,1-a]isoquinoline-1-carbonitrile (AMD-D-257) (18.5 mg, yield 19 %) as a white solid.

LCMS (ESI-MS): mass calcd C₁₈H₁₁FN₂S 306.1 m/z, found 307.0 [M+H]⁺.

¹H NMR (400 MHz, CDCl₃) δ 8.89 (dd, J = 8.8, 5.2 Hz, 1H), 7.79 (s, 1H), 7.58-7.56 (m, 1H), 7.51-7.48 (m, 1H), 7.41-7.35 (m, 1H), 7.25-7.21 (m, 2H), 6.76 (s, 1H), 2.50 (s, 3H).

### <Preparation Example 6> Preparation of Example 18 (compound represented by Formula 20; AMD-D-258)

The compound of Example 18 was synthesized according to Reaction Scheme 11 below.

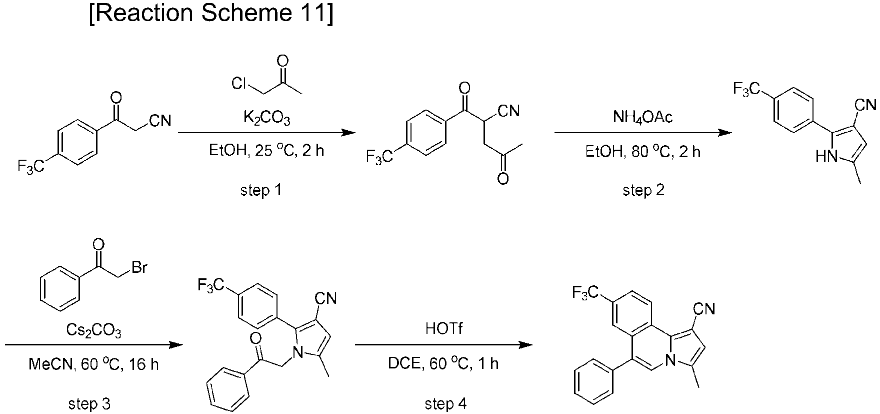

### Step 1. Preparation of 4-oxo-2-(4-(trifluoromethyl)benzoyl)pentanenitrile)

3-Oxo-3-[4-(trifluoromethyl)phenyl]propanenitrile (5 g, 0.023 mol) and potassium carbonate (8.12 g, 0.058 mol) were mixed in ethanol (150 mL), and 1-chloropropan-2-one (2.39 g, 0.025 mol) was added thereto. The mixture was stirred at 25 °C for 2 hours. Water (100 mL) was added to the reaction mixture, followed by extraction with dichloromethane (100 mL × 3). The organic layers were combined, washed with saturated sodium chloride solution, dried over sodium sulfate, and concentrated. The concentrate was purified by silica gel column chromatography (ethyl acetate/petroleum ether, volume ratio = 15:85) to obtain 4-oxo-2-(4-(trifluoromethyl)benzoyl)pentanenitrile (5 g, yield 79 %) as a yellow solid.

### Step 2. Preparation of 5-methyl-2-(4-(trifluoromethyl)phenyl)-1H-pyrrole-3-carbonitrile)

4-Oxo-2-(4-(trifluoromethyl)benzoyl)pentanenitrile (2.5 g, 0.009 mol) and ammonium acetate (1.51 g, 0.019 mol) were mixed in ethanol (50 mL), and the mixture was stirred at 80 °C for 2 hours. Water (50 mL) was added to the reaction mixture, followed by extraction with dichloromethane (100 mL × 3). The organic layers were combined, washed with saturated sodium chloride solution, dried over sodium sulfate, and concentrated. The concentrate was purified by silica gel column chromatography (ethyl acetate/petroleum ether, volume ratio = 15:85) to obtain 5-methyl-2-(4-(trifluoromethyl)phenyl)-1H-pyrrole-3-carbonitrile (1.1 g, yield 43 %) as a white solid.

### Step 3. Preparation of 5-methyl-1-(2-oxo-2-phenylethyl)-2-(4-(trifluoromethyl)phenyl)-1H-pyrrole-3-carbonitrile)

5-Methyl-2-(4-(trifluoromethyl)phenyl)-1H-pyrrole-3-carbonitrile (200 mg, 0.799 mmol) and 2-bromo-1-phenylethan-1-one (190.92 mg, 0.959 mmol) were mixed in acetonitrile (9 mL), and cesium carbonate (520.86 mg, 1.598 mmol) was added thereto. The mixture was stirred at 60 °C for 16 hours. Water (10 mL) was added to the reaction mixture, followed by extraction with dichloromethane (20 mL × 3). The organic layers were combined, washed with saturated sodium chloride solution, dried over sodium sulfate, and concentrated. The concentrate was purified by silica gel column chromatography (ethyl acetate/petroleum ether, volume ratio = 8:92) to obtain 5-methyl-1-(2-oxo-2-phenylethyl)-2-(4-(trifluoromethyl)phenyl)-1H-pyrrole-3-carbonitrile (168 mg, yield 45 %) as a yellow solid.

### Step 4. Preparation of 3-methyl-6-phenyl-8-(trifluoromethyl)pyrrolo[2,1-a]isoquinoline-1-carbonitrile (Example 18)

5-Methyl-1-(2-oxo-2-phenylethyl)-2-(4-(trifluoromethyl)phenyl)-1H-pyrrole-3-carbonitrile (100 mg, 0.271 mmol), trifluoromethanesulfonic acid (407.47 mg, 2.715 mmol), and 1,2-dichloroethane (5 mL) were mixed, and the mixture was stirred at 60 °C for 1 hour. Water (10 mL) was added to the reaction mixture, followed by extraction with dichloromethane (15 mL × 3). The organic layers were combined, washed with saturated sodium chloride solution, dried over sodium sulfate, and concentrated. The concentrate was purified by silica gel column chromatography (ethyl acetate/petroleum ether, volume ratio = 5:95) to obtain 3-methyl-6-phenyl-8-(trifluoromethyl)pyrrolo[2,1-a]isoquinoline-1-carbonitrile (27 mg, yield 27 %) as a white solid.

LCMS (ESI-MS): mass calcd C₂₁H₁₃F₃N₂350.1 m/z, found 350.7 [M+H]⁺.

¹H NMR (400 MHz, CDCl₃) δ 9.00 (d, J = 8.4 Hz, 1H), 7.89 (s, 1H), 7.84-7.81 (m, 1H), 7.70 (s, 1H), 7.58-7.51 (m, 3H), 7.48-7.46 (m, 2H), 6.84 (s, 1H), 2.52 (s, 3H).

### <Preparation Example 7> Preparation of Example 19 (compound represented by Formula 21; AMD-D-259)

The compound of Example 19 was synthesized according to Reaction Scheme 12 below.

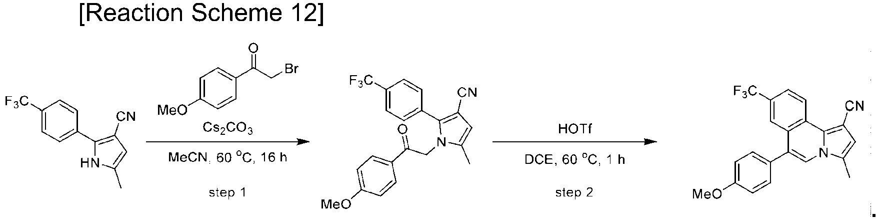

### Step 1. Preparation of 1-(2-(4-methoxyphenyl)-2-oxoethyl)-5-methyl-2-(4-(trifluoromethyl)phenyl)-1H-pyrrole-3-carbonitrile)

5-Methyl-2-(4-(trifluoromethyl)phenyl)-1H-pyrrole-3-carbonitrile (200 mg, 0.799 mmol), which is the intermediate of Step 2 of Example 18, and 2-bromo-1-(4-methoxyphenyl)ethan-1-one (219.72 mg, 0.959 mmol) were mixed in acetonitrile (9 mL), and cesium carbonate (520.86 mg, 1.598 mmol) was added thereto. The mixture was stirred at 60 °C for 16 hours. Water (10 mL) was added to the reaction mixture, followed by extraction with dichloromethane (20 mL × 3). The organic layers were combined, washed with saturated sodium chloride solution, dried over sodium sulfate, and concentrated. The concentrate was purified by silica gel column chromatography (ethyl acetate/petroleum ether, volume ratio = 8:92) to obtain 1-(2-(4-methoxyphenyl)-2-oxoethyl)-5-methyl-2-(4-(trifluoromethyl)phenyl)-1H-pyrrole-3-carbonitrile (150 mg, yield 37 %) as a yellow solid.

### Step 2. Preparation of 6-(4-methoxyphenyl)-3-methyl-8-(trifluoromethyl)pyrrolo[2,1-a]isoquinoline-1-carbonitrile (Example 19)

1-(2-(4-Methoxyphenyl)-2-oxoethyl)-5-methyl-2-(4-(trifluoromethyl)phenyl)-1H-pyrrole-3-carbonitrile (100 mg, 0.251 mmol), trifluoromethanesulfonic acid (376.7 mg, 2.51 mmol), and 1,2-dichloroethane (5 mL) were mixed, and the mixture was stirred at 60 °C for 1 hour. Water (10 mL) was added to the reaction mixture, followed by extraction with dichloromethane (15 mL × 3). The organic layers were combined, washed with saturated sodium chloride solution, dried over sodium sulfate, and concentrated. The concentrate was purified by silica gel column chromatography (ethyl acetate/petroleum ether, volume ratio = 5:95) to obtain 6-(4-methoxyphenyl)-3-methyl-8-(trifluoromethyl)pyrrolo[2,1-a]isoquinoline-1-carbonitrile (14 mg, yield 14 %) as a white solid.

LCMS (ESI-MS): mass calcd C₂₂H₁₅F₃N₂₀ 380.1 m/z, found 380.7 [M+H]⁺.

¹H NMR (400 MHz, CDCl₃) δ 8.99 (d, J = 8.4 Hz, 1H), 7.91 (s, 1H), 7.83-7.80 (m, 1H), 7.67 (s, 1H), 7.43-7.36 (m, 2H), 7.12-7.05 (m, 2H), 6.83 (s, 1H), 3.92 (s, 3H), 2.51 (s, 3H).

### <Preparation Example 8> Preparation of Example 20 (compound represented by Formula 22; AMD-D-260)

The compound of Example 20 was synthesized according to Reaction Scheme 13 below.

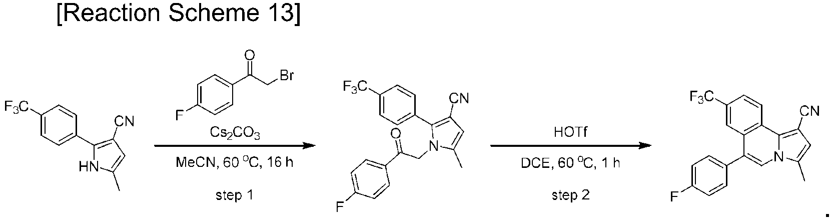

### Step 1. Preparation of 1-(2-(4-fluorophenyl)-2-oxoethyl)-5-methyl-2-(4-(trifluoromethyl)phenyl)-1H-pyrrole-3-carbonitrile)

5-Methyl-2-(4-(trifluoromethyl)phenyl)-1H-pyrrole-3-carbonitrile (200 mg, 0.799 mmol), which is the intermediate of Step 2 of Example 18, and 2-bromo-1-(4-fluorophenyl)ethan-1-one (208.17 mg, 0.959 mmol) were mixed in acetonitrile (9 mL), and cesium carbonate (520.86 mg, 1.598 mmol) was added thereto. The mixture was stirred at 60 °C for 16 hours. Water (10 mL) was added to the reaction mixture, followed by extraction with dichloromethane (20 mL × 3). The organic layers were combined, washed with saturated sodium chloride solution, dried over sodium sulfate, and concentrated. The concentrate was purified by silica gel column chromatography (ethyl acetate/petroleum ether, volume ratio = 10:90) to obtain 1-(2-(4-fluorophenyl)-2-oxoethyl)-5-methyl-2-(4-(trifluoromethyl)phenyl)-1H-pyrrole-3-carbonitrile (100 mg, yield 29 %) as a yellow solid.

### Step 2. Preparation of 6-(4-fluorophenyl)-3-methyl-8-(trifluoromethyl)pyrrolo[2,1-a]isoquinoline-1-carbonitrile (Example 20)

1-(2-(4-Fluorophenyl)-2-oxoethyl)-5-methyl-2-(4-(trifluoromethyl)phenyl)-1H-pyrrole-3-carbonitrile (100 mg, 0.258 mmol), trifluoromethanesulfonic acid (388.41 mg, 2.588 mmol), and 1,2-dichloroethane (5 mL) were mixed, and the mixture was stirred at 60 °C for 1 hour. Water (10 mL) was added to the reaction mixture, followed by extraction with dichloromethane (15 mL × 3). The organic layers were combined, washed with saturated sodium chloride solution, dried over sodium sulfate, and concentrated. The concentrate was purified by silica gel column chromatography (ethyl acetate/petroleum ether, volume ratio = 6:94) to obtain 6-(4-fluorophenyl)-3-methyl-8-(trifluoromethyl)pyrrolo[2,1-a]isoquinoline-1-carbonitrile (10 mg, yield 10 %) as a white solid.

LCMS (ESI-MS): mass calcd C₂₁H₁₂F₄N₂ 368.1 m/z, found 368.7 [M+H]⁺.

¹H NMR (400 MHz, CDCl₃) δ 9.00 (d, J = 8.4 Hz, 1H), 7.84-7.82 (m, 2H), 7.68 (s, 1H), 7.48-7.40 (m, 2H), 7.28-7.27 (m, 1H), 7.24 (s, 1H), 6.85 (s, 1H), 2.53 (s, 3H).

### <Preparation Example 9> Preparation of Example 21 (compound represented by Formula 23; AMD-D-262)

The compound of Example 21 was synthesized according to Reaction Scheme 14 below.

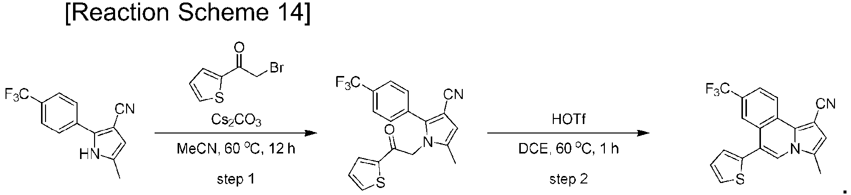

### Step 1. Preparation of 5-methyl-1-(2-oxo-2-(thiophen-2-yl)ethyl)-2-(4-(trifluoromethyl)phenyl)-1H-pyrrole-3-carbonitrile)

5-Methyl-2-(4-(trifluoromethyl)phenyl)-1H-pyrrole-3-carbonitrile (200 mg, 0.799 mmol), which is the intermediate of Step 2 of Example 18, and 2-bromo-1-(thiophen-2-yl)ethan-1-one (196.7 mg, 0.959 mmol) were mixed in acetonitrile (9 mL), and cesium carbonate (520.86 mg, 1.598 mmol) was added thereto. The mixture was stirred at 60 °C for 16 hours. Water (10 mL) was added to the reaction mixture, followed by extraction with dichloromethane (20 mL × 3). The organic layers were combined, washed with saturated sodium chloride solution, dried over sodium sulfate, and concentrated. The concentrate was purified by silica gel column chromatography (ethyl acetate/petroleum ether, volume ratio = 10:90) to obtain 5-methyl-1-(2-oxo-2-(thiophen-2-yl)ethyl)-2-(4-(trifluoromethyl)phenyl)-1H-pyrrole-3-carbonitrile (80 mg, yield 24 %) as a yellow solid.

### Step 2. Preparation of 3-methyl-6-(thiophen-2-yl)-8-(trifluoromethyl)pyrrolo[2,1-a]isoquinoline-1-carbonitrile (Example 21)

5-Methyl-1-(2-oxo-2-(thiophen-2-yl)ethyl)-2-(4-(trifluoromethyl)phenyl)-1H-pyrrole-3-carbonitrile (80 mg, 0.213 mmol), trifluoromethanesulfonic acid (320.72 mg, 2.137 mmol), and 1,2-dichloroethane (4 mL) were mixed, and the mixture was stirred at 60 °C for 1 hour. Water (10 mL) was added to the reaction mixture, followed by extraction with dichloromethane (15 mL × 3). The organic layers were combined, washed with saturated sodium chloride solution, dried over sodium sulfate, and concentrated. The concentrate was purified by silica gel column chromatography (ethyl acetate/petroleum ether, volume ratio = 5:95) to obtain 3-methyl-6-(thiophen-2-yl)-8-(trifluoromethyl)pyrrolo[2,1-a]isoquinoline-1-carbonitrile (11 mg, yield 13 %) as a white solid.

LCMS (ESI-MS): mass calcd C₁₉H₁₁F₃N₂S 356.1 m/z, found 356.7 [M+H]⁺.

¹H NMR (400 MHz, CDCl₃) δ 8.99 (d, J = 8.8 Hz, 1H), 8.18 (s, 1H), 7.85-7.84 (m, 2H), 7.52-7.51 (m, 1H), 7.27 (s, 1H), 7.24-7.23 (m, 1H), 6.84 (s, 1H), 2.53 (s, 3H).

### <Preparation Example 10> Preparation of Example 22 (compound represented by Formula 24; IK15g)

The compound of Example 22 was synthesized according to Reaction Scheme 15 below.

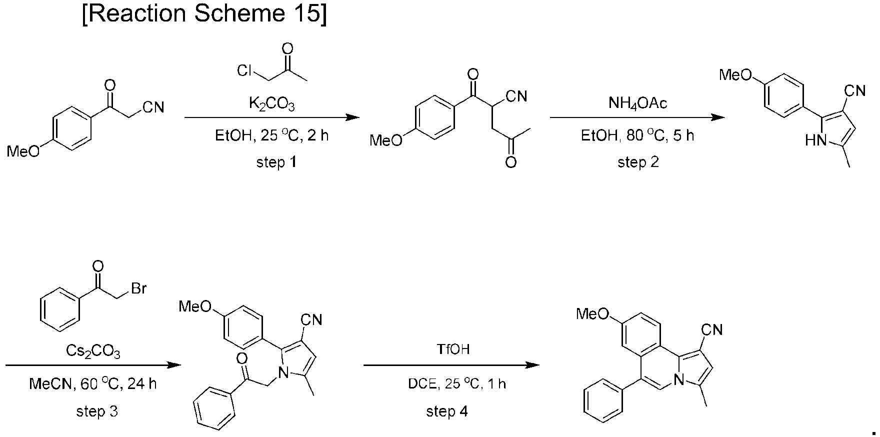

### Step 1. Preparation of 2-(4-methoxybenzoyl)-4-oxopentanenitrile)

3-(4-Methoxyphenyl)-3-oxopropanenitrile (2 g, 0.0114 mol), 1-chloropropan-2-one (1.16 g, 0.0125 mol), and potassium carbonate (3.94 g, 0.0285 mol) were mixed in ethanol (24 mL), and the mixture was stirred at 25 °C for 2 hours. Water (100 mL) was added to the reaction mixture, followed by extraction with dichloromethane (100 mL × 3). The organic layers were combined, washed with saturated sodium chloride solution, dried over sodium sulfate, and concentrated. The concentrate was purified by silica gel column chromatography (ethyl acetate/hexane, volume ratio = 40:60) to obtain 2-(4-methoxybenzoyl)-4-oxopentanenitrile (2.3 g, yield 78 %) as a white solid.

### Step 2. Preparation of 2-(4-methoxyphenyl)-5-methyl-1H-pyrrole-3-carbonitrile)

2-(4-Methoxybenzoyl)-4-oxopentanenitrile (1.8 g, 0.0078 mol) and ammonium acetate (1.26 g, 0.0164 mol) were mixed in ethanol (15 mL), and the mixture was stirred at 80 °C for 5 hours. Water (200 mL) was added to the reaction mixture, followed by extraction with ethyl acetate (200 mL × 3). The organic layers were combined, washed with saturated sodium chloride solution, dried over sodium sulfate, and concentrated. The concentrate was purified by silica gel column chromatography (ethyl acetate/hexane, volume ratio = 30:70) to obtain 2-(4-methoxyphenyl)-5-methyl-1H-pyrrole-3-carbonitrile (1.7 g, yield 92 %) as a yellow solid.

### Step 3. Preparation of 2-(4-methoxyphenyl)-5-methyl-1-(2-oxo-2-phenylethyl)-1H-pyrrole-3-carbonitrile)

2-(4-Methoxyphenyl)-5-methyl-1H-pyrrole-3-carbonitrile (200 mg, 0.9423 mmol), 2-bromo-1-phenylethan-1-one (225.07 mg, 1.1307 mmol), and cesium carbonate (614.04 mg, 1.8846 mmol) were mixed in acetonitrile (5 mL), and the mixture was stirred at 60 °C for 24 hours. The reaction mixture was concentrated under reduced pressure, and the residue was purified by silica gel column chromatography (ethyl acetate/hexane, volume ratio = 30:70) to obtain 2-(4-methoxyphenyl)-5-methyl-1-(2-oxo-2-phenylethyl)-1H-pyrrole-3-carbonitrile (140 mg, yield 40 %) as a yellow solid.

### Step 4. Preparation of 8-methoxy-3-methyl-6-phenylpyrrolo[2,1-a]isoquinoline-1-carbonitrile (Example 22)

2-(4-Methoxyphenyl)-5-methyl-1-(2-oxo-2-phenylethyl)-1H-pyrrole-3-carbonitrile (70 mg, 0.2119 mmol), trifluoromethanesulfonic acid (318.02 mg, 2.119 mmol), and 1,2-dichloroethane (6 mL) were mixed, and the mixture was stirred at 25 °C for 1 hour. The reaction was quenched with saturated aqueous sodium bicarbonate solution, and the mixture was extracted with dichloromethane. The organic layers were combined, washed with saturated sodium chloride solution, dried over sodium sulfate, and concentrated. The residue was purified by Prep-HPLC (Gemini 5 µm C18, 150 × 21.2 mm, 70 - 95 % acetonitrile/0.1 % formic acid) to obtain 8-methoxy-3-methyl-6-phenylpyrrolo[2,1-a]isoquinoline-1-carbonitrile (AMD-D-263) (23 mg, yield 33 %) as a white solid.

LCMS (ESI-MS): mass calcd C₂₁H₁₆N₂₀ 312.1 m/z, found 312.7 [M+H]⁺.

¹H NMR (400 MHz, CDCl₃) δ 8.84 (d, J = 9.0 Hz, 1H), 7.59 (s, 1H), 7.56-7.49 (m, 5H), 7.26-7.22 (m, 1H), 7.05 (s, 1H), 6.71 (s, 1H), 3.79 (s, 3H), 2.46 (s, 3H).

### <Preparation Example 11> Preparation of Example 23 (compound represented by Formula 25; AMD-D-264)

The compound of Example 23 was synthesized according to Reaction Scheme 16 below.

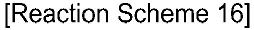

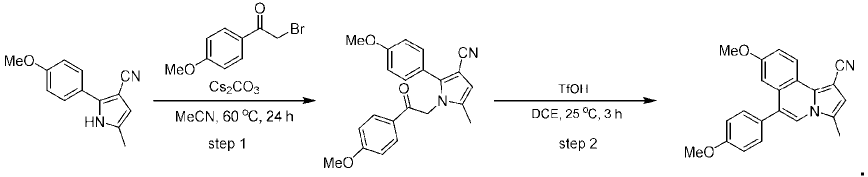

### Step 1. Preparation of 2-(4-methoxyphenyl)-1-(2-(4-methoxyphenyl)-2-oxoethyl)-5-methyl-1H-pyrrole-3-carbonitrile)

2-(4-Methoxyphenyl)-5-methyl-1H-pyrrole-3-carbonitrile (200 mg, 0.9423 mmol), which is the intermediate of Step 2 of Example 22, 2-bromo-1-(4-methoxyphenyl)ethan-1-one (259.0 mg, 1.1307 mmol), and cesium carbonate (614.04 mg, 1.8846 mmol) were mixed in acetonitrile (5 mL), and the mixture was stirred at 60 °C for 24 hours. The reaction mixture was concentrated under reduced pressure, and the residue was purified by silica gel column chromatography (ethyl acetate/hexane, volume ratio = 30:70) to obtain 2-(4-methoxyphenyl)-1-(2-(4-methoxyphenyl)-2-oxoethyl)-5-methyl-1H-pyrrole-3-carbonitrile (340 mg, yield 80 %) as a yellow solid.

### Step 2. Preparation of 8-methoxy-6-(4-methoxyphenyl)-3-methylpyrrolo[2,1-a]isoquinoline-1-carbonitrile (Example 23)

2-(4-Methoxyphenyl)-1-(2-(4-methoxyphenyl)-2-oxoethyl)-5-methyl-1H-pyrrole-3-carbonitrile (100 mg, 0.2775 mmol), trifluoromethanesulfonic acid (416.47 mg, 2.775 mmol), and 1,2-dichloroethane (6 mL) were mixed, and the mixture was stirred at 25 °C for 3 hours. The reaction was quenched with saturated aqueous sodium bicarbonate solution, and the mixture was extracted with dichloromethane. The organic layers were combined, washed with saturated sodium chloride solution, dried over sodium sulfate, and concentrated. The residue was purified by Prep-HPLC (Gemini 5 µm C18, 150 × 21.2 mm, 70 - 95 % acetonitrile/0.1 % formic acid) to obtain 8-methoxy-6-(4-methoxyphenyl)-3-methylpyrrolo[2,1-a]isoquinoline-1-carbonitrile (AMD-D-264) (17 mg, yield 17 %) as a white solid.

LCMS (ESI-MS): mass calcd C₂₂H₁₈N₂O₂ 342.1 m/z, found 342.7 [M+H]⁺.

¹H NMR (400 MHz, CDCl₃) δ 8.83 (d, J = 9.0 Hz, 1H), 7.57 (s, 1H), 7.43-7.37 (m, 2H), 7.24 (m, 1H), 7.09-7.02 (m, 3H), 6.70 (s, 1H), 3.91 (s, 3H), 3.79 (s, 3H), 2.46 (s, 3H).

### <Preparation Example 12> Preparation of Example 24 (compound represented by Formula 26; AMD-D-265)

The compound of Example 24 was synthesized according to Reaction Scheme 17 below.

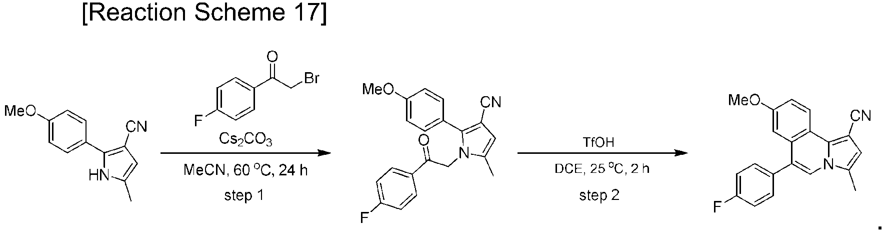

### Step 1. Preparation of 1-(2-(4-fluorophenyl)-2-oxoethyl)-2-(4-methoxyphenyl)-5-methyl-1H-pyrrole-3-carbonitrile)

2-(4-Methoxyphenyl)-5-methyl-1H-pyrrole-3-carbonitrile (200 mg, 0.9423 mmol), which is the intermediate of Step 2 of Example 22, 2-bromo-1-(4-fluorophenyl)ethan-1-one (245.41 mg, 1.1307 mmol), and cesium carbonate (614.04 mg, 1.8846 mmol) were mixed in acetonitrile (15 mL), and the mixture was stirred at 60 °C for 24 hours. The reaction mixture was concentrated under reduced pressure, and the residue was purified by silica gel column chromatography (ethyl acetate/hexane, volume ratio = 30:70) to obtain 1-(2-(4-fluorophenyl)-2-oxoethyl)-2-(4-methoxyphenyl)-5-methyl-1H-pyrrole-3-carbonitrile (100 mg, yield 27 %) as a yellow oil.

### Step 2. Preparation of 6-(4-fluorophenyl)-8-methoxy-3-methylpyrrolo[2,1-a]isoquinoline-1-carbonitrile (Example 24)

1-(2-(4-Fluorophenyl)-2-oxoethyl)-2-(4-methoxyphenyl)-5-methyl-1H-pyrrole-3-carbonitrile (80 mg, 0.2296 mmol), trifluoromethanesulfonic acid (344.58 mg, 2.296 mmol), and 1,2-dichloroethane (6 mL) were mixed, and the mixture was stirred at 25 °C for 2 hours. The reaction was quenched with saturated aqueous sodium bicarbonate solution, and the mixture was extracted with dichloromethane. The organic layers were combined, washed with saturated sodium chloride solution, dried over sodium sulfate, and concentrated. The residue was purified by Prep-HPLC (Gemini 5 µm C18, 150 × 21.2 mm, 70 - 95 % acetonitrile/0.1 % formic acid) to obtain 6-(4-fluorophenyl)-8-methoxy-3-methylpyrrolo[2,1-a]isoquinoline-1-carbonitrile (14.3 mg, yield 18 %) as a white solid.

LCMS (ESI-MS): mass calcd C₂₁H₁₅FN₂O 330.1 m/z, found 330.7 [M+H]⁺.

¹H NMR (400 MHz, CDCl₃) δ 8.84 (d, J = 9.0 Hz, 1H), 7.57 (s, 1H), 7.48-7.42 (m, 2H), 7.24-7.18 (m, 3H), 6.97 (s, 1H), 6.71 (s, 1H), 3.80 (s, 3H), 2.47 (s, 3H).

### <Preparation Example 13> Preparation of Example 25 (compound represented by Formula 27; AMD-D-267)

The compound of Example 25 was synthesized according to Reaction Scheme 18 below.

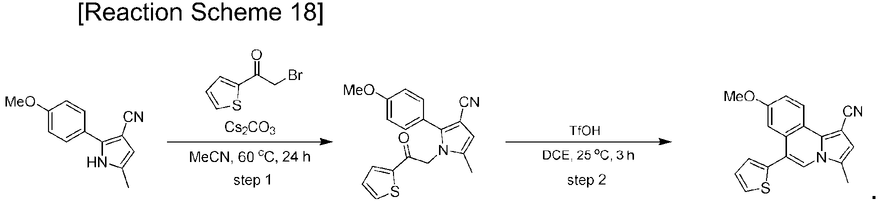

### Step 1. Preparation of 2-(4-methoxyphenyl)-5-methyl-1-(2-oxo-2-(thiophen-2-yl)ethyl)-1H-pyrrole-3-carbonitrile

2-(4-Methoxyphenyl)-5-methyl-1H-pyrrole-3-carbonitrile (350 mg, 1.649 mmol), which is the intermediate of Step 2 of Example 22, 2-bromo-1-(thiophen-2-yl)ethan-1-one (405.8 mg, 1.979 mmol), and cesium carbonate (1074.5 mg, 3.298 mmol) were mixed in acetonitrile (15 mL), and the mixture was stirred at 60 °C for 24 hours. The reaction mixture was concentrated under reduced pressure, and the residue was purified by silica gel column chromatography (ethyl acetate/hexane, volume ratio = 30:70) to obtain 2-(4-methoxyphenyl)-5-methyl-1-(2-oxo-2-(thiophen-2-yl)ethyl)-1H-pyrrole-3-carbonitrile (100 mg, yield 16 %) as a yellow oil.

### Step 2. Preparation of 8-methoxy-3-methyl-6-(thiophen-2-yl)pyrrolo[2,1-a]isoquinoline-1-carbonitrile (Example 25)

2-(4-Methoxyphenyl)-5-methyl-1-(2-oxo-2-(thiophen-2-yl)ethyl)-1H-pyrrole-3-carbonitrile (90 mg, 0.2675 mmol), trifluoromethanesulfonic acid (401.5 mg, 2.675 mmol), and 1,2-dichloroethane (6 mL) were mixed, and the mixture was stirred at 25 °C for 3 hours. The reaction was quenched with saturated aqueous sodium bicarbonate solution, and the mixture was extracted with dichloroethane. The organic layers were combined, washed with saturated sodium chloride solution, dried over sodium sulfate, and concentrated. The residue was purified by Prep-HPLC (Gemini 5 µm C18, 150 × 21.2 mm, 60 - 95 % acetonitrile/0.1 % formic acid) to obtain 8-methoxy-3-methyl-6-(thiophen-2-yl)pyrrolo[2,1-a]isoquinoline-1-carbonitrile (15 mg, yield 17 %) as a white solid.

LCMS (ESI-MS): mass calcd C₁₉H₁₄N₂OS 318.1 m/z, found 319.1 [M+H]⁺.

¹H NMR (400 MHz, CDCl₃) δ 8.82 (d, J = 9.0 Hz, 1H), 7.74 (s, 1H), 7.49-7.47 (m, 1H), 7.35 (s, 1H), 7.27 (s, 1H), 7.25-7.21 (m, 2H), 6.70 (s, 1H), 3.84 (s, 3H), 2.47 (s, 3H).

### <Preparation Example 14> Preparation of Example 26 (compound represented by Formula 28; IK15j)

The compound of Example 26 was synthesized according to Reaction Scheme 19 below.

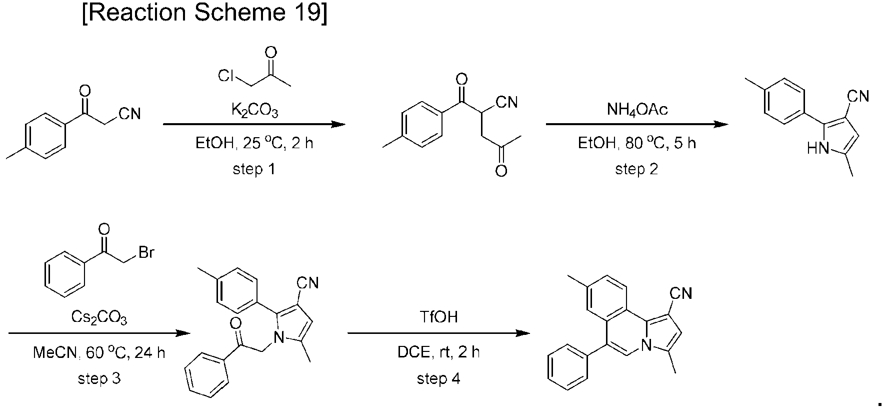

### Step 1. Preparation of 2-(4-methylbenzoyl)-4-oxopentanenitrile

3-Oxo-3-(p-tolyl)propanenitrile (5 g, 31.44 mmol) and 1-chloropropan-2-one (4.3 g, 47.17 mmol) were mixed in ethanol (80 mL), and potassium carbonate (8.7 g, 62.88 mmol) was added thereto. The mixture was stirred under nitrogen at 25 °C for 2 hours. Water (50 mL) was added to the reaction mixture, followed by extraction with ethyl acetate (120 mL × 3). The organic layers were combined, washed with saturated sodium chloride solution, dried over sodium sulfate, and concentrated. The residue was purified by silica gel column chromatography (ethyl acetate/petroleum ether, volume ratio = 5:95) to obtain 2-(4-methylbenzoyl)-4-oxopentanenitrile (3.1 g, yield 49 %).

### Step 2. Preparation of 5-methyl-2-(p-tolyl)-1H-pyrrole-3-carbonitrile

2-(4-Methylbenzoyl)-4-oxopentanenitrile (3.1 g, 14.42 mmol) and ammonium acetate (2.2 g, 28.83 mmol) were mixed in ethanol (50 mL), and the mixture was stirred at 80 °C for 5 hours. Water (50 mL) was added to the reaction mixture, followed by extraction with dichloromethane (100 mL × 3). The organic layers were combined, washed with saturated sodium chloride solution, dried over sodium sulfate, and concentrated. The residue was purified by silica gel column chromatography (ethyl acetate/petroleum ether, volume ratio = 10:90) to obtain 5-methyl-2-(p-tolyl)-1H-pyrrole-3-carbonitrile (2.0 g, yield 71 %) as a yellow solid.

### Step 3. Preparation of 5-methyl-1-(2-oxo-2-phenylethyl)-2-(p-tolyl)-1H-pyrrole-3-carbonitrile

5-Methyl-2-(p-tolyl)-1H-pyrrole-3-carbonitrile (1.0 g, 5.1 mmol) and 2-bromo-1-phenylethan-1-one (2.0 g, 10.2 mmol) were mixed in acetonitrile (60 mL), and cesium carbonate (3.3 g, 10.2 mmol) was added thereto. The mixture was stirred at 60 °C for 24 hours. Water (40 mL) was added to the reaction mixture, followed by extraction with dichloromethane (80 mL × 3). The organic layers were combined, washed with saturated sodium chloride solution, dried over sodium sulfate, and concentrated. The residue was purified by silica gel column chromatography (ethyl acetate/petroleum ether, volume ratio = 15:85) to obtain 5-methyl-1-(2-oxo-2-phenylethyl)-2-(p-tolyl)-1H-pyrrole-3-carbonitrile (0.5 g, yield 31 %) as a yellow solid.

### Step 4. Preparation of 3,8-dimethyl-6-phenylpyrrolo[2,1-a]isoquinoline-1-carbonitrile (Example 26)

5-Methyl-1-(2-oxo-2-phenylethyl)-2-(p-tolyl)-1H-pyrrole-3-carbonitrile (500 mg, 1.6 mmol), trifluoromethanesulfonic acid (2.4 g, 16.0 mmol), and 1,2-dichloroethane (30 mL) were mixed, and the mixture was stirred at room temperature for 2 hours. Water (25 mL) was added to the reaction mixture, followed by extraction with dichloromethane (50 mL × 3). The organic layers were combined, washed with saturated sodium chloride solution, dried over sodium sulfate, and concentrated. The residue was purified by silica gel column chromatography (ethyl acetate/petroleum ether, volume ratio = 8:92) to obtain 3,8-dimethyl-6-phenylpyrrolo[2,1-a]isoquinoline-1-carbonitrile (AMD-D-268) (300 mg, yield 63 %) as a white solid.

LCMS (ESI-MS): mass calcd C₂₁H₁₆N₂ 296.1 m/z, found 296.7 [M+H]⁺.

¹H NMR (400 MHz, CDCl₃) δ 8.80 (d, J = 8.4 Hz, 1H), 7.66-7.35 (m, 8H), 6.73 (s, 1H), 2.47 (s, 3H), 2.42 (s, 3H).

### <Preparation Example 15> Preparation of Example 27 (compound represented by Formula 29; IK15k)

The compound of Example 27 was synthesized according to Reaction Scheme 20 below.

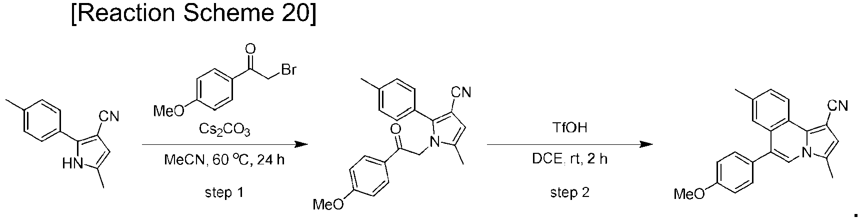

### Step 1. Preparation of 1-(2-(4-methoxyphenyl)-2-oxoethyl)-5-methyl-2-(p-tolyl)-1H-pyrrole-3-carbonitrile

5-Methyl-2-(p-tolyl)-1H-pyrrole-3-carbonitrile (250 mg, 1.28 mmol), which is the intermediate of Step 2 of Example 26, and 2-bromo-1-(4-methoxyphenyl)ethan-1-one (440 mg, 1.92 mmol) were mixed in acetonitrile (15 mL), and cesium carbonate (832 mg, 2.56 mmol) was added thereto. The mixture was stirred at 60 °C for 24 hours. Water (10 mL) was added to the reaction mixture, followed by extraction with dichloromethane (20 mL × 3). The organic layers were combined, washed with saturated sodium chloride solution, dried over sodium sulfate, and concentrated. The residue was purified by silica gel column chromatography (ethyl acetate/petroleum ether, volume ratio = 15:85) to obtain 1-(2-(4-methoxyphenyl)-2-oxoethyl)-5-methyl-2-(p-tolyl)-1H-pyrrole-3-carbonitrile (120 mg, yield 27 %) as a yellow solid.

### Step 2. Preparation of 6-(4-methoxyphenyl)-3,8-dimethylpyrrolo[2,1-a]isoquinoline-1-carbonitrile (Example 27)

1-(2-(4-Methoxyphenyl)-2-oxoethyl)-5-methyl-2-(p-tolyl)-1H-pyrrole-3-carbonitrile (50 mg, 0.15 mmol), trifluoromethanesulfonic acid (225 mg, 1.5 mmol), and 1,2-dichloroethane (2 mL) were mixed, and the mixture was stirred at room temperature for 2 hours. Water (4 mL) was added to the reaction mixture, followed by extraction with dichloromethane (10 mL × 3). The organic layers were combined, washed with saturated sodium chloride solution, dried over sodium sulfate, and concentrated. The residue was purified by silica gel column chromatography (ethyl acetate/petroleum ether, volume ratio = 8:92) to obtain 6-(4-methoxyphenyl)-3,8-dimethylpyrrolo[2,1-a]isoquinoline-1-carbonitrile (10.7 mg, yield 22 %) as a white solid.

LCMS (ESI-MS): mass calcd C₂₂H₁₈N₂₀ 326.1 m/z, found 326.8 [M+H]⁺.

¹H NMR (400 MHz, CDCl₃) δ 8.79 (d, J = 8.4 Hz, 1H), 7.56 (s, 1H), 7.48-7.33 (m, 4H), 7.09-7.01 (m, 2H), 6.72 (s, 1H), 3.91 (s, 3H), 3.00-2.25 (m, 6H).

### <Preparation Example 16> Preparation of Example 28 (compound represented by Formula 30; AMD-D-270)

The compound of Example 28 was synthesized according to Reaction Scheme 21 below.

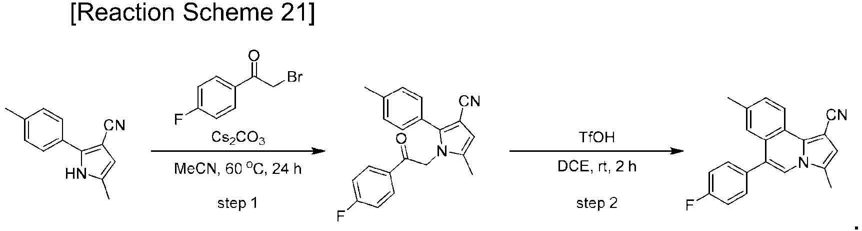

### Step 1. Preparation of 1-(2-(4-fluorophenyl)-2-oxoethyl)-5-methyl-2-(p-tolyl)-1H-pyrrole-3-carbonitrile

5-Methyl-2-(p-tolyl)-1H-pyrrole-3-carbonitrile (250 mg, 1.28 mmol), which is the intermediate of Step 2 of Example 26, and 2-bromo-1-(4-fluorophenyl)ethan-1-one (416 mg, 1.92 mmol) were mixed in acetonitrile (15 mL), and cesium carbonate (832 mg, 2.56 mmol) was added thereto. The mixture was stirred at 60 °C for 24 hours. Water (10 mL) was added to the reaction mixture, followed by extraction with dichloromethane (20 mL × 3). The organic layers were combined, washed with saturated sodium chloride solution, dried over sodium sulfate, and concentrated. The residue was purified by silica gel column chromatography (ethyl acetate/petroleum ether, volume ratio = 15:85) to obtain 1-(2-(4-fluorophenyl)-2-oxoethyl)-5-methyl-2-(p-tolyl)-1H-pyrrole-3-carbonitrile (110 mg, yield 26 %) as a yellow solid.

### Step 2. Preparation of 6-(4-fluorophenyl)-3,8-dimethylpyrrolo[2,1-a]isoquinoline-1-carbonitrile (Example 28)

1-(2-(4-Fluorophenyl)-2-oxoethyl)-5-methyl-2-(p-tolyl)-1H-pyrrole-3-carbonitrile (50 mg, 0.15 mmol), trifluoromethanesulfonic acid (225 mg, 1.5 mmol), and 1,2-dichloroethane (2 mL) were mixed, and the mixture was stirred at room temperature for 2 hours. Water (4 mL) was added to the reaction mixture, followed by extraction with dichloromethane (10 mL × 3). The organic layers were combined, washed with saturated sodium chloride solution, dried over sodium sulfate, and concentrated. The residue was purified by silica gel column chromatography (ethyl acetate/petroleum ether, volume ratio = 9:91) to obtain 6-(4-fluorophenyl)-3,8-dimethylpyrrolo[2,1-a]isoquinoline-1-carbonitrile (10.9 mg, yield 23 %) as a white solid.

LCMS (ESI-MS): mass calcd C₂₁H₁₅FN₂ 314.1 m/z, found 314.7 [M+H]⁺.

¹H NMR (400 MHz, CDCl₃) δ 8.80 (d, J = 8.4 Hz, 1H), 7.56 (s, 1H), 7.50-7.38 (m, 3H), 7.33 (s, 1H), 7.25-7.14 (m, 2H), 6.74 (s, 1H), 2.48-2.43 (m, 6H).

### <Preparation Example 17> Preparation of Example 29 (compound represented by Formula 31; AMD-D-272)

The compound of Example 29 was synthesized according to Reaction Scheme 22 below.

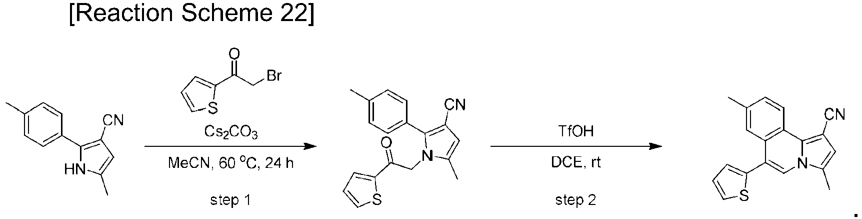

### Step 1. Preparation of 5-methyl-2-(4-methylcyclohexa-1,3-dien-1-yl)-1-(2-oxo-2-(thiophen-2-yl)ethyl)-1 H-pyrrole-3-carbonitrile

5-Methyl-2-(p-tolyl)-1H-pyrrole-3-carbonitrile (250 mg, 1.28 mmol), which is the intermediate of Step 2 of Example 26, and 2-bromo-1-(thiophen-2-yl)ethan-1-one (394 mg, 1.92 mmol) were mixed in acetonitrile (15 mL), and cesium carbonate (832 mg, 2.56 mmol) was added thereto. The mixture was stirred at 60 °C for 24 hours. Water (10 mL) was added to the reaction mixture, followed by extraction with dichloromethane (20 mL × 3). The organic layers were combined, washed with saturated sodium chloride solution, dried over sodium sulfate, and concentrated. The residue was purified by silica gel column chromatography (ethyl acetate/petroleum ether, volume ratio = 20:80) to obtain 5-methyl-2-(4-methylcyclohexa-1,3-dien-1-yl)-1-(2-oxo-2-(thiophen-2-yl)ethyl)-1H-pyrrole-3-carbonitrile (100 mg, yield 24 %) as a yellow solid.

### Step 2. Preparation of 3,8-dimethyl-6-(thiophen-2-yl)pyrrolo[2,1-a]isoquinoline-1-carbonitrile (Example 29)

5-Methyl-2-(4-methylcyclohexa-1,3-dien-1-yl)-1-(2-oxo-2-(thiophen-2-yl)ethyl)-1H-pyrrole-3-carbonitrile (50 mg, 0.16 mmol), trifluoromethanesulfonic acid (240 mg, 1.6 mmol), and 1,2-dichloroethane (2 mL) were mixed, and the mixture was stirred at room temperature for 2 hours. Water (4 mL) was added to the reaction mixture, followed by extraction with dichloromethane (10 mL × 3). The organic layers were combined, washed with saturated sodium chloride solution, dried over sodium sulfate, and concentrated. The residue was purified by silica gel column chromatography (ethyl acetate/petroleum ether, volume ratio = 9:91) to obtain 3,8-dimethyl-6-(thiophen-2-yl)pyrrolo[2,1-a]isoquinoline-1-carbonitrile (10.4 mg, yield 22 %) as a white solid.

LCMS (ESI-MS): mass calcd C₁₉H₁₄N₂S 302.1 m/z, found 302.6 [M+H]⁺.

¹H NMR (400 MHz, CDCl₃) δ 8.78 (d, J = 8.4 Hz, 1H), 7.73-7.67 (m, 2H), 7.53-7.42 (m, 2H), 7.27-7.11 (m, 2H), 6.73 (s, 1H), 2.63-2.22 (m, 6H).

### <Preparation Example 18> Preparation of Example 30 (compound represented by Formula 32; AMD-D-273)

The compound of Example 30 was synthesized according to Reaction Scheme 23 below.

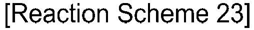

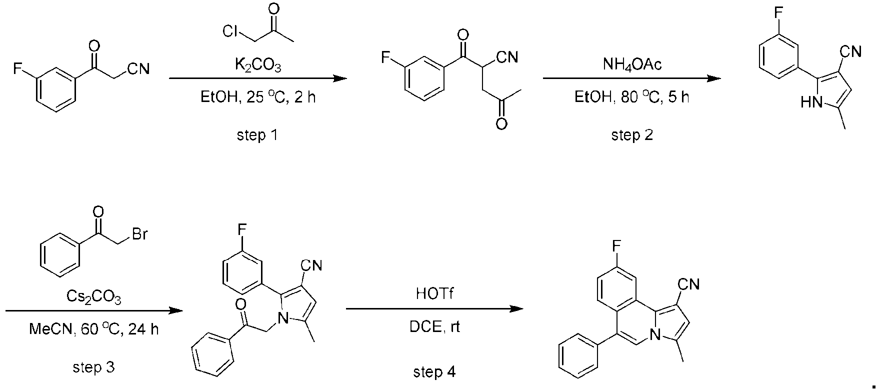

### Step 1. Preparation of 2-(3-fluorobenzoyl)-4-oxopentanenitrile

3-(3-Fluorophenyl)-3-oxopropanenitrile (3.5 g, 21.5 mmol) and potassium carbonate (7.42 g, 23.65 mmol) were mixed in ethanol (50 mL), and 1-chloropropan-2-one (2.19 g, 23.65 mmol) was added thereto. The mixture was stirred at 25 °C for 2 hours. Water (100 mL) was added to the reaction mixture, followed by extraction with dichloromethane (60 mL × 3). The organic layers were combined, washed with saturated sodium chloride solution, dried over sodium sulfate, and concentrated. The residue was purified by silica gel column chromatography (ethyl acetate/petroleum ether, volume ratio = 10:90) to obtain 2-(3-fluorobenzoyl)-4-oxopentanenitrile (4.6 g, yield 97 %) as a yellow oil.

### Step 2. Preparation of 2-(3-fluorophenyl)-5-methyl-1H-pyrrole-3-carbonitrile

2-(3-Fluorobenzoyl)-4-oxopentanenitrile (4.7 g, 21.4 mmol) and ammonium acetate (3.3 g, 42.8 mmol) were mixed in ethanol (50 mL), and the mixture was stirred at 80 °C for 5 hours. The reaction mixture was concentrated, and the residue was purified by silica gel column chromatography (ethyl acetate/petroleum ether, volume ratio = 10:90) to obtain 2-(3-fluorophenyl)-5-methyl-1H-pyrrole-3-carbonitrile (2.84 g, yield 66 %) as a yellow solid.

### Step 3. Preparation of 2-(3-fluorophenyl)-5-methyl-1-(2-oxo-2-phenylethyl)-1H-pyrrole-3-carbonitrile

2-(3-Fluorophenyl)-5-methyl-1H-pyrrole-3-carbonitrile (300 mg, 1.5 mmol), 2-bromo-1-phenylethan-1-one (450 mg, 2.25 mmol), and cesium carbonate (975 mg, 3.0 mmol) were mixed in acetonitrile (17 mL), and the mixture was stirred at 60 °C for 24 hours. Water (20 mL) was added to the reaction mixture, followed by extraction with dichloromethane (40 mL × 3). The organic layers were combined, washed with saturated sodium chloride solution, dried over sodium sulfate, and concentrated. The residue was purified by silica gel column chromatography (ethyl acetate/petroleum ether, volume ratio = 15:85) to obtain 2-(3-fluorophenyl)-5-methyl-1-(2-oxo-2-phenylethyl)-1H-pyrrole-3-carbonitrile (120 mg, yield 38 %) as a yellow solid.

### Step 4. Preparation of 9-fluoro-3-methyl-6-phenylpyrrolo[2,1-a]isoquinoline-1-carbonitrile (Example 30)

2-(3-Fluorophenyl)-5-methyl-1-(2-oxo-2-phenylethyl)-1H-pyrrole-3-carbonitrile (60 mg, 0.19 mmol), trifluoromethanesulfonic acid (285 mg, 1.9 mmol), and 1,2-dichloroethane (3 mL) were mixed, and the mixture was stirred at room temperature for 2 hours. Water (4 mL) was added to the reaction mixture, followed by extraction with dichloromethane (15 mL × 3). The organic layers were combined, washed with saturated sodium chloride solution, dried over sodium sulfate, and concentrated. The residue was purified by silica gel column chromatography (ethyl acetate/petroleum ether, volume ratio = 10:90) to obtain 9-fluoro-3-methyl-6-phenylpyrrolo[2,1-a]isoquinoline-1-carbonitrile (10.9 mg, yield 19 %) as a white solid.

LCMS (ESI-MS): mass calcd C₂₀H₁₃FN₂ 300.1 m/z, found 300.7 [M+H]⁺.

¹H NMR (400 MHz, CDCl₃) δ 8.55 (dd, J = 7.2, 2.4 Hz, 1H), 7.73-7.38 (m, 7H), 7.24-7.11 (m, 1H), 6.78 (s, 1H), 2.49 (s, 3H).

### <Preparation Example 19> Preparation of Example 31 (compound represented by Formula 33; AMD-D-274)

The compound of Example 31 was synthesized according to Reaction Scheme 24 below.

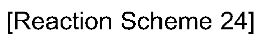

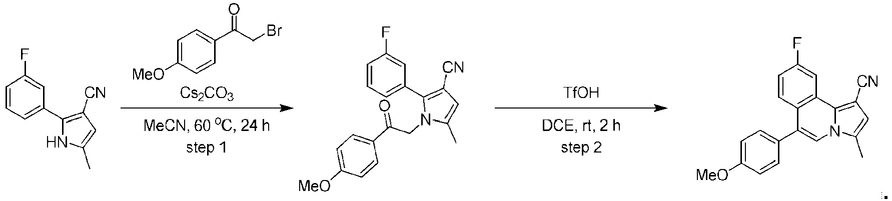

### Step 1. Preparation of 2-(3-fluorophenyl)-1-(2-(4-methoxyphenyl)-2-oxoethyl)-5-methyl-1H-pyrrole-3-carbonitrile

2-(3-Fluorophenyl)-5-methyl-1H-pyrrole-3-carbonitrile (270 mg, 1.35 mmol), which is the intermediate of Step 2 of Example 30, and 2-bromo-1-(4-methoxyphenyl)ethan-1-one (462 mg, 2.03 mmol) were mixed in acetonitrile (15 mL), and cesium carbonate (877 mg, 2.7 mmol) was added thereto. The mixture was stirred at 60 °C for 24 hours. Water (20 mL) was added to the reaction mixture, followed by extraction with dichloromethane (40 mL × 3). The organic layers were combined, washed with saturated sodium chloride solution, dried over sodium sulfate, and concentrated. The residue was purified by silica gel column chromatography (ethyl acetate/petroleum ether, volume ratio = 16:84) to obtain 2-(3-fluorophenyl)-1-(2-(4-methoxyphenyl)-2-oxoethyl)-5-methyl-1H-pyrrole-3-carbonitrile (150 mg, yield 43 %) as a yellow solid.

### Step 2. Preparation of 9-fluoro-6-(4-methoxyphenyl)-3-methylpyrrolo[2,1-a]isoquinoline-1-carbonitrile (Example 31)

2-(3-Fluorophenyl)-1-(2-(4-methoxyphenyl)-2-oxoethyl)-5-methyl-1H-pyrrole-3-carbonitrile (100 mg, 0.29 mmol), trifluoromethanesulfonic acid (435 mg, 2.9 mmol), and 1,2-dichloroethane (5 mL) were mixed, and the mixture was stirred at room temperature for 2 hours. Water (5 mL) was added to the reaction mixture, followed by extraction with dichloromethane (15 mL × 3). The organic layers were combined, washed with saturated sodium chloride solution, dried over sodium sulfate, and concentrated. The residue was purified by silica gel column chromatography (ethyl acetate/petroleum ether, volume ratio = 12:88) to obtain 9-fluoro-6-(4-methoxyphenyl)-3-methylpyrrolo[2,1-a]isoquinoline-1-carbonitrile (AMD-D-274) (10.6 mg, yield 11 %) as a white solid.

LCMS (ESI-MS): mass calcd C₂₁H₁₅FN₂O 330.1 m/z, found 330.7 [M+H]⁺.

¹H NMR (400 MHz, CDCl₃) δ 8.53 (dd, J = 9.6, 2.4 Hz, 1H), 7.65-7.62 (m, 1H), 7.56 (s, 1H), 7.45-7.34 (m, 2H), 7.24-7.13 (m, 1H), 7.10-7.02 (m, 2H), 6.77 (s, 1H), 3.91 (s, 3H), 2.49 (s, 3H).

### <Preparation Example 20> Preparation of Example 32 (compound represented by Formula 34; AMD-D-275)

The compound of Example 32 was synthesized according to Reaction Scheme 25 below.

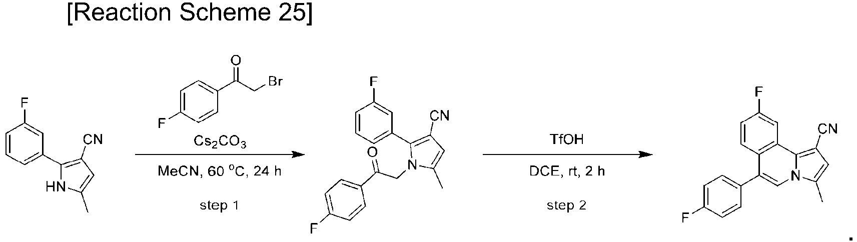

### Step 1. Preparation of 2-(3-fluorophenyl)-1-(2-(4-fluorophenyl)-2-oxoethyl)-5-methyl-1H-pyrrole-3-carbonitrile

2-(3-Fluorophenyl)-5-methyl-1H-pyrrole-3-carbonitrile (270 mg, 1.35 mmol), which is the intermediate of Step 2 of Example 30, 2-bromo-1-(4-fluorophenyl)ethan-1-one (440 mg, 2.03 mmol), and cesium carbonate (877 mg, 2.7 mmol) were mixed in acetonitrile (15 mL). The mixture was stirred at 60 °C for 24 hours. Water (20 mL) was added to the reaction mixture, followed by extraction with dichloromethane (40 mL × 3). The organic layers were combined, washed with saturated sodium chloride solution, dried over sodium sulfate, and concentrated. The residue was purified by silica gel column chromatography (ethyl acetate/petroleum ether, volume ratio = 16:84) to obtain 2-(3-fluorophenyl)-1-(2-(4-fluorophenyl)-2-oxoethyl)-5-methyl-1H-pyrrole-3-carbonitrile (100 mg, yield 22 %) as a yellow solid.

### Step 2. Preparation of 9-fluoro-6-(4-fluorophenyl)-3-methylpyrrolo[2,1-a]isoquinoline-1-carbonitrile (Example 32)

**2-(3-Fluorophenyl)-1-(2-(4-fluorophenyl)-2-oxoethyl)-5-methyl-1H-**pyrrole-3-carbonitrile (100 mg, 0.30 mmol), trifluoromethanesulfonic acid (450 mg, 3.0 mmol), and 1,2-dichloroethane (5 mL) were mixed, and the mixture was stirred at room temperature for 2 hours. Water (5 mL) was added to the reaction mixture, followed by extraction with dichloromethane (15 mL × 3). The organic layers were combined, washed with saturated sodium chloride solution, dried over sodium sulfate, and concentrated. The residue was purified by silica gel column chromatography (ethyl acetate/petroleum ether, volume ratio = 13:87) to obtain 9-fluoro-6-(4-fluorophenyl)-3-methylpyrrolo[2,1-a]isoquinoline-1-carbonitrile (10.6 mg, yield 11 %) as a white solid.

LCMS (ESI-MS): mass calcd C₂₀H₁₂F₂N₂ 318.1 m/z, found 318.7 [M+H]⁺.

¹H NMR (400 MHz, CDCl₃) δ 8.54 (dd, J = 9.6, 2.4 Hz, 1H), 7.58-7.54 (m, 2H), 7.49-7.42 (m, 2H), 7.28-7.10 (m, 3H), 6.78 (s, 1H), 2.50 (s, 3H).

### <Preparation Example 21> Preparation of Example 33 (compound represented by Formula 35; AMD-D-277)

The compound of Example 33 was synthesized according to Reaction Scheme 26 below.

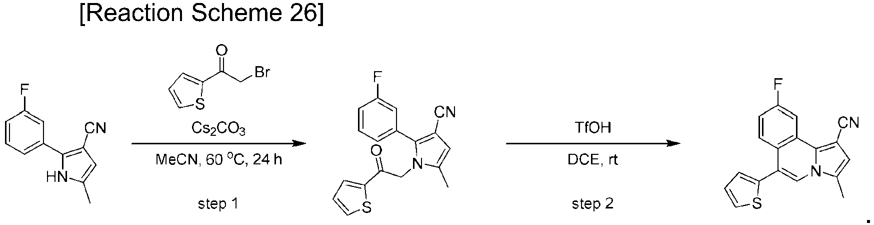

### Step 1. Preparation of 2-(3-fluorophenyl)-5-methyl-1-(2-oxo-2-(thiophen-2-yl)ethyl)-1H-pyrrole-3-carbonitrile

2-(3-Fluorophenyl)-5-methyl-1H-pyrrole-3-carbonitrile (270 mg, 1.35 mmol), which is the intermediate of Step 2 of Example 30, and 2-bromo-1-(thiophen-2-yl)ethan-1-one (416 mg, 2.03 mmol) were mixed in acetonitrile (15 mL), and cesium carbonate (877 mg, 2.7 mmol) was added thereto. The mixture was stirred at 60 °C for 24 hours. Water (20 mL) was added to the reaction mixture, followed by extraction with dichloromethane (40 mL × 3). The organic layers were combined, washed with saturated sodium chloride solution, dried over sodium sulfate, and concentrated. The residue was purified by silica gel column chromatography (ethyl acetate/petroleum ether, volume ratio = 16:84) to obtain 2-(3-fluorophenyl)-5-methyl-1-(2-oxo-2-(thiophen-2-yl)ethyl)-1H-pyrrole-3-carbonitrile (150 mg, yield 46 %) as a yellow solid.

### Step 2. Preparation of 9-fluoro-3-methyl-6-(thiophen-2-yl)pyrrolo[2,1-a]isoquinoline-1-carbonitrile (Example 33)

2-(3-Fluorophenyl)-5-methyl-1-(2-oxo-2-(thiophen-2-yl)ethyl)-1H-pyrrole-3-carbonitrile (80 mg, 0.25 mmol), trifluoromethanesulfonic acid (375 mg, 2.5 mmol), and 1,2-dichloroethane (3 mL) were mixed, and the mixture was stirred at room temperature for 2 hours. Water (5 mL) was added to the reaction mixture, followed by extraction with dichloromethane (15 mL × 3). The organic layers were combined, washed with saturated sodium chloride solution, dried over sodium sulfate, and concentrated. The residue was purified by silica gel column chromatography (ethyl acetate/petroleum ether, volume ratio = 20:80) to obtain 9-fluoro-3-methyl-6-(thiophen-2-yl)pyrrolo[2,1-a]isoquinoline-1-carbonitrile (10.9 mg, yield 14 %) as a white solid.

LCMS (ESI-MS): mass calcd C₁₈H₁₁FN₂S 306.1 m/z, found 306.7 [M+H]⁺.

¹H NMR (400 MHz, CDCl₃) δ 8.52 (dd, J = 9.6, 2.4 Hz, 1H), 7.92-7.88 (m, 1H), 7.73 (s, 1H), 7.49 (d, J = 4.8 Hz, 1H), 7.24-7.21 (m, 3H), 6.78 (s, 1H), 2.50 (s, 3H).

### <Preparation Example 22> Preparation of Example 34 (compound represented by Formula 36; AMD-D-278)

The compound of Example 34 was synthesized according to Reaction Scheme 27 below.

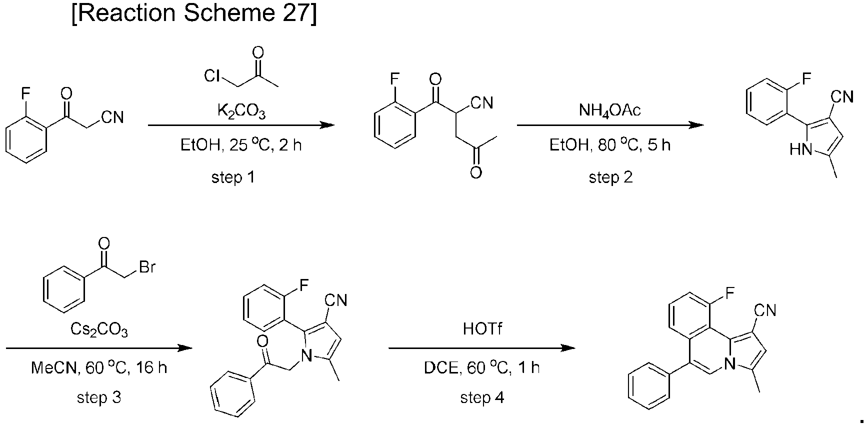

### Step 1. Preparation of 2-(2-fluorobenzoyl)-4-oxopentanenitrile

3-(2-Fluorophenyl)-3-oxopropanenitrile (3.23 g, 19.8 mmol) and potassium carbonate (6.83 g, 49.5 mmol) were mixed in ethanol (50 mL), and 1-chloropropan-2-one (2.02 g, 21.78 mmol) was added thereto. The mixture was stirred at 25 °C for 2 hours. Water (100 mL) was added to the reaction mixture, followed by extraction with ethyl acetate (100 mL × 3). The organic layers were combined, washed with saturated sodium chloride solution, dried over sodium sulfate, and concentrated. The residue was purified by silica gel column chromatography (ethyl acetate/petroleum ether, volume ratio = 10:90) to obtain 2-(2-fluorobenzoyl)-4-oxopentanenitrile (4.3 g, yield 98 %) as a yellow oil.

### Step 2. Preparation of 2-(2-fluorophenyl)-5-methyl-1H-pyrrole-3-carbonitrile

2-(2-Fluorobenzoyl)-4-oxopentanenitrile (4.66 g, 21.3 mmol) and ammonium acetate (3.28 g, 42.6 mmol) were mixed in ethanol (50 mL), and the mixture was stirred at 80 °C for 5 hours. The reaction mixture was concentrated, and the residue was purified by silica gel column chromatography (ethyl acetate/petroleum ether, volume ratio = 15:85) to obtain 2-(2-fluorophenyl)-5-methyl-1H-pyrrole-3-carbonitrile (3.04 g, yield 71 %) as a yellow solid.

### Step 3. Preparation of 2-(2-fluorophenyl)-5-methyl-1-(2-oxo-2-phenylethyl)-1H-pyrrole-3-carbonitrile

2-(2-Fluorophenyl)-5-methyl-1H-pyrrole-3-carbonitrile (200 mg, 0.999 mmol) and 2-bromo-1-phenylethan-1-one (238.6 mg, 1.20 mmol) were dissolved in acetonitrile (10 mL), and cesium carbonate (651 mg, 1.998 mmol) was added thereto. The mixture was stirred at 60 °C for 16 hours. Water (30 mL) was added to the reaction mixture, followed by extraction with dichloromethane (30 mL × 3). The organic layers were washed with saturated sodium chloride solution, dried over sodium sulfate, and concentrated. The residue was purified by silica gel column chromatography (ethyl acetate/petroleum ether, volume ratio = 15:85) to obtain 2-(2-fluorophenyl)-5-methyl-1-(2-oxo-2-phenylethyl)-1H-pyrrole-3-carbonitrile (230 mg, yield 72 %) as a yellow oil.

### Step 4. Preparation of 10-fluoro-3-methyl-6-phenylpyrrolo[2,1-a]isoquinoline-1-carbonitrile (Example 34)

2-(2-Fluorophenyl)-5-methyl-1-(2-oxo-2-phenylethyl)-1H-pyrrole-3-carbonitrile (100 mg, 0.314 mmol) was dissolved in 1,2-dichloroethane (8 mL), and trifluoromethanesulfonic acid (235.7 mg, 1.57 mmol) was added thereto. The mixture was stirred at 60 °C for 1 hour. Water (20 mL) was added to the reaction mixture, followed by extraction with dichloromethane (20 mL × 3). The organic layers were washed with saturated sodium chloride solution, dried over sodium sulfate, and concentrated. The residue was purified by silica gel column chromatography (ethyl acetate/petroleum ether, volume ratio = 8:92) to obtain 10-fluoro-3-methyl-6-phenylpyrrolo[2,1-a]isoquinoline-1-carbonitrile (10.6 mg, yield 11 %) as a white solid.

LCMS (ESI-MS): mass calcd C₂₀H₁₃FN₂ 300.1 m/z, found 301.1 [M+H]⁺.

¹H NMR (400 MHz, CDCl₃) δ 7.64 (s, 1H), 7.56-7.46 (m, 5H), 7.43-7.39 (m, 2H), 7.38-7.33 (m, 1H), 6.90 (s, 1H), 2.50 (s, 3H).

### <Preparation Example 23> Preparation of Example 35 (compound represented by Formula 37; AMD-D-279)

The compound of Example 35 was synthesized according to Reaction Scheme 28 below.

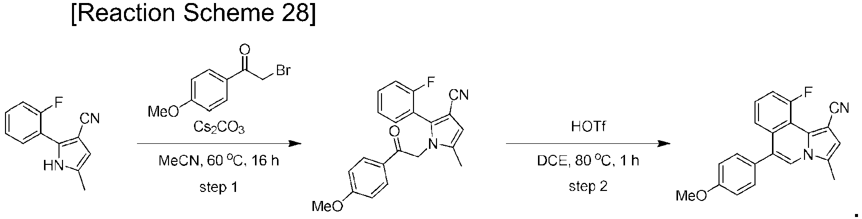

### Step 1. Preparation of 2-(2-fluorophenyl)-1-(2-(4-methoxyphenyl)-2-oxoethyl)-5-methyl-1H-pyrrole-3-carbonitrile)

2-(2-Fluorophenyl)-5-methyl-1H-pyrrole-3-carbonitrile (200 mg, 0.999 mmol), which is the intermediate of Step 2 of Example 34, and 2-bromo-1-(4-methoxyphenyl)ethan-1-one (343.23 mg, 1.50 mmol) were dissolved in acetonitrile (10 mL), and cesium carbonate (651 mg, 1.998 mmol) was added thereto. The mixture was stirred at 60 °C for 16 hours. Water (30 mL) was added to the reaction mixture, followed by extraction with dichloromethane (30 mL × 3). The organic layers were washed with saturated sodium chloride solution, dried over sodium sulfate, and concentrated. The residue was purified by silica gel column chromatography (ethyl acetate/petroleum ether, volume ratio = 15:85) to obtain 2-(2-fluorophenyl)-1-(2-(4-methoxyphenyl)-2-oxoethyl)-5-methyl-1H-pyrrole-3-carbonitrile (330 mg, yield 94 %) as a yellow oil.

### Step 2. Preparation of 10-fluoro-6-(4-methoxyphenyl)-3-methylpyrrolo[2,1-a]isoquinoline-1-carbonitrile (Example 35)

2-(2-Fluorophenyl)-1-(2-(4-methoxyphenyl)-2-oxoethyl)-5-methyl-1H-pyrrole-3-carbonitrile (800 mg, 2.299 mmol), trifluoromethanesulfonic acid (3.448 g, 22.99 mmol), and 1,2-dichloroethane (50 mL) were mixed, and the mixture was stirred at 80 °C for 1 hour. Water (50 mL) was added to the reaction mixture, followed by extraction with dichloromethane (70 mL × 3). The organic layers were washed with saturated sodium chloride solution, dried over sodium sulfate, and concentrated. The residue was purified by silica gel column chromatography (ethyl acetate/petroleum ether, volume ratio = 10:90) to obtain 10-fluoro-6-(4-methoxyphenyl)-3-methylpyrrolo[2,1-a]isoquinoline-1-carbonitrile (22 mg, yield 1 %) as a white solid.

LCMS (ESI-MS): mass calcd C₂₁H₁₅FN₂O 330.1 m/z, found 330.7 [M+H]⁺.

¹H NMR (400 MHz, DMSO-d₆) δ 8.02 (s, 1H), 7.63-7.55 (m, 2H), 7.51-7.44 (m, 3H), 7.16-7.06 (m, 3H), 3.85 (s, 3H), 2.54 (s, 3H).

### <Preparation Example 24> Preparation of Example 36 (compound represented by Formula 38; AMD-D-280)

The compound of Example 36 was synthesized according to Reaction Scheme 29 below.

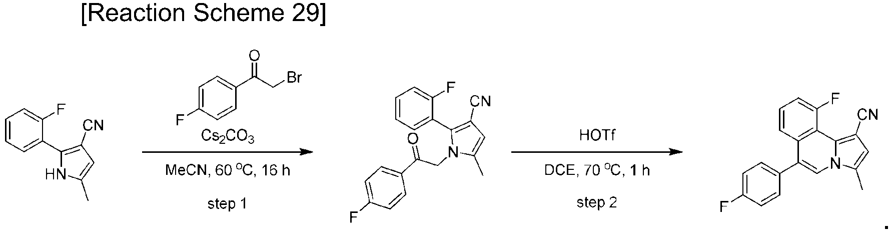

### Step 1. Preparation of 2-(2-fluorophenyl)-1-(2-(4-fluorophenyl)-2-oxoethyl)-5-methyl-1H-pyrrole-3-carbonitrile)

2-(2-Fluorophenyl)-5-methyl-1H-pyrrole-3-carbonitrile (200 mg, 0.999 mmol), which is the intermediate of Step 2 of Example 34, and 2-bromo-1-(4-fluorophenyl)ethan-1-one (325.19 mg, 1.50 mmol) were dissolved in acetonitrile (10 mL), and cesium carbonate (651 mg, 1.998 mmol) was added thereto. The mixture was stirred at 60 °C for 16 hours. Water (30 mL) was added to the reaction mixture, followed by extraction with dichloromethane (30 mL × 3). The organic layers were washed with saturated sodium chloride solution, dried over sodium sulfate, and concentrated. The residue was purified by silica gel column chromatography (ethyl acetate/petroleum ether, volume ratio = 10:90) to obtain 2-(2-fluorophenyl)-1-(2-(4-fluorophenyl)-2-oxoethyl)-5-methyl-1H-pyrrole-3-carbonitrile (230 mg, yield 68 %) as a yellow oil.

### Step 2. Preparation of 10-fluoro-6-(4-fluorophenyl)-3-methylpyrrolo[2,1-a]isoquinoline-1-carbonitrile (Example 36)

2-(2-Fluorophenyl)-1-(2-(4-fluorophenyl)-2-oxoethyl)-5-methyl-1H-pyrrole-3-carbonitrile (100 mg, 0.297 mmol), trifluoromethanesulfonic acid (446.19 mg, 2.973 mmol), and 1,2-dichloroethane (5 mL) were mixed, and the mixture was stirred at 70 °C for 1 hour. Water (10 mL) was added to the reaction mixture, followed by extraction with dichloromethane (15 mL × 3). The organic layers were washed with saturated sodium chloride solution, dried over sodium sulfate, and concentrated. The residue was purified by silica gel column chromatography (ethyl acetate/petroleum ether, volume ratio = 10:90) to obtain 10-fluoro-6-(4-fluorophenyl)-3-methylpyrrolo[2,1-a]isoquinoline-1-carbonitrile (15 mg, yield 15 %) as a white solid.

LCMS (ESI-MS): mass calcd C₂₀H₁₂F₂N₂ 318.1 m/z, found 318.7 [M+H]⁺.

¹H NMR (400 MHz, DMSO-d₆) δ 8.08 (s, 1H), 7.62-7.57 (m, 4H), 7.46-7.35 (m, 3H), 7.08 (s, 1H), 2.54 (s, 3H).

### <Preparation Example 25> Preparation of Example 37 (compound represented by Formula 39; AMD-D-284)

The compound of Example 37 was synthesized according to Reaction Scheme 30 below.

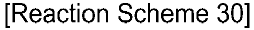

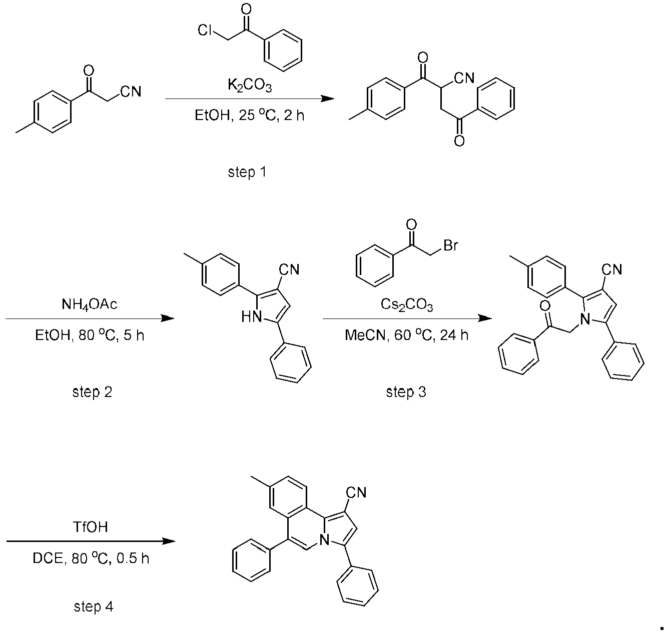

### Step 1. Preparation of 2-(4-methylbenzoyl)-4-oxo-4-phenylbutanenitrile

3-Oxo-3-(p-tolyl)propanenitrile (500 mg, 3.14 mmol) and 2-chloro-1-phenylethan-1-one (534 mg, 3.45 mmol) were mixed in ethanol (8 mL), and potassium carbonate (1085 mg, 7.85 mmol) was added thereto under a nitrogen atmosphere. The mixture was stirred at 25 °C for 2 hours. Water (5 mL) was added to the reaction mixture, followed by extraction with ethyl acetate (15 mL × 3). The organic layers were washed with saturated sodium chloride solution, dried over sodium sulfate, and concentrated. The residue was purified by silica gel column chromatography (ethyl acetate/petroleum ether, volume ratio = 5:95) to obtain 2-(4-methylbenzoyl)-4-oxo-4-phenylbutanenitrile (260 mg, yield 29 %).

### Step 2. Preparation of 5-phenyl-2-(p-tolyl)-1H-pyrrole-3-carbonitrile

2-(4-Methylbenzoyl)-4-oxo-4-phenylbutanenitrile (260 mg, 0.94 mmol) and ammonium acetate (145 mg, 1.88 mmol) were mixed in ethanol (5 mL), and the mixture was stirred at 80 °C for 5 hours. Water (5 mL) was added to the reaction mixture, followed by extraction with dichloromethane (10 mL × 3). The organic layers were washed with saturated sodium chloride solution, dried over sodium sulfate, and concentrated. The residue was purified by silica gel column chromatography (ethyl acetate/petroleum ether, volume ratio = 10:90) to obtain 5-phenyl-2-(p-tolyl)-1H-pyrrole-3-carbonitrile (166 mg, yield 66 %) as a yellow solid.

### Step 3. Preparation of 1-(2-oxo-2-phenylethyl)-5-phenyl-2-(p-tolyl)-1H-pyrrole-3-carbonitrile

5-Phenyl-2-(p-tolyl)-1H-pyrrole-3-carbonitrile (160 mg, 0.62 mmol) and 2-bromo-1-phenylethan-1-one (185 mg, 0.93 mmol) were dissolved in acetonitrile (6 mL), and cesium carbonate (404 mg, 1.24 mmol) was added thereto. The mixture was stirred at 60 °C for 24 hours. Water (10 mL) was added to the reaction mixture, followed by extraction with dichloromethane (20 mL × 3). The organic layers were washed with saturated sodium chloride solution, dried over sodium sulfate, and concentrated. The residue was purified by silica gel column chromatography (ethyl acetate/petroleum ether, volume ratio = 15:85) to obtain 1-(2-oxo-2-phenylethyl)-5-phenyl-2-(p-tolyl)-1H-pyrrole-3-carbonitrile (160 mg, yield 68 %) as a yellow solid.

### Step 4. Preparation of 8-methyl-3,6-diphenylpyrrolo[2,1-a]isoquinoline-1-carbonitrile (Example 37)

1-(2-Oxo-2-phenylethyl)-5-phenyl-2-(p-tolyl)-1H-pyrrole-3-carbonitrile (100 mg, 0.26 mmol), trifluoromethanesulfonic acid (199 mg, 1.33 mmol), and 1,2-dichloroethane (10 mL) were mixed, and the mixture was stirred at 80 °C for 0.5 hours. Water (20 mL) was added to the reaction mixture, followed by extraction with dichloromethane (20 mL × 3). The organic layers were washed with saturated sodium chloride solution, dried over sodium sulfate, and concentrated. The residue was purified by Prep-HPLC (20 - 60 % acetonitrile/0.1 % aqueous ammonia) to obtain 8-methyl-3,6-diphenylpyrrolo[2,1-a]isoquinoline-1-carbonitrile (23.3 mg, yield 24 %) as a white solid.

LCMS (ESI-MS): mass calcd C₂₆H₁₈N₂ 358.1 m/z, found 359.0 [M+H]⁺.

¹H NMR (400 MHz, CDCl₃) δ 8.11 (d, J = 7.6 Hz, 1H), 7.79 (s, 1H), 7.57-7.50 (m, 4H), 7.47-7.45 (m, 2H), 7.45-7.40 (m, 3H), 7.39-7.33 (m, 3H), 7.29 (s, 1H), 2.43 (s, 3H).

### <Preparation Example 26> Preparation of Example 38 (compound represented by Formula 40; AMD-D-286)

The compound of Example 38 was synthesized according to Reaction Scheme 31 below.

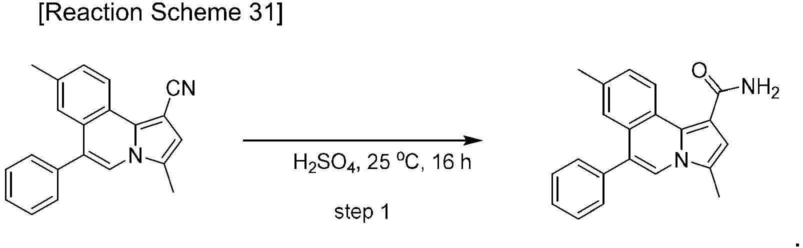

### Step 1. Preparation of 3,8-dimethyl-6-phenylpyrrolo[2,1-a]isoquinoline-1-carboxamide (Example 38)

3,8-Dimethyl-6-phenylpyrrolo[2,1-a]isoquinoline-1-carbonitrile (Example 26, 300 mg, 1.01 mmol) and sulfuric acid (3 mL) were mixed, and the mixture was stirred at 25 °C for 16 hours. Water (10 mL) was added to the reaction mixture, followed by extraction with dichloromethane (10 mL × 3). The organic layers were washed, dried over sodium sulfate, and concentrated. The residue was purified by silica gel column chromatography (methanol/dichloromethane, volume ratio = 10:90) to obtain 3,8-dimethyl-6-phenylpyrrolo[2,1-a]isoquinoline-1-carboxamide (150 mg, yield 47 %) as a white solid.

LCMS (ESI-MS): mass calcd for C₂₁H₁₈N₂O 314.1 m/z, found 314.8 [M+H]⁺

¹H NMR (400 MHz, DMSO-d₆) δ 9.59 (d, J = 8.4 Hz, 1H), 7.80 (s, 1H), 7.62-7.45 (m, 5H), 7.35 (d, J = 8.4 Hz, 1H), 7.28 (s, 1H), 6.90 (s, 1H), 2.47 (s, 3H), 2.34 (s, 3H)

### <Preparation Example 27> Preparation of Example 39 (compound represented by Formula 41; AMD-D-287)

The compound of Example 39 was synthesized according to Reaction Scheme 32 below.

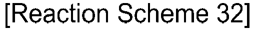

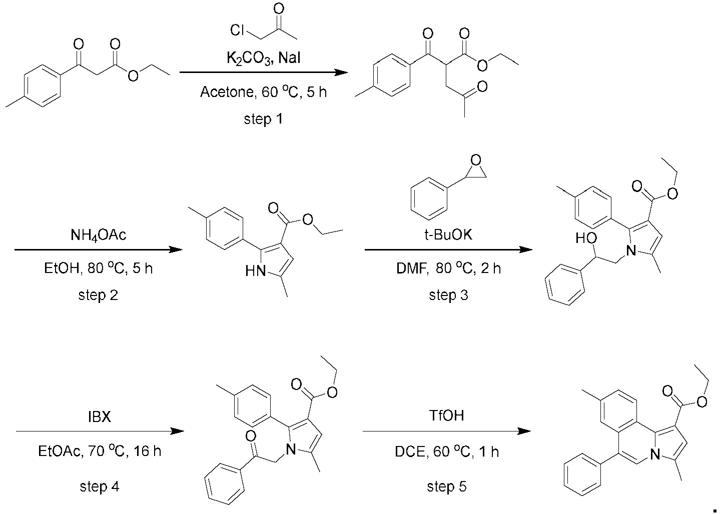

### Step 1. Preparation of ethyl 2-(4-methylbenzoyl)-4-oxopentanoate

Ethyl 3-(4-methylphenyl)-3-oxopropanoate (7 g, 33.9 mmol), sodium iodide (1.02 g, 6.70 mmol), and potassium carbonate (11.71 g, 84.7 mmol) were mixed in acetone (50 mL), and 1-chloropropan-2-one (3.45 g, 37.29 mmol) was added thereto. The mixture was stirred at 60 °C for 5 hours. The reaction mixture was diluted with water (150 mL), followed by extraction with ethyl acetate (100 mL × 3). The organic layers were combined, washed with saturated sodium chloride solution, dried over anhydrous sodium sulfate, and concentrated under reduced pressure. The residue was purified by silica gel column chromatography (ethyl acetate/hexane, volume ratio = 5:95) to obtain ethyl 2-(4-methylbenzoyl)-4-oxopentanoate (8 g, yield 90 %) as a yellow solid.

### Step 2. Preparation of ethyl 5-methyl-2-(p-tolyl)-1H-pyrrole-3-carboxylate

Ethyl 2-(4-methylbenzoyl)-4-oxopentanoate (8 g, 30.5 mmol) was dissolved in ethanol (80 mL), and ammonium acetate (4.70 g, 61.0 mmol) was added thereto. The mixture was stirred at 80 °C for 5 hours. The reaction mixture was concentrated, and the residue was purified by silica gel column chromatography (ethyl acetate/hexane, volume ratio = 10:90) to obtain ethyl 5-methyl-2-(p-tolyl)-1H-pyrrole-3-carboxylate (7 g, yield 94 %) as a yellow solid.

### Step 3. Preparation of ethyl 1-(2-hydroxy-2-phenylethyl)-5-methyl-2-(p-tolyl)-1H-pyrrole-3-carboxylate

Ethyl 5-methyl-2-(p-tolyl)-1H-pyrrole-3-carboxylate (2 g, 8 mmol), 2-phenyloxirane (1.48 g, 12 mmol), and potassium tert-butoxide (2.76 g, 24 mmol) were mixed in dimethylformamide (DMF, 60 mL), and the mixture was heated at 80 °C for 2 hours. The reaction mixture was diluted with water (100 mL), followed by extraction with ethyl acetate (100 mL × 3). The organic layers were washed with saturated sodium chloride solution, dried over anhydrous sodium sulfate, and concentrated. The residue was crystallized from a mixed solvent of ethyl acetate/hexane (1:1), filtered, and dried in vacuum to obtain ethyl 1-(2-hydroxy-2-phenylethyl)-5-methyl-2-(p-tolyl)-1H-pyrrole-3-carboxylate (500 mg, yield 17 %) as a white solid.

### Step 4. Preparation of ethyl 5-methyl-1-(2-oxo-2-phenylethyl)-2-(p-tolyl)-1H-pyrrole-3-carboxylate)

Ethyl 1-(2-hydroxy-2-phenylethyl)-5-methyl-2-(p-tolyl)-1H-pyrrole-3-carboxylate (500 mg, 1.375 mmol) and iodoxybenzoic acid (577.84 mg, 2.063 mmol) were dissolved in ethyl acetate (10 mL), and the mixture was stirred at 70 °C for 16 hours. The reaction mixture was diluted with water (20 mL), followed by extraction with ethyl acetate (20 mL × 3). The organic layers were washed with saturated sodium chloride solution, dried over anhydrous sodium sulfate, and concentrated. The residue was purified by silica gel column chromatography (ethyl acetate/petroleum ether, volume ratio = 15:85) to obtain ethyl 5-methyl-1-(2-oxo-2-phenylethyl)-2-(p-tolyl)-1H-pyrrole-3-carboxylate (300 mg, yield 60 %) as a white solid.

### Step 5. Preparation of ethyl 3,8-dimethyl-6-phenylpyrrolo[2,1-a]isoquinoline-1-carboxylate (Example 39)

Ethyl 5-methyl-2-(4-methylphenyl)-1-(2-oxo-2-phenylethyl)-pyrrole-3-carboxylate (100 mg, 0.276 mmol), trifluoromethanesulfonic acid (415.27 mg, 2.767 mmol), and 1,2-dichloroethane (5 mL) were mixed, and the mixture was stirred at 60 °C for 1 hour. The reaction mixture was diluted with water (10 mL), followed by extraction with dichloromethane (15 mL × 3). The organic layers were washed with saturated sodium chloride solution, dried over anhydrous sodium sulfate, and concentrated. The residue was purified by silica gel column chromatography (ethyl acetate/petroleum ether, volume ratio = 5:95) to obtain ethyl 3,8-dimethyl-6-phenylpyrrolo[2,1-a]isoquinoline-1-carboxylate (7 mg, yield 7 %) as a white solid.

LCMS (ESI-MS): mass calcd for C₂₃H₂₁NO₂ 343.2 m/z, found 343.8 [M+H]⁺.

¹H NMR (400 MHz, CDCl₃) δ 9.85 (d, J = 8.4 Hz, 1H), 7.58 (s, 1H), 7.56-7.47 (m, 5H), 7.46-7.34 (m, 2H), 7.06-7.05 (m, 1H), 4.40 (q, J = 7.2 Hz, 2H), 2.47 (s, 3H), 2.40 (s, 3H), 1.44 (t, J = 7.2 Hz, 3H).

### <Preparation Example 28> Preparation of Example 40 (compound represented by Formula 42; AMD-D-289)

The compound of Example 40 was synthesized according to Reaction Scheme 33 below.

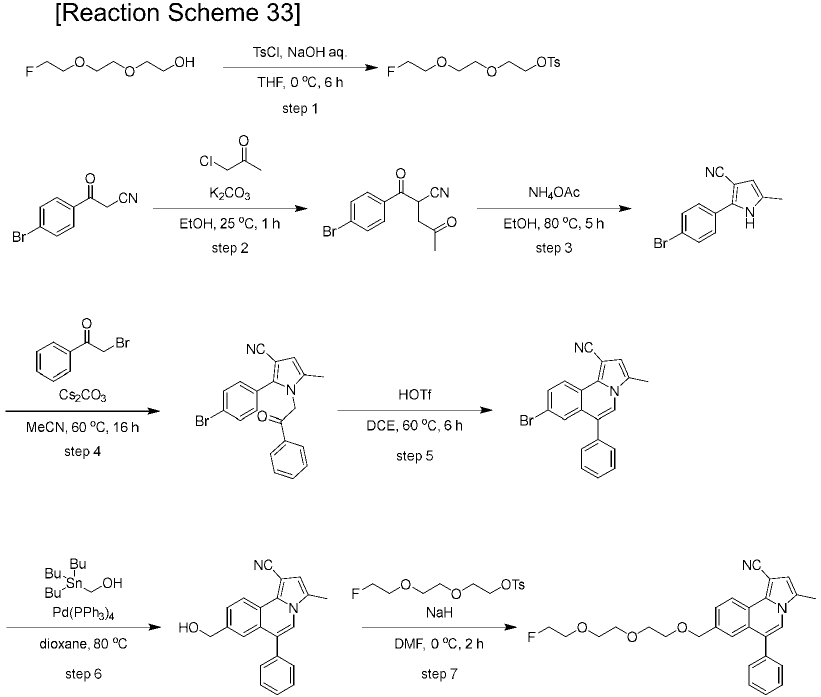

### Step 1. Preparation of 2-(2-(2-fluoroethoxy)ethoxy)ethyl 4-methylbenzenesulfonate

2-(2-(2-Fluoroethoxy)ethoxy)ethanol (300 mg, 1.97 mmol) and an aqueous sodium hydroxide solution (5 N, 1 mL) were mixed in tetrahydrofuran (10 mL), followed by the addition of 4-methylbenzenesulfonyl chloride (570 mg, 3.0 mmol). The mixture was stirred at 0 °C for 6 hours. The reaction mixture was diluted with water (15 mL), followed by extraction with ethyl acetate (10 mL × 3). The organic layer was washed with saturated sodium chloride solution, dried over anhydrous sodium sulfate, and concentrated under reduced pressure. The residue was purified by silica gel column chromatography (ethyl acetate/petroleum ether, volume ratio = 20:80) to obtain the target compound (220 mg, yield 36 %) as a yellow oil.

### Step 2. Preparation of 2-(4-bromobenzoyl)-4-oxopentanenitrile

3-(4-Bromophenyl)-3-oxopropanenitrile (6.0 g, 26.8 mmol), 1-chloropropan-2-one (4.9 g, 53.6 mmol), and potassium carbonate (7.4 g, 53.6 mmol) were mixed in ethanol (100 mL), and the mixture was stirred at room temperature (25 °C) for 1 hour. The reaction mixture was diluted with water (100 mL), followed by extraction with dichloromethane (80 mL × 3). The organic layer was washed with saturated sodium chloride solution, dried over anhydrous sodium sulfate, and concentrated. The residue was purified by silica gel column chromatography (ethyl acetate/petroleum ether, volume ratio = 13:87) to obtain a yellow solid (4.1 g, yield 55 %).

### Step 3. Preparation of 2-(4-bromophenyl)-5-methyl-1H-pyrrole-3-carbonitrile

2-(4-Bromobenzoyl)-4-oxopentanenitrile (4.1 g, 14.6 mmol) and ammonium acetate (2.26 g, 29.3 mmol) were mixed in ethanol (60 mL), and the mixture was stirred at 80 °C for 5 hours. The reaction mixture was diluted with water (80 mL), followed by extraction with dichloromethane (60 mL × 3). The organic layer was washed, dried, and concentrated. The residue was purified by silica gel column chromatography (EtOAc/PE, volume ratio = 20:80) to obtain a yellow solid (3.0 g, yield 79 %).

### Step 4. Preparation of 2-(4-bromophenyl)-5-methyl-1-(2-oxo-2-phenylethyl)-1H-pyrrole-3-carbonitrile

2-(4-Bromophenyl)-5-methyl-1H-pyrrole-3-carbonitrile (3.0 g, 11.5 mmol), 2-bromo-1-phenylethan-1-one (4.6 g, 23.0 mmol), and cesium carbonate (7.5 g, 23.0 mmol) were mixed in acetonitrile (150 mL), and the mixture was stirred at 60 °C for 16 hours. The reaction mixture was diluted with water (200 mL), followed by extraction with dichloromethane (150 mL × 3). The residue was purified by silica gel column chromatography (ethyl acetate/petroleum ether, volume ratio = 40:60) to obtain a yellow solid (2.0 g, yield 46 %).

### Step 5. Preparation of 8-bromo-3-methyl-6-phenylpyrrolo[2,1-a]isoquinoline-1-carbonitrile

2-(4-Bromophenyl)-5-methyl-1-(2-oxo-2-phenylethyl)-1H-pyrrole-3-carbonitrile (2.0 g, 7.9 mmol) and trifluoromethanesulfonic acid (11.9 g, 79.0 mmol) were dissolved in 1,2-dichloroethane (150 mL), and the mixture was stirred at 60 °C for 6 hours. The reaction mixture was diluted with water (100 mL), followed by extraction with dichloromethane (80 mL × 3). The residue was purified by silica gel column chromatography (methanol/dichloromethane, volume ratio = 2:98) to obtain a yellow oil (1.2 g, yield 42 %).

### Step 6. Preparation of 8-(hydroxymethyl)-3-methyl-6-phenylpyrrolo[2,1-a]isoquinoline-1-carbonitrile

8-Bromo-3-methyl-6-phenylpyrrolo[2,1-a]isoquinoline-1-carbonitrile (300 mg, 0.83 mmol), (tributylstannyl)methanol (533 mg, 1.66 mmol), and tetrakis(triphenylphosphine)palladium (92 mg, 0.08 mmol) were dissolved in dioxane (10 mL), and the mixture was stirred at 80 °C for 16 hours under a nitrogen atmosphere. The reaction mixture was diluted with water (20 mL), extracted with dichloromethane (20 mL × 3), and purified to obtain a red solid (170 mg, yield 66 %).

### Step 7. Preparation of 8-((2-(2-(2-fluoroethoxy)ethoxy)ethoxy)methyl)-3-methyl-6-phenylpyrrolo[2,1-a]isoquinoline-1-carbonitrile (Example 40)

8-(Hydroxymethyl)-3-methyl-6-phenylpyrrolo[2,1-a]isoquinoline-1-carbonitrile (60 mg, 0.19 mmol) and sodium hydride (38 mg, 0.95 mmol) were mixed in N,N-dimethylformamide (DMF, 5 mL), and 2-(2-(2-fluoroethoxy)ethoxy)ethyl 4-methylbenzenesulfonate (116 mg, 0.38 mmol) was added thereto. The mixture was stirred at 0 °C for 2 hours, diluted with water (10 mL), and extracted with ethyl acetate (10 mL × 3). The residue was purified by prep-HPLC (Gemini 5 µm C18 column, 150 × 21.2 mm, 30-90 % acetonitrile/purified water containing 0.1 % formic acid) to obtain a yellow solid (10.3 mg, yield 12 %).

LCMS (ESI-MS): mass calcd C₂₇H₂₇FN₂O₃ 446.2 m/z, found 446.8 [M+H]⁺.

¹H NMR (400 MHz, CDCl₃) δ 8.89 (d, J = 8.4 Hz, 1H), 7.75-7.43 (m, 8H), 6.76 (s, 1H), 4.70-4.55 (m, 3H), 4.53-4.44 (m, 1H), 3.82-3.74 (m, 1H), 3.74-3.54 (m, 9H), 2.49 (s, 3H).

### <Preparation Example 29> Preparation of Example 41 (compound represented by Formula 43; AMD-D-271)

The compound of Example 41 was synthesized according to Reaction Scheme 34 below.

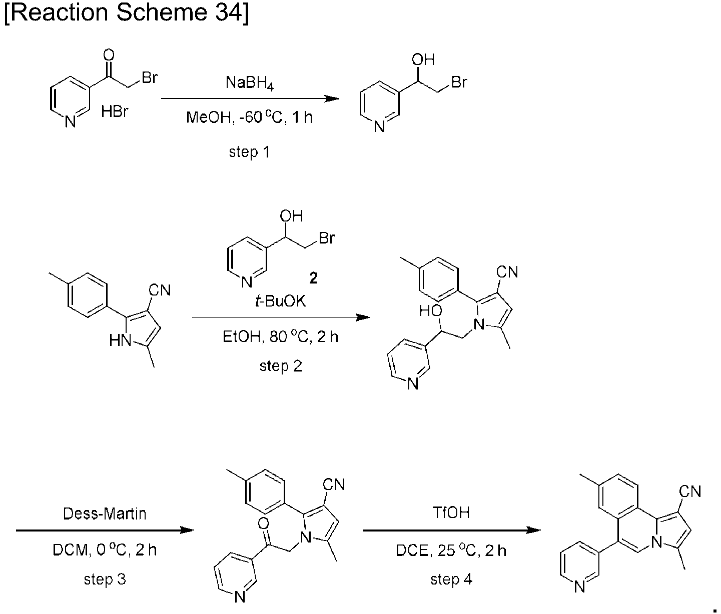

### Step 1. Preparation of 2-bromo-1-(pyridin-3-yl)ethan-1-ol

To a suspension of Sodium borohydride (1.6 g, 42 mmol) in methanol (90 mL) was added a solution of 2-bromo-1-(pyridin-3-yl)ethan-1-one hydrobromide (3.0 g, 10.5 mmol) suspended in methanol (60 mL) slowly at -60°C for 1 hour. After the addition was completed, the reaction mixture was diluted with water (150 mL) and extracted with ethyl acetate (100 mL × 3). The organic layer was concentrated under reduced pressure to obtain 2-bromo-1-(pyridin-3-yl)ethan-1-ol (600 mg, yield 28 %). Due to its instability at 25 °C, the compound was used immediately in the next step without purification.

### Step 2. Preparation of 1-(2-hydroxy-2-(pyridin-3-yl)ethyl)-5-methyl-2-(p-tolyl)-1H-pyrrole-3-carbonitrile

5-Methyl-2-(p-tolyl)-1H-pyrrole-3-carbonitrile (400 mg, 2.04 mmol) and potassium tert-butoxide (120 mg, 4.08 mmol) were dissolved in ethanol (10 mL), and 2-bromo-1-(pyridin-3-yl)ethan-1-ol (494 mg, 2.45 mmol) prepared in Step 1 was added thereto. The reaction mixture was stirred at 80 °C for 2 hours. After the reaction was completed, the reaction solution was diluted with water (10 mL) and extracted with ethyl acetate (10 mL × 3). The organic layers were washed with saturated sodium chloride solution, dried over sodium sulfate, and concentrated under reduced pressure. The concentrate was purified by silica gel column chromatography (ethyl acetate/petroleum ether, volume ratio = 1:1) to obtain the target compound, 1-(2-hydroxy-2-(pyridin-3-yl)ethyl)-5-methyl-2-(p-tolyl)-1H-pyrrole-3-carbonitrile (160 mg, yield 25 %) as a yellow oil.

### Step 3. Preparation of 5-methyl-1-(2-oxo-2-(pyridin-3-yl)ethyl)-2-(p-tolyl)-1H-pyrrole-3-carbonitrile

The compound prepared in Step 2 (140 mg, 0.38 mmol) and Dess-Martin periodinane (240 mg, 0.57 mmol) were dissolved in dichloromethane (5 mL), and the mixture was reacted at 0 °C for 2 hours. The reaction mixture was diluted with water (10 mL), followed by extraction with dichloromethane (10 mL × 3). The organic layers were washed with saturated sodium chloride solution, dried over sodium sulfate, and concentrated under reduced pressure. The concentrate was purified by silica gel column chromatography (ethyl acetate/petroleum ether, volume ratio = 4:6) to obtain the target compound, 5-methyl-1-(2-oxo-2-(pyridin-3-yl)ethyl)-2-(p-tolyl)-1H-pyrrole-3-carbonitrile (60 mg, yield 43 %) as a white solid.

### Step 4. Preparation of 3,8-dimethyl-6-(pyridin-3-yl)pyrrolo[2,1-a]isoquinoline-1-carbonitrile (Example 41)

The compound prepared in Step 3 (50 mg, 0.15 mmol) and trifluoromethanesulfonic acid (112 mg, 0.75 mmol) were dissolved in 1,2-dichloroethane (5 mL), and the mixture was reacted at room temperature (25 °C) for 2 hours. After the reaction, the reaction solution was diluted with water (10 mL) and extracted with dichloromethane (10 mL × 3). The organic layers were washed with saturated sodium chloride solution, dried over sodium sulfate, and concentrated under reduced pressure. The concentrate was purified by prep-HPLC (Gemini 5 µm C18 column, 150 × 21.2 mm, mobile phase: 30-90 % acetonitrile/water containing 0.1 % formic acid) to obtain the final compound, 3,8-dimethyl-6-(pyridin-3-yl)pyrrolo[2,1-a]isoquinoline-1-carbonitrile (10.2 mg, yield 23 %) as a white solid.

LCMS (ESI-MS): mass calcd C₂₀H₁₅N₃ 297.1, found 297.6 [M+H]⁺.

¹H NMR (400 MHz, CDCl₃) δ 8.95-8.43 (m, 3H), 7.98-7.87 (m, 1H), 7.68-7.53 (m, 2H), 7.50-7.48 (m, 1H), 7.24 (s, 1H), 6.76 (s, 1H), 2.49 (s, 3H), 2.44 (s, 3H).

### <Experimental Example 1> Optical Properties of IK15 for Detecting Aβ Oligomers

To evaluate the potential of the 41 compounds of the present disclosure (IK15 compounds: Examples 1 to 13, 22, 26, and 27; and AMD compounds: Examples 14 to 21, 23 to 25, and 28 to 41) as imaging agents for detecting Aβ oligomers, changes in the IK15 fluorescence spectra were analyzed in the presence of Aβ monomers (mAβ), Aβ oligomers (oAβ), and Aβ fibrils (fAβ).

To prepare monomeric Aβ samples, Aβ aggregates were treated with 20 mM EPPS (4-(2-hydroxyethyl)-1-piperazinepropanesulfonic acid), which dissociates Aβ aggregates into monomeric forms. To prepare oligomeric and fibrillar Aβ, Aβ peptides were incubated according to a previously known Aβ aggregation protocol (Lee, H. Y. *et al.* Amyloid Against Amyloid: Dimeric Amyloid Fragment Ameliorates Cognitive Impairments by Direct Clearance of Oligomers and Plaques. *Angew Chem Int Ed Engl* **62,** e202210209 (2023)).

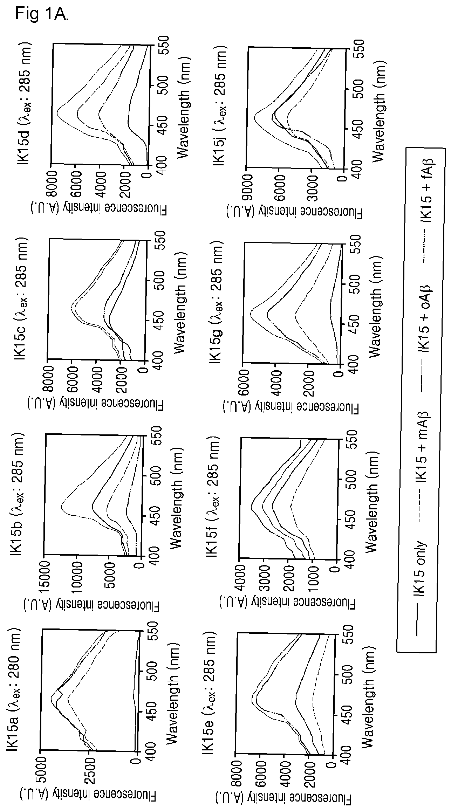

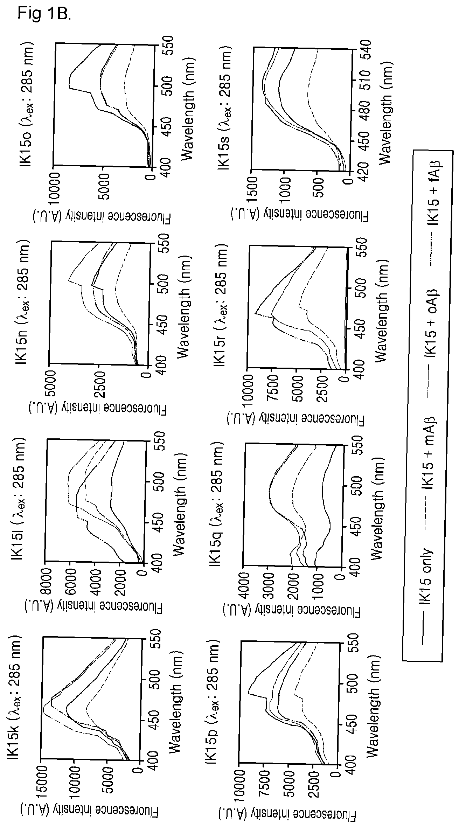

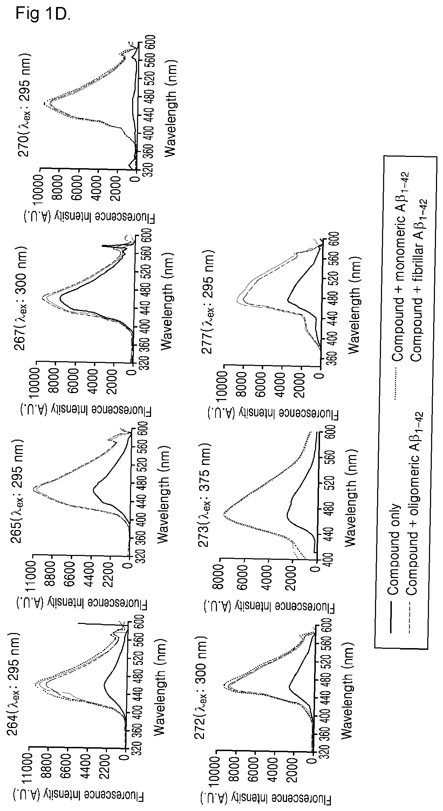

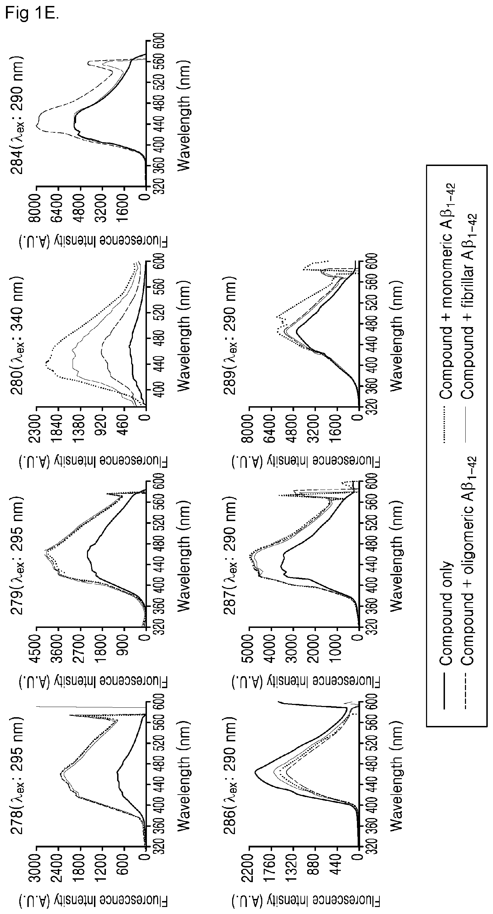

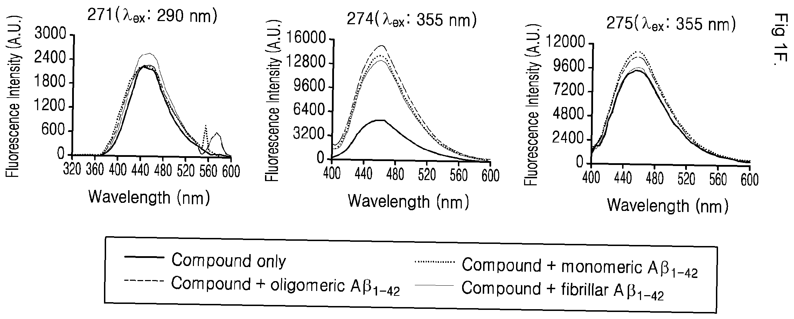

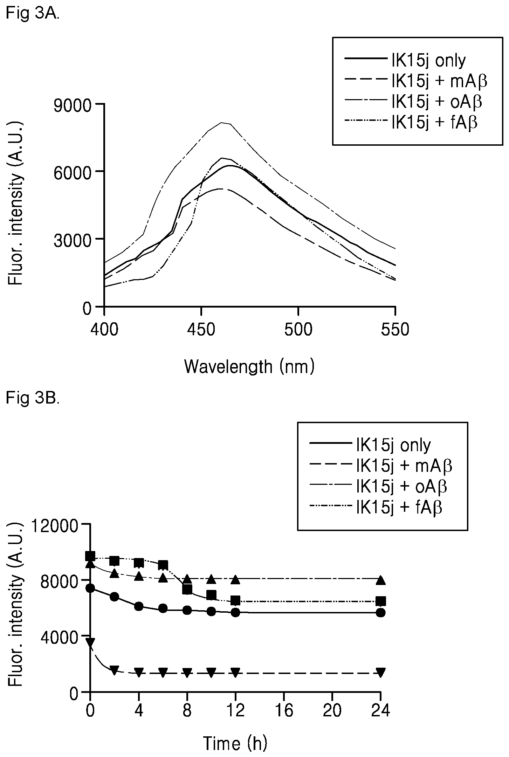

Emission scan analysis revealed that for IK15o or IK15p, the fluorescence intensity of the emission spectrum of the candidate compound mixed with Aβ was lower than that of the emission spectrum of the compound alone. Among the IK15 compounds, it was observed that the remaining 14 IK15 compounds (excluding those mentioned above) exhibited increased emission intensity when bound to Aβ monomers, Aβ oligomers, or Aβ fibrils. Among them, IK15a, IK15b, IK15c, IK15d, IK15e, IK15g, IK15j, IK15k, IK15l, and IK15n showed the greatest and most specific increase in emission intensity when bound to Aβ oligomers (FIGS. 1A, 1B, and 3A).

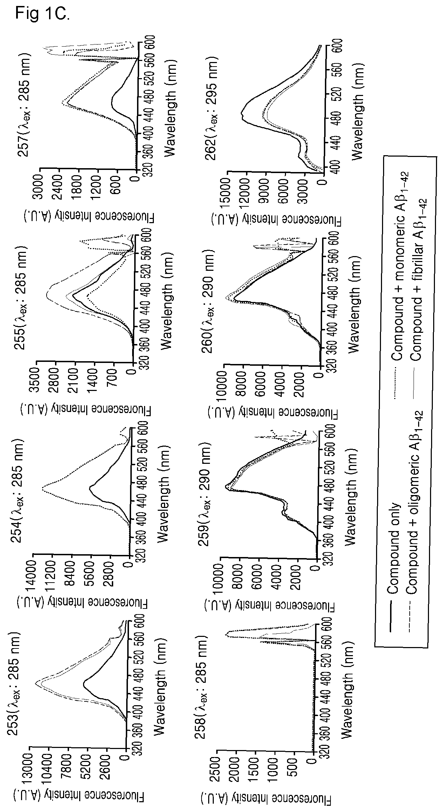

In addition, among the AMD compounds, it was confirmed that AMD-D-255 exhibited a specific increase in fluorescence emission intensity upon binding to Aβ oligomers, similar to the IK15j compound. It was confirmed that AMD-D-253, AMD-D-254, AMD-D-257, AMD-D-264, AMD-D-272, AMD-D-273, AMD-D-277, AMD-D-278, AMD-D-279, and AMD-D-280 showed low fluorescence emission intensity alone, but exhibited a specific increase in fluorescence signals when bound to Aβ monomers, Aβ oligomers, or Aβ fibrils. Furthermore, AMD-D-270 was confirmed to be an Aβ-selective fluorescent compound that showed almost no fluorescence alone but exhibited fluorescence only when bound to Aβ monomers, Aβ oligomers, or Aβ fibrils (FIGS. 1C to 1F).

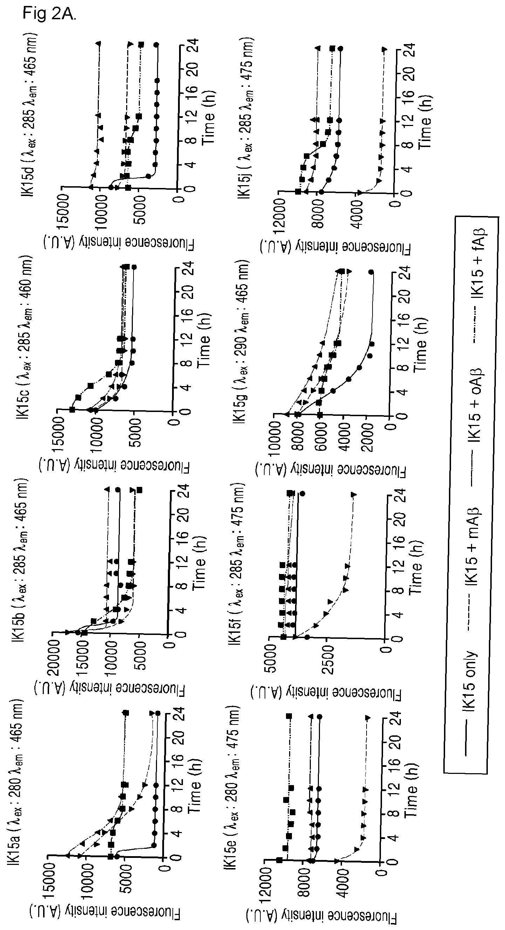

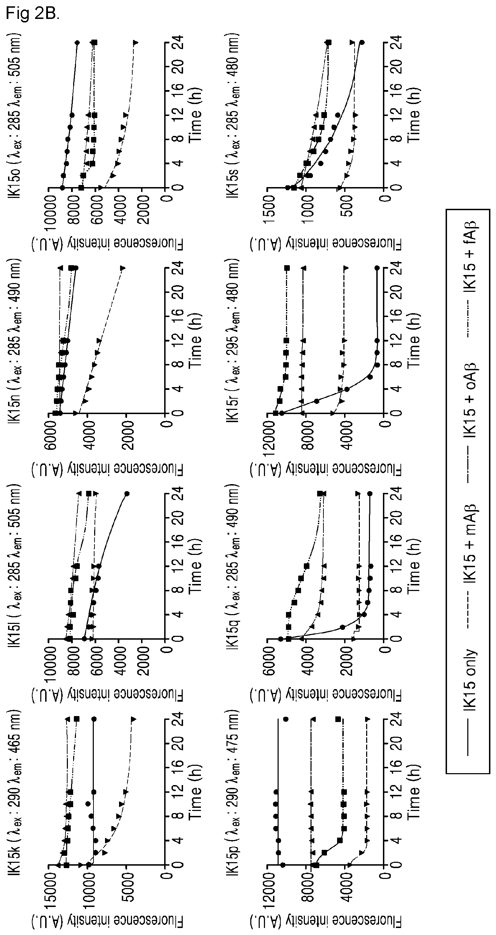

In addition, to analyze the fluorescence changes of the 16 IK15 compounds over time, the fluorescence intensities of these candidate compounds mixed with Aβ monomers (mAβ), Aβ oligomers (oAβ), and Aβ fibrils (fAβ) were monitored for 24 hours. It was confirmed that over time, the fluorescence intensity decreased due to photobleaching; however, when bound to Aβ, the signal remained stable and the fluorescence intensity decreased only slightly. Consistent with the previous emission spectrum data, it was observed that when IK15a, IK15b, IK15c, IK15d, IK15e, IK15g, IK15j, IK15k, IK15l, or IK15n were bound to Aβ oligomers, the fluorescence intensity decreased only slightly while remaining stable overall (FIGS. 2A, 2B, and 3B).

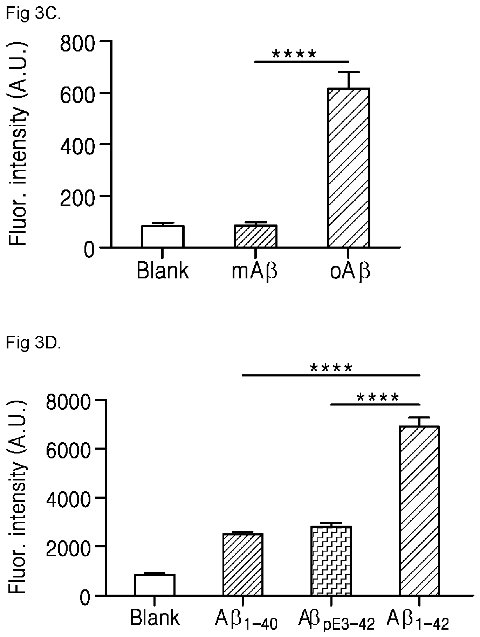

In addition, considering that EPPS might interfere with the interaction between IK15j and Aβ monomers, Aβ monomers and oligomers were immobilized on a plate according to the Aβ immobilization protocol of Reference Example 2, and then incubated with IK15j. Briefly, Aβ monomers (mAβ) and Aβ oligomers (oAβ) at 10 µM each were immobilized on a plate, treated with IK15j (100 µM) for 24 hours, and the fluorescence intensity was then measured; blank wells were treated with IK15j (100 µM) alone without immobilization of Aβ species. As a result, in contrast to Aβ monomers, the fluorescence signal of IK15j markedly increased when Aβ oligomers were used (FIG. 3C). These results confirm a specific interaction between IK15j and Aβ oligomers.

### <Experimental Example 2> Specificity of IK15 for Detecting Aβ₁₋₄₂ Oligomers

Aβ peptides exhibit variations in length or N-/C-terminal modifications, and Aβ₁₋₄₀, Aβ₁₋₄₂, and pyroglutamate Aβ₃₋₄₂ (Aβ_{pE3-42}) are most commonly found in the brains of AD patients. The difference between Aβ₁₋₄₀ and Aβ₁₋₄₂ is the presence of two additional residues at the C-terminus of Aβ₁₋₄₂. Aβ_{pE3-42} is produced through multistep protein modification of Aβ₁₋₄₂, *wherein* glutamate at *position* 3 of the N-terminus is converted to pyroglutamate.

Accordingly, Aβ variant oligomers were prepared and incubated with IK15j to investigate the selectivity of the compound for Aβ variants. Briefly, samples of oligomeric Aβ₁₋₄₀, Aβ₁₋₄₂, and Aβ_{pE3-42} (10 µM each) were each treated with IK15j (10 µM), and the fluorescence intensity of each Aβ variant sample was measured. As a result, the fluorescence intensity of IK15j increased markedly in the presence of Aβ₁₋₄₂ oligomers compared to that of Aβ₁₋₄₀ or Aβ_{pE3-42} oligomers (FIG. 3D).

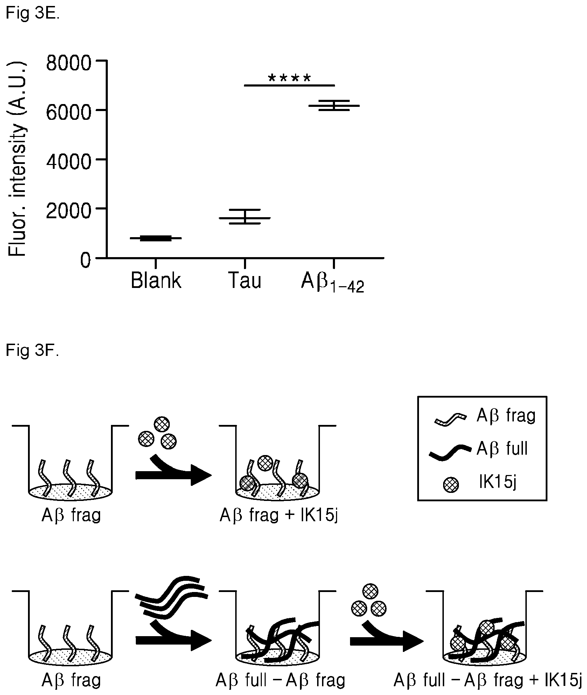

Considering that tau protein aggregation is also a major pathological feature of AD, the selectivity of IK15j for Aβ₁₋₄₂ over tau domains was further evaluated. Tau protein possesses four repeat domains, and repeat domain 3 is associated with the microtubule-binding domain of tau protein (Le, L. et al. Self-Aggregating Tau Fragments Recapitulate Pathologic Phenotypes and Neurotoxicity of Alzheimer's Disease in Mice. Adv Sci (Weinh), e2302035 (2023)). Since repeat domain 3 is a core region of tau aggregates, IK15j was incubated with the tau domain or with Aβ₁₋₄₂, and the resulting changes in fluorescence intensity were compared. Briefly, Aβ₁₋₄₂ and tau aggregate samples were each treated with IK15j, and the fluorescence intensity was measured. As a result, the signal of IK15j increased in the presence of Aβ₁₋₄₂ oligomers compared to that of the tau domain (FIG. 3E). These results indicate that IK15j selectively binds to Aβ₁₋₄₂ oligomers.

### <Experimental Example 3> Identification of the IK15 Binding Site on Aβ₁₋₄₂ Oligomers

Although Aβ aggregates are considered neuropathogenic biomarkers of AD, identifying targeting sites for Aβ imaging probes remains challenging due to the transient and polymorphic nature of Aβ. Experiments were performed according to Reference Examples 2 and 3 to identify the IK15 binding site on Aβ₁₋₄₂ oligomers. Briefly, 37 hexamer fragments of Aβ₁₋₄₂, denoted as Aβₙ₋₍ₙ₊₅₎ (n = 1 to 37), were synthesized and immobilized on a plate to generate a MAP. Two MAP conditions were prepared to investigate whether IK15 targets a specific sequence of Aβ₁₋₄₂ or the structure of Aβ₁₋₄₂ oligomers. First, to identify the targeting sequence of IK15j, the MAP was treated with Aβₙ₋₍ₙ₊₅₎ + IK15j for 24 hours. Second, to induce Aβ-Aβ formation, the MAP was incubated with additional Aβ₁₋₄₂ peptides for 8 hours. Subsequently, the MAP was treated with IK15j for 24 hours to determine the targeting position of the compound within Aβ₁₋₄₂ oligomers (FIG. 3F).

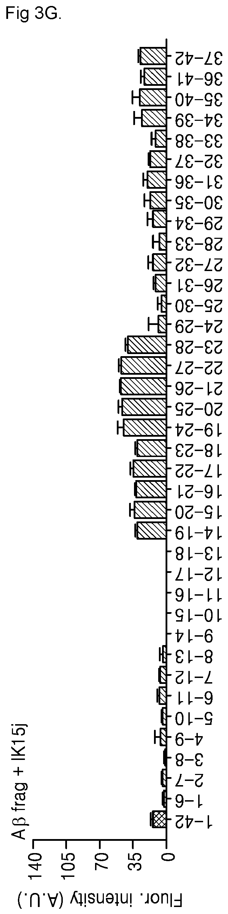

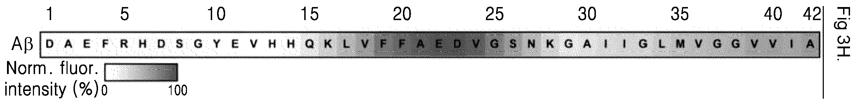

As a result, an increase in IK15j signal was observed in 10 Aβ fragments (Aβ₁₄₋₁₉ to Aβ₂₃₋₂₈) compared to Aβ₁₋₄₂ (FIG. 3G). Although a heatmap using a white-and-red two-color gradient indicated an interaction between IK15j and the Aβ₁₉₋₂₅ region (SEQ ID NO: 1; FFAEDVG), the IK15j signal was relatively low across all fragments (FIG. 3H), indicating that IK15j does not bind to a specific sequence of Aβ₁₋₄₂. These results suggest that IK15j cannot detect monomeric Aβ₁₋₄₂ because the compound does not interact with a specific sequence of Aβ₁₋42.

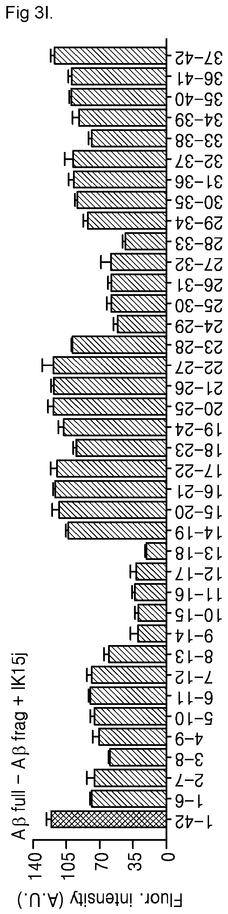

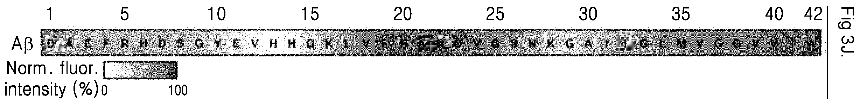

In addition, changes in the fluorescence intensity of IK15j were measured for each complex formed between Aβ₁₋₄₂ fragments and Aβ₁₋₄₂. As a result, the overall signal of IK15j increased in the presence of Aβ₁₋₄₂-Aβₙ₋₍ₙ₊₅₎ complexes, indicating that IK15j interacts with the structure of Aβ oligomers. This compound bound strongly to complexes of Aβ₁₋₄₂ with two major domains: Aβ₁₉₋₂₅ (SEQ ID NO: 1; FFAEDVG) and Aβ₃₅₋₄₂ (SEQ ID NO: 2; MVGGVVIA) (FIGS. 3I and 3J).

The central hydrophobic region containing the KLVFFA domain is known to be the most aggregation-prone sequence in Aβ₁₋₄₂, and the GGVVIA domain is a hydrophobic C-terminal core that stabilizes the hydrophobic interactions of Aβ. These findings confirm that IK15j targets the hydrophobic core region of Aβ oligomers. In particular, this compound exhibited moderate binding affinity for the N-terminus of Aβ. This hydrophilic region, Aβ₁₋₈ (SEQ ID NO: 3; DAEFRHDS), is known to alter the aggregation propensity of Aβ and influence the secondary structure of Aβ oligomers. IK15j interacts with both hydrophobic and amphiphilic regions of Aβ oligomers, thereby enabling stable binding to Aβ oligomers. Collectively, these results indicate that IK15j interacts with the structure of Aβ oligomers and that the compound specifically targets the hydrophobic core of Aβ aggregates and the hydrophilic N-terminus of Aβ oligomers.

### <Experimental Example 4> Detection of Soluble Aβ Oligomers in Brain Lysates, CSF, and Plasma of 5XFAD Mouse Models

The 5XFAD transgenic AD mouse model expresses human APP and presenilin-1, thereby exhibiting major pathological features of AD, including Aβ aggregate formation in the brain.

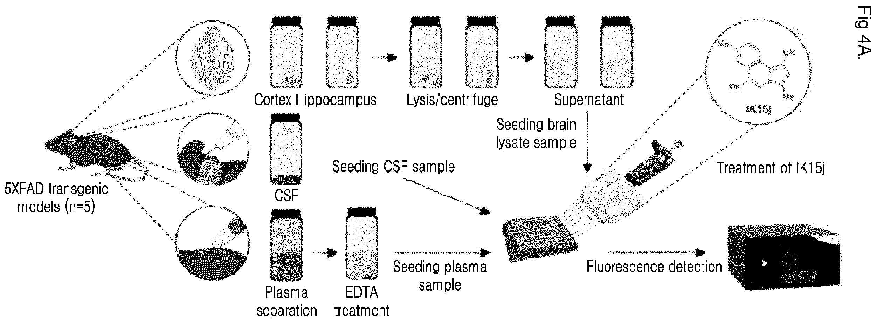

The *ex vivo* experimental protocol for IK15 using the 5XFAD mouse model is shown in FIG. 4A. Specifically, as described in Reference Example 4, since soluble Aβ species are detectable from 2 months of age in the 5XFAD transgenic mouse model and their levels increase significantly with age, 6-month-old female 5XFAD transgenic mice (n = 3) and age-matched female wild-type mice (n = 3) were prepared. Subsequently, each mouse was sacrificed to obtain brain lysates (cortex and hippocampus), CSF, and plasma as described in Reference Examples 5 to 7.

Brain lysates (cortex and hippocampus) and CSF from 5XFAD or wild-type mice were each treated with the 16 IK15 example compounds at 50 µM. Thereafter, each sample was placed in the wells of a 96-well half-area black microplate. The total volume of each sample was 100 µL. After treatment with the compounds of the aforementioned Examples for 24 hours, samples were scanned at the respective excitation/emission wavelengths of each compound to monitor changes in fluorescence signals, and the fluorescence was detected using a microplate reader.

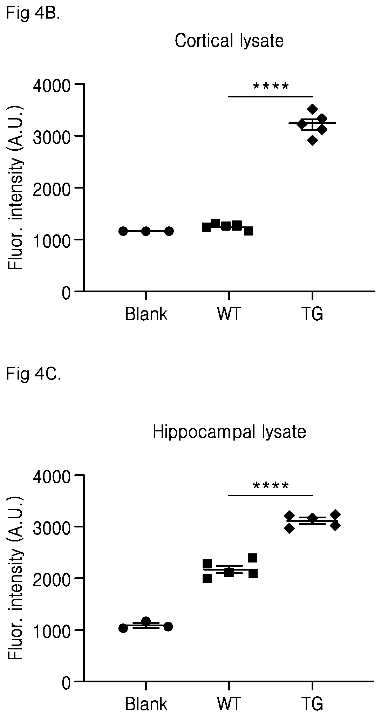

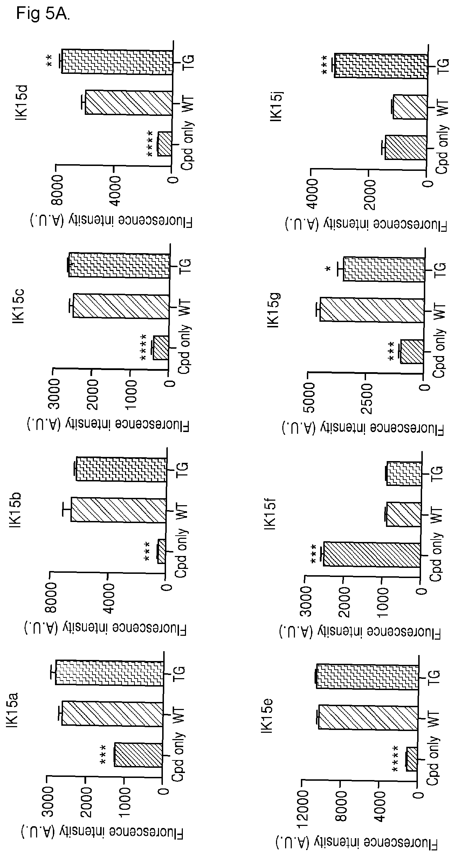

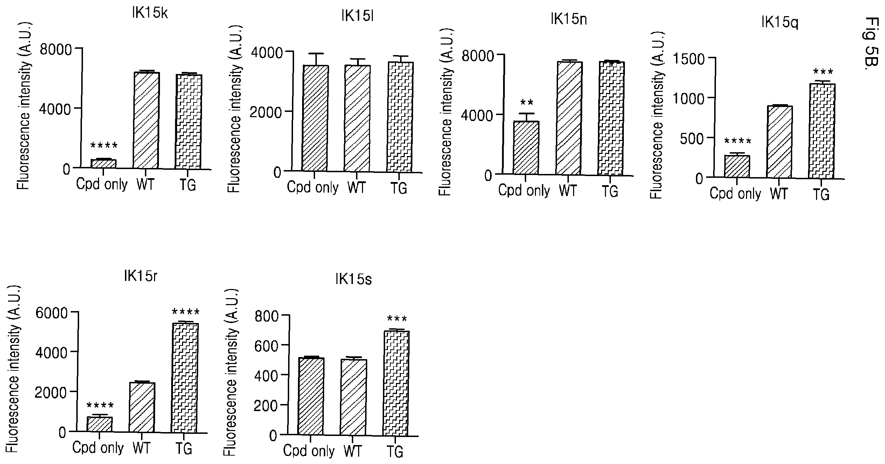

As a result, cortical samples treated with IK15d, IK15g, IK15j, IK15q, IK15r, or IK15s showed a significant difference in fluorescence intensity between 5XFAD transgenic mice and wild-type mice. In the analysis using IK15d, IK15j, IK15q, IK15r, or IK15s, the fluorescence intensity increased in the 5XFAD transgenic mouse cortex, whereas in the analysis using IK15g, the fluorescence intensity increased in the wild-type mouse cortex. These results confirmed that cortical analysis using six IK15 compounds (IK15d, IK15g, IK15j, IK15q, IK15r, and IK15s) could distinguish between 5XFAD transgenic mice and wild-type mice (FIGS. 4B, 5A, and 5B).

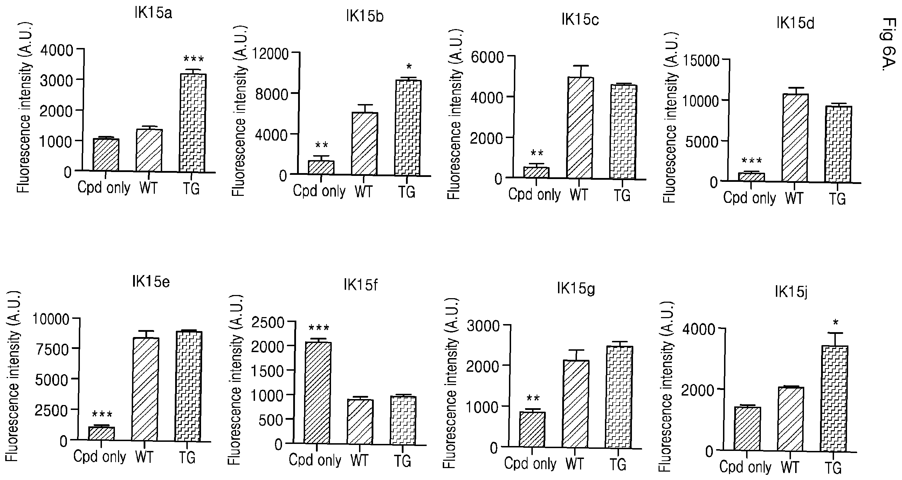

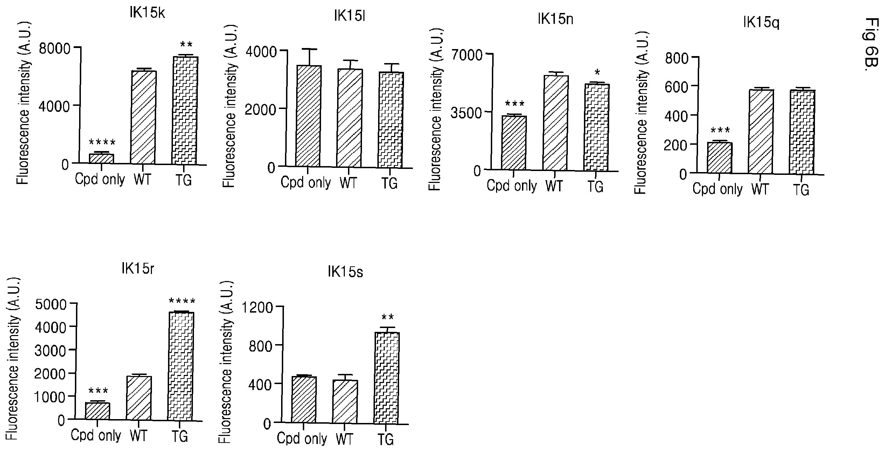

In addition, hippocampal samples treated with IK15a, IK15b, IK15j, IK15k, IK15n, IK15r, or IK15s showed a significant difference in fluorescence intensity between 5XFAD transgenic mice and wild-type mice. In the analysis using IK15a, IK15b, IK15j, IK15k, IK15r, or IK15s, the fluorescence intensity increased in the 5XFAD transgenic mouse hippocampus, whereas in the analysis using IK15n, the fluorescence intensity increased in the wild-type mouse hippocampus. These results confirmed that hippocampal analysis using seven IK15 compounds (IK15a, IK15b, IK15j, IK15k, IK15n, IK15r, and IK15s) could distinguish between 5XFAD transgenic mice and wild-type mice (FIGS. 4C, 6A, and 6B).

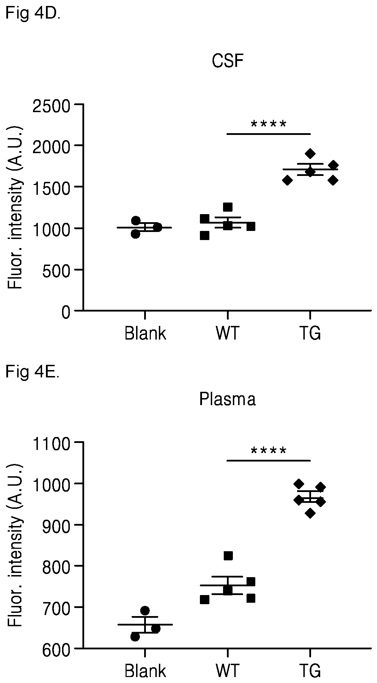

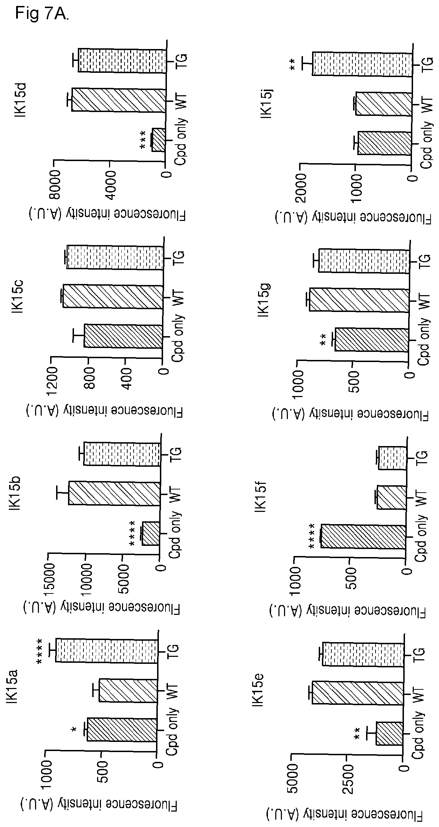

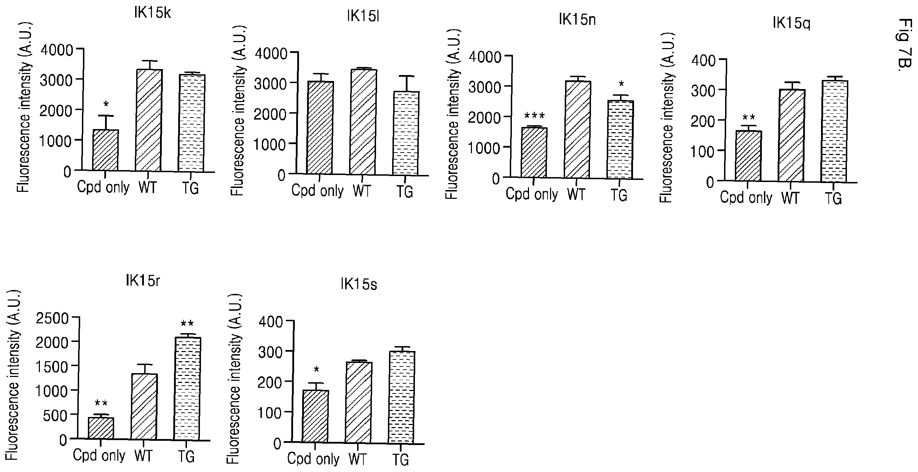

Furthermore, CSF samples treated with IK15a, IK15j, IK15n, or IK15r showed a significant difference in fluorescence intensity between 5XFAD transgenic mice and wild-type mice. In the analysis using IK15a, IK15j, or IK15r, the fluorescence intensity increased in the CSF of 5XFAD transgenic mice, whereas in the analysis using IK15n, the fluorescence intensity increased in the CSF of wild-type mice. These results confirmed that CSF analysis using four IK15 compounds (IK15a, IK15j, IK15n, and IK15r) could distinguish between 5XFAD transgenic mice and wild-type mice (FIGS. 4D, 7A, and 7B).

To conduct an analysis between Aβ oligomers in plasma and the IK15 compounds using the four IK15 compounds (IK15a, IK15j, IK15r, and IK15s) selected from the above experiments for their high binding capacity to Aβ oligomers, plasma samples were prepared from 6-month-old female 5XFAD transgenic (TG) mice (n = 3) and age-matched female wild-type (WT) mice (n = 3). After collecting each plasma sample and treating it with EDTA, each sample was treated with the respective IK15 compound (50 µM) and placed in the wells of a 96-well half-area black microplate. The total volume of each sample was 50 µL. To monitor changes in fluorescence signals after 24 hours, each of the 4 compounds was scanned at the respective excitation/emission wavelengths, and the fluorescence was detected using a microplate reader.

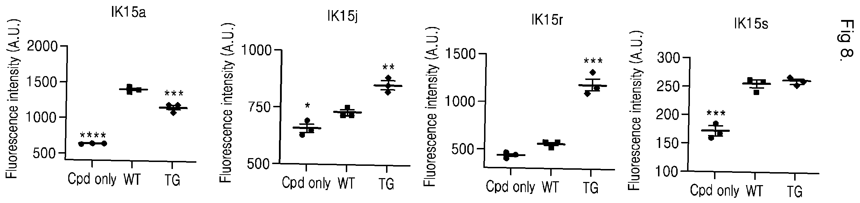

As a result, plasma analysis using IK15a, IK15j, or IK15r among the four candidate compounds showed a significant difference in fluorescence intensity between 5XFAD transgenic mice and wild-type mice. For IK15a, the fluorescence intensity increased in wild-type mouse plasma, whereas for IK15j and IK15r, the fluorescence intensity increased in 5XFAD transgenic mouse plasma. These results confirmed that plasma analysis using three IK15 compounds (IK15a, IK15j, and IK15r) could distinguish between 5XFAD transgenic mice and wild-type mice (FIGS. 4E and 8).

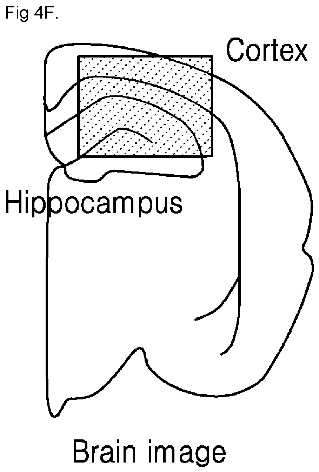

In addition, to verify whether IK15j accurately targeted Aβ oligomers, brain staining was performed in the hippocampal and cortical regions using IK15j and the anti-Aβ antibody 6E10 as described in Reference Example 8 to visualize the colocalization of signals (FIG. 4F).

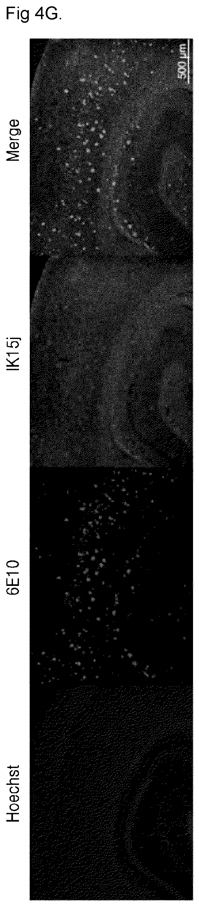

As a result, as shown in FIGS. 4F and 4G, no signal for IK15j was observed in either the hippocampal or cortical regions, confirming that IK15j does not detect insoluble plaques in the 5XFAD mouse brain.

These findings confirm that IK15j selectively detects Aβ oligomers in the brain lysates, CSF, and plasma of the 5XFAD mouse model.

### <Experimental Example 5> Confirmation of Interaction Between IK15 and Plasma Aβ

To investigate whether the compound of the present disclosure interacts with Aβ oligomers in plasma, Aβ oligomers were spiked into human plasma (at 100, 10, 1, 0.1, 0.01, 0.001, and 0 pg/mL) and treated with IK15j (100 µM) for 8 hours, as described in Reference Example 9.

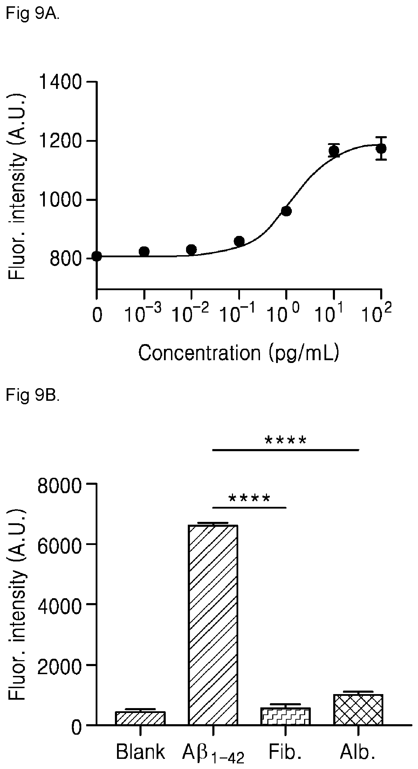

As a result, the signal of IK15j increased significantly in the 1 pg/mL spiked sample compared to blank human plasma (0 pg/mL). Compared to the control, the fluorescence intensity of the 10-100 pg/mL Aβ spiked samples exhibited a 52 % increase (FIG. 9A).

### <Experimental Example 6> Confirmation of Interaction Between the Compound of the Present Disclosure and Plasma Proteins

Excluding water, the solid components of plasma consist of coagulation factors and plasma proteins. Albumin is the most abundant protein in plasma, and fibrinogen is a major coagulation factor in plasma. Considering the possibility that fibrinogen or albumin might interact with AD diagnostic tools and interfere with compound selectivity, IK15j (25 µM) was incubated with Aβ₁₋₄₂, albumin, or fibrinogen as described in Reference Example 10, and the fluorescence signal of each sample was measured to evaluate the selectivity of the compound of the present disclosure against these proteins.

As a result, the fluorescence intensity of IK15j increased markedly in the presence of Aβ₁₋₄₂ oligomers compared to other proteins. In contrast, IK15j did not interact with fibrinogen or albumin (FIG. 9B).

In addition, to investigate whether albumin interferes with the binding between IK15j and Aβ₁₋₄₂ oligomers, IK15j was co-incubated with Aβ₁₋₄₂ oligomers and albumin as described in Reference Example 11, and the fluorescence signal of each sample was analyzed.

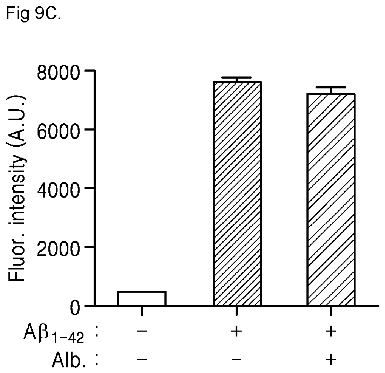

As a result, it was observed that while the signal from the Aβ₁₋₄₂ oligomer and albumin sample decreased compared to the Aβ₁₋₄₂ oligomer-only sample, the difference was not significant (FIG. 9C).

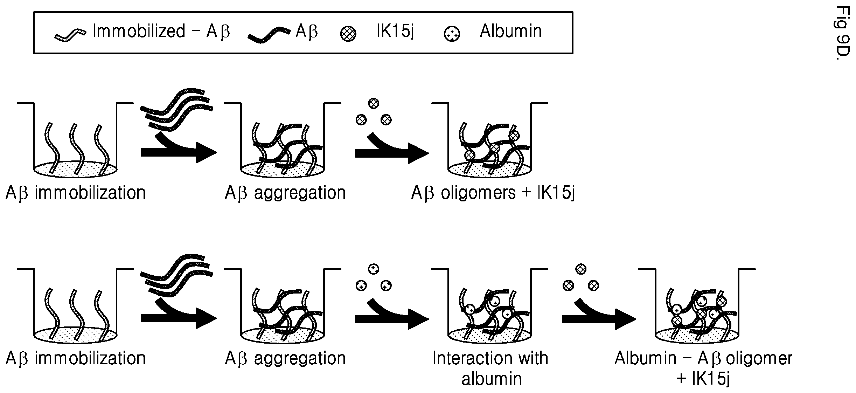

In addition, to confirm that albumin does not mask the target site of IK15j, Aβ oligomer-albumin complexes and Aβ oligomers were each immobilized on a plate as described in Reference Example 11. Subsequently, both wells were treated with IK15j (25 µM), and the signals were compared (FIG. 9D).

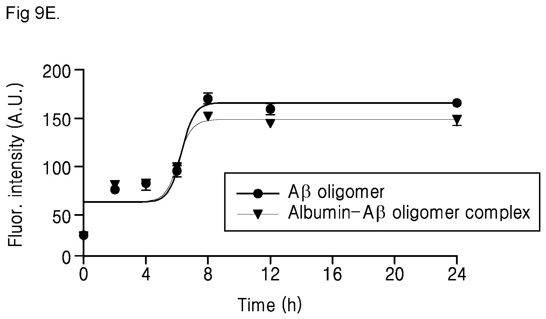

As a result, no significant difference in fluorescence intensity was observed between the Aβ oligomer-albumin complex and Aβ oligomers alone. These results confirm that IK15j interacts with both Aβ oligomers and Aβ oligomer-albumin complexes in plasma, indicating that albumin does not mask the target site of IK15j (FIG. 9E).

The foregoing description of the present disclosure is for illustrative purposes, and it will be understood by those of ordinary skill in the art to which the present disclosure pertains that the present disclosure can be easily modified into other specific forms without changing its technical spirit or essential features. Therefore, the examples described above should be understood as being illustrative in all aspects and not limiting.

## Claims

1. A compound represented by Formula 1 or a salt thereof: 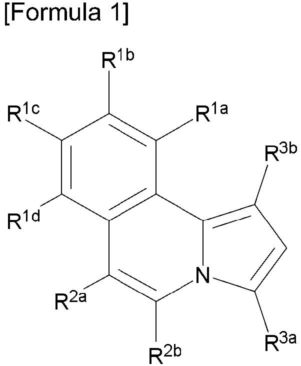 wherein, in Formula 1,
R^{1a} and R^{1b} are each independently hydrogen, deuterium, halogen, amino, hydroxy, substituted or unsubstituted C₁₋₁₀ alkyl, substituted or unsubstituted C₂₋₁₀ alkenyl, substituted or unsubstituted C₂₋₁₀ alkynyl, substituted or unsubstituted C₁₋₁₀ alkoxy, substituted or unsubstituted 6- to 10-membered aryl, or substituted or unsubstituted 5- to 10-membered heteroaryl;
R^{1c} and R^{1d} are each independently hydrogen, deuterium, halogen, amino, hydroxy, substituted or unsubstituted C₁₋₁₀ alkyl, substituted or unsubstituted C₂₋₁₀ alkenyl, substituted or unsubstituted C₂₋₁₀ alkynyl, substituted or unsubstituted C₁₋₁₀ alkoxy, substituted or unsubstituted 6- to 10-membered aryl, or substituted or unsubstituted 5- to 10-membered heteroaryl, or
R^{1c} and R^{1d} are connected to each other to form a compound represented by Formula 2; 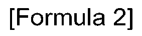 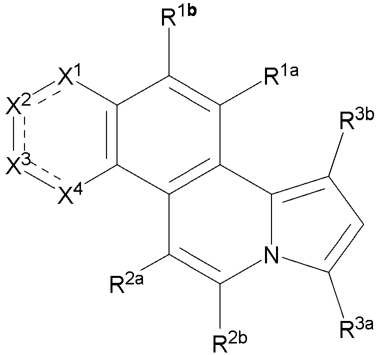
wherein, in Formula 2, X¹, X², X³, and X⁴ are each independently C(R⁴), nitrogen, oxygen, sulfur, or phosphorus;
R⁴ is hydrogen, deuterium, halogen, amino, hydroxy, substituted or unsubstituted C₁₋₁₀ alkyl, substituted or unsubstituted C₂₋₁₀ alkenyl, substituted or unsubstituted C₂₋₁₀ alkynyl, substituted or unsubstituted C₁₋₁₀ alkoxy, substituted or unsubstituted 6- to 10-membered aryl, or substituted or unsubstituted 5- to 10-membered heteroaryl;
R^{2a} and R^{2b} are each independently hydrogen, deuterium, halogen, amino, hydroxy, substituted or unsubstituted C₁₋₁₀ alkyl, substituted or unsubstituted C₂₋₁₀ alkenyl, substituted or unsubstituted C₂₋₁₀ alkynyl, substituted or unsubstituted C₁₋₁₀ alkoxy, substituted or unsubstituted 6- to 10-membered aryl, or substituted or unsubstituted 5- to 10-membered heteroaryl; and
R^{3a} and R^{3b} are each independently hydrogen, deuterium, halogen, amino, hydroxy, -CN, -C(O)NH₂, -C(O)NH₂, -C(O)O(C₁₋₁₀ alkyl), substituted or unsubstituted C₁₋₁₀ alkyl, substituted or unsubstituted 6- to 10-membered aryl, or substituted or unsubstituted 5- to 10-membered heteroaryl.

2. The compound or salt of claim 1,
wherein R^{1a} and R^{1b}are each independently hydrogen, deuterium, or halogen;
R^{1c} and R^{1d} are each independently hydrogen, deuterium, halogen, amino, hydroxy, substituted or unsubstituted C₁₋₅ alkyl, substituted or unsubstituted C₂₋₅ alkenyl, substituted or unsubstituted C₂₋₅ alkynyl, substituted or unsubstituted C₁₋₅ alkoxy, substituted or unsubstituted 6-membered aryl, or substituted or unsubstituted 6-membered heteroaryl, or
R^{1c} and R^{1d} are connected to each other to form the compound represented by Formula 2, and
X¹, X², X³, and X⁴ are each independently C(R⁴), nitrogen, oxygen, sulfur, or phosphorus, and
R⁴ is hydrogen, deuterium, or halogen;
R^{2a} and R^{2b} are each independently hydrogen, deuterium, halogen, substituted or unsubstituted C₁₋₅ alkyl, substituted or unsubstituted C₂₋₅ alkenyl, substituted or unsubstituted C₂₋₅ alkynyl, substituted or unsubstituted 6- to 10-membered aryl, or substituted or unsubstituted 5- to 10-membered heteroaryl; and
R^{3a} and R^{3b} are each independently hydrogen, deuterium, halogen, amino, hydroxy, -CN, -C(O)NH₂, -C(O)NH₂, -C(O)O(C₁₋₅ alkyl), substituted or unsubstituted C₁₋₅ alkyl, or substituted or unsubstituted 6- to 10-membered aryl.

3. The compound or salt of claim 1,
wherein R^{1a} and R^{1b} are each independently hydrogen or halogen; R^{1c} and R^{1d} are each independently hydrogen, halogen, substituted or unsubstituted C₁₋₅ alkyl, substituted or unsubstituted C₁₋₅ alkoxy, or substituted or unsubstituted 6-membered aryl, or
R^{1c} and R^{1d} are connected to each other to form the compound represented by Formula 2, and
X¹, X², X³, and X⁴ are each independently C(R⁴), and R⁴ is hydrogen;
R^{2a} and R^{2b} are each independently hydrogen, halogen, substituted or unsubstituted C₁₋₅ alkyl, substituted or unsubstituted 6- to 10-membered aryl, or substituted or unsubstituted 5- to 6-membered heteroaryl; and
R^{3a} and R^{3b} are each independently hydrogen, -CN, -C(O)NH₂, - C(O)O(C₁₋₅ alkyl), substituted or unsubstituted C₁₋₅ alkyl, or substituted or unsubstituted 6-membered aryl.

4. A method of preparing a compound represented by Formula 1, the method comprising, as shown in Reaction Scheme 1:
obtaining Compound 1003 by reacting Compound 1001 and Compound 1002 in an organic solvent (Step 1);
obtaining Compound 1004 by reacting Compound 1003 and NH₄OAc in an organic solvent (Step 2);
obtaining Compound 1006 by reacting Compound 1004 with Compound 1005-1, Compound 1005-2, or Compound 1005-3 in an organic solvent (Step 3); and
obtaining the compound represented by Formula 1 (Compound 1007) by reacting Compound 1006 and a catalyst in an organic solvent (Step 4): 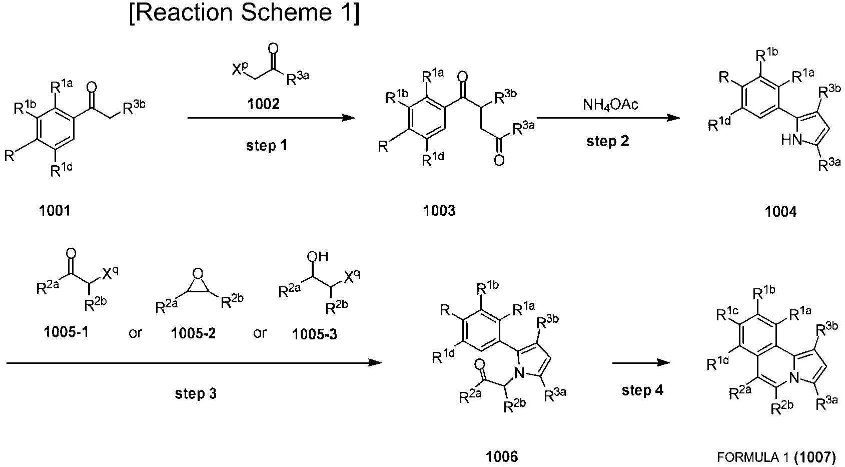
wherein, in Reaction Scheme 1,
R is R^{1c} or X^{q};
X^{p} and X^{q} are each independently hydrogen, deuterium, or halogen; R^{1a} and R^{1b} are each independently hydrogen, deuterium, halogen, amino, hydroxy, substituted or unsubstituted C₁₋₁₀ alkyl, substituted or unsubstituted C₂₋₁₀ alkenyl, substituted or unsubstituted C₂₋₁₀ alkynyl, substituted or unsubstituted C₁₋₁₀ alkoxy, substituted or unsubstituted 6- to 10-membered aryl, or substituted or unsubstituted 5- to 10-membered heteroaryl;
R^{1c} and R^{1d} are each independently hydrogen, deuterium, halogen, amino, hydroxy, substituted or unsubstituted C₁₋₁₀ alkyl, substituted or unsubstituted C₂₋₁₀ alkenyl, substituted or unsubstituted C₂₋₁₀ alkynyl, substituted or unsubstituted C₁₋₁₀ alkoxy, substituted or unsubstituted 6- to 10-membered aryl, or substituted or unsubstituted 5- to 10-membered heteroaryl, or
R^{1c} and R^{1d} are connected to each other to form a compound represented by Formula 2; 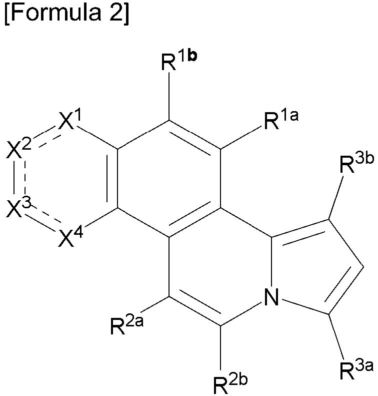
wherein, in Formula 2, X¹, X², X³, and X⁴ are each independently C(R⁴), nitrogen, oxygen, sulfur, or phosphorus;
R⁴ is hydrogen, deuterium, halogen, amino, hydroxy, substituted or unsubstituted C₁₋₁₀ alkyl, substituted or unsubstituted C₂₋₁₀ alkenyl, substituted or unsubstituted C₂₋₁₀ alkynyl, substituted or unsubstituted C₁₋₁₀ alkoxy, substituted or unsubstituted 6- to 10-membered aryl, or substituted or unsubstituted 5- to 10-membered heteroaryl;
R^{2a} and R^{2b} are each independently hydrogen, deuterium, halogen, amino, hydroxy, substituted or unsubstituted C₁₋₁₀ alkyl, substituted or unsubstituted C₂₋₁₀ alkenyl, substituted or unsubstituted C₂₋₁₀ alkynyl, substituted or unsubstituted C₁₋₁₀ alkoxy, substituted or unsubstituted 6- to 10-membered aryl, or substituted or unsubstituted 5- to 10-membered heteroaryl; and
R^{3a} and R^{3b} are each independently hydrogen, deuterium, halogen, amino, hydroxy, -CN, -C(O)NH₂, -C(O)NH₂, -C(O)O(C₁₋₁₀ alkyl), substituted or unsubstituted C₁₋₁₀ alkyl, substituted or unsubstituted 6- to 10-membered aryl, or substituted or unsubstituted 5- to 10-membered heteroaryl.

5. The method of claim 4, wherein the organic solvent in each of Steps 1 to 4 is each independently at least one selected from the group consisting of methanol, ethanol, propanol, acetonitrile, dichloroethane (DCE), ethyl acetate, dimethyl sulfoxide, and dimethylformamide.

6. The method of claim 4, wherein the catalyst in Step 4 is at least one selected from the group consisting of trifluoromethanesulfonic acid (TfOH), methanesulfonic acid (MsOH), p-toluenesulfonic acid (p-TsOH), and scandium triflate (Sc(OTf)₃).

7. A composition for detecting amyloid-beta (Aβ), the composition comprising a compound represented by Formula 1 or a pharmaceutically acceptable salt thereof: 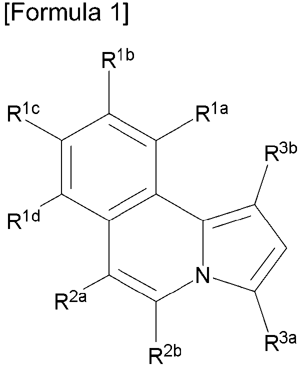 wherein, in Formula 1,
R^{1a} and R^{1b} are each independently hydrogen, deuterium, halogen, amino, hydroxy, substituted or unsubstituted C₁₋₁₀ alkyl, substituted or unsubstituted C₂₋₁₀ alkenyl, substituted or unsubstituted C₂₋₁₀ alkynyl, substituted or unsubstituted C₁₋₁₀ alkoxy, substituted or unsubstituted 6- to 10-membered aryl, or substituted or unsubstituted 5- to 10-membered heteroaryl;
R^{1c} and R^{1d} are each independently hydrogen, deuterium, halogen, amino, hydroxy, substituted or unsubstituted C₁₋₁₀ alkyl, substituted or unsubstituted C₂₋₁₀ alkenyl, substituted or unsubstituted C₂₋₁₀ alkynyl, substituted or unsubstituted C₁₋₁₀ alkoxy, substituted or unsubstituted 6- to 10-membered aryl, or substituted or unsubstituted 5- to 10-membered heteroaryl, or
R^{1c} and R^{1d} are connected to each other to form a compound represented by Formula 2; 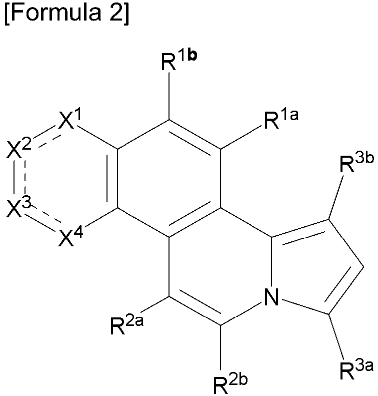
wherein, in Formula 2, X¹, X², X³, and X⁴ are each independently C(R⁴), nitrogen, oxygen, sulfur, or phosphorus;
R⁴ is hydrogen, deuterium, halogen, amino, hydroxy, substituted or unsubstituted C₁₋₁₀ alkyl, substituted or unsubstituted C₂₋₁₀ alkenyl, substituted or unsubstituted C₂₋₁₀ alkynyl, substituted or unsubstituted C₁₋₁₀ alkoxy, substituted or unsubstituted 6- to 10-membered aryl, or substituted or unsubstituted 5- to 10-membered heteroaryl;
R^{2a} and R^{2b} are each independently hydrogen, deuterium, halogen, amino, hydroxy, substituted or unsubstituted C₁₋₁₀ alkyl, substituted or unsubstituted C₂₋₁₀ alkenyl, substituted or unsubstituted C₂₋₁₀ alkynyl, substituted or unsubstituted C₁₋₁₀ alkoxy, substituted or unsubstituted 6- to 10-membered aryl, or substituted or unsubstituted 5- to 10-membered heteroaryl; and
R^{3a} and R^{3b} are each independently hydrogen, deuterium, halogen, amino, hydroxy, -CN, -C(O)NH₂, -C(O)NH₂, -C(O)O(C₁₋₁₀ alkyl), substituted or unsubstituted C₁₋₁₀ alkyl, substituted or unsubstituted 6- to 10-membered aryl, or substituted or unsubstituted 5- to 10-membered heteroaryl.

8. The composition for detecting amyloid-beta (Aβ) of claim 7, wherein the Aβ is at least one selected from the group consisting of Aβ oligomers, Aβ protofibrils, Aβ fibrils, and Aβ plaques.

9. The composition for detecting amyloid-beta (Aβ) of claim 7, wherein the Aβ comprises a variant of Aβ.

10. The composition for detecting amyloid-beta (Aβ) of claim 7, wherein the compound represented by Formula 1 or a pharmaceutically acceptable salt thereof interacts with one or more domains selected from the group consisting of a domain comprising the amino acid sequence of SEQ ID NO: 1, a domain comprising the amino acid sequence of SEQ ID NO: 2, and a domain comprising the amino acid sequence of SEQ ID NO: 3.

11. A method of detecting amyloid-beta (Aβ), the method comprising contacting an isolated biological sample with a compound represented by Formula 1 or a pharmaceutically acceptable salt thereof: 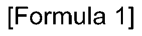 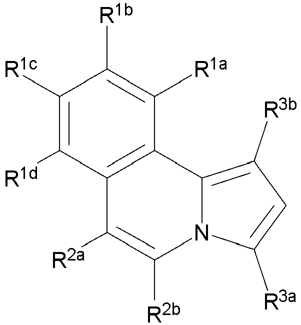 wherein, in Formula 1,
R^{1a} and R^{1b} are each independently hydrogen, deuterium, halogen, amino, hydroxy, substituted or unsubstituted C₁₋₁₀ alkyl, substituted or unsubstituted C₂₋₁₀ alkenyl, substituted or unsubstituted C₂₋₁₀ alkynyl, substituted or unsubstituted C₁₋₁₀ alkoxy, substituted or unsubstituted 6- to 10-membered aryl, or substituted or unsubstituted 5- to 10-membered heteroaryl;
R^{1c} and R^{1d} are each independently hydrogen, deuterium, halogen, amino, hydroxy, substituted or unsubstituted C₁₋₁₀ alkyl, substituted or unsubstituted C₂₋₁₀ alkenyl, substituted or unsubstituted C₂₋₁₀ alkynyl, substituted or unsubstituted C₁₋₁₀ alkoxy, substituted or unsubstituted 6- to 10-membered aryl, or substituted or unsubstituted 5- to 10-membered heteroaryl, or
R^{1c} and R^{1d} are connected to each other to form a compound represented by Formula 2; 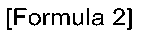 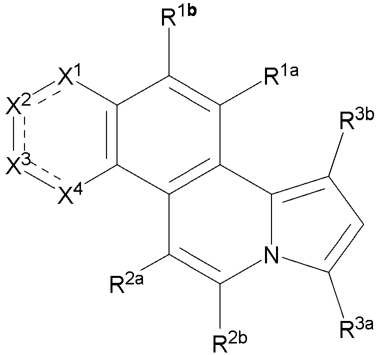
wherein, in Formula 2, X¹, X², X³, and X⁴ are each independently C(R⁴), nitrogen, oxygen, sulfur, or phosphorus;
R⁴ is hydrogen, deuterium, halogen, amino, hydroxy, substituted or unsubstituted C₁₋₁₀ alkyl, substituted or unsubstituted C₂₋₁₀ alkenyl, substituted or unsubstituted C₂₋₁₀ alkynyl, substituted or unsubstituted C₁₋₁₀ alkoxy, substituted or unsubstituted 6- to 10-membered aryl, or substituted or unsubstituted 5- to 10-membered heteroaryl;
R^{2a} and R^{2b} are each independently hydrogen, deuterium, halogen, amino, hydroxy, substituted or unsubstituted C₁₋₁₀ alkyl, substituted or unsubstituted C₂₋₁₀ alkenyl, substituted or unsubstituted C₂₋₁₀ alkynyl, substituted or unsubstituted C₁₋₁₀ alkoxy, substituted or unsubstituted 6- to 10-membered aryl, or substituted or unsubstituted 5- to 10-membered heteroaryl; and
R^{3a} and R^{3b} are each independently hydrogen, deuterium, halogen, amino, hydroxy, -CN, -C(O)NH₂, -C(O)NH₂, -C(O)O(C₁₋₁₀ alkyl), substituted or unsubstituted C₁₋₁₀ alkyl, substituted or unsubstituted 6- to 10-membered aryl, or substituted or unsubstituted 5- to 10-membered heteroaryl.

12. The method of detecting amyloid-beta (Aβ) of claim 11, wherein the isolated biological sample is at least one selected from the group consisting of blood, plasma, cerebrospinal fluid, cortical lysate, and hippocampal lysate.

13. A composition for diagnosing a neurodegenerative disease, the composition comprising a compound represented by Formula 1 or a pharmaceutically acceptable salt thereof: 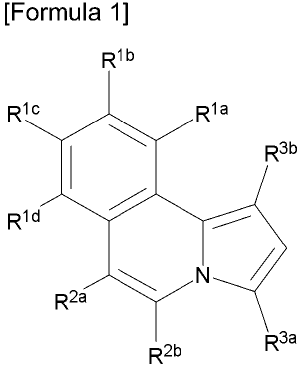 wherein, in Formula 1,
R^{1a} and R^{1b} are each independently hydrogen, deuterium, halogen, amino, hydroxy, substituted or unsubstituted C₁₋₁₀ alkyl, substituted or unsubstituted C₂₋₁₀ alkenyl, substituted or unsubstituted C₂₋₁₀ alkynyl, substituted or unsubstituted C₁₋₁₀ alkoxy, substituted or unsubstituted 6- to 10-membered aryl, or substituted or unsubstituted 5- to 10-membered heteroaryl;
R^{1c} and R^{1d} are each independently hydrogen, deuterium, halogen, amino, hydroxy, substituted or unsubstituted C₁₋₁₀ alkyl, substituted or unsubstituted C₂₋₁₀ alkenyl, substituted or unsubstituted C₂₋₁₀ alkynyl, substituted or unsubstituted C₁₋₁₀ alkoxy, substituted or unsubstituted 6- to 10-membered aryl, or substituted or unsubstituted 5- to 10-membered heteroaryl, or
R^{1c} and R^{1d} are connected to each other to form a compound represented by Formula 2; 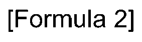 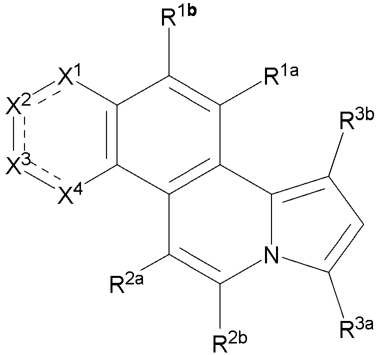
wherein, in Formula 2, X¹, X², X³, and X⁴ are each independently C(R⁴), nitrogen, oxygen, sulfur, or phosphorus;
R⁴ is hydrogen, deuterium, halogen, amino, hydroxy, substituted or unsubstituted C₁₋₁₀ alkyl, substituted or unsubstituted C₂₋₁₀ alkenyl, substituted or unsubstituted C₂₋₁₀ alkynyl, substituted or unsubstituted C₁₋₁₀ alkoxy, substituted or unsubstituted 6- to 10-membered aryl, or substituted or unsubstituted 5- to 10-membered heteroaryl;
R^{2a} and R^{2b} are each independently hydrogen, deuterium, halogen, amino, hydroxy, substituted or unsubstituted C₁₋₁₀ alkyl, substituted or unsubstituted C₂₋₁₀ alkenyl, substituted or unsubstituted C₂₋₁₀ alkynyl, substituted or unsubstituted C₁₋₁₀ alkoxy, substituted or unsubstituted 6- to 10-membered aryl, or substituted or unsubstituted 5- to 10-membered heteroaryl; and
R^{3a} and R^{3b} are each independently hydrogen, deuterium, halogen, amino, hydroxy, -CN, -C(O)NH₂, -C(O)NH₂, -C(O)O(C₁₋₁₀ alkyl), substituted or unsubstituted C₁₋₁₀ alkyl, substituted or unsubstituted 6- to 10-membered aryl, or substituted or unsubstituted 5- to 10-membered heteroaryl.

14. A method of providing information regarding diagnosis of a neurodegenerative disease, the method comprising contacting an isolated biological sample with a compound represented by Formula 1 or a pharmaceutically acceptable salt thereof: 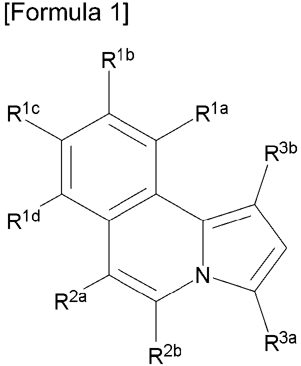 wherein, in Formula 1,
R^{1a} and R^{1b} are each independently hydrogen, deuterium, halogen, amino, hydroxy, substituted or unsubstituted C₁₋₁₀ alkyl, substituted or unsubstituted C₂₋₁₀ alkenyl, substituted or unsubstituted C₂₋₁₀ alkynyl, substituted or unsubstituted C₁₋₁₀ alkoxy, substituted or unsubstituted 6- to 10-membered aryl, or substituted or unsubstituted 5- to 10-membered heteroaryl;
R^{1c} and R^{1d} are each independently hydrogen, deuterium, halogen, amino, hydroxy, substituted or unsubstituted C₁₋₁₀ alkyl, substituted or unsubstituted C₂₋₁₀ alkenyl, substituted or unsubstituted C₂₋₁₀ alkynyl, substituted or unsubstituted C₁₋₁₀ alkoxy, substituted or unsubstituted 6- to 10-membered aryl, or substituted or unsubstituted 5- to 10-membered heteroaryl, or
R^{1c} and R^{1d} are connected to each other to form a compound represented by Formula 2; 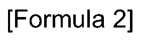 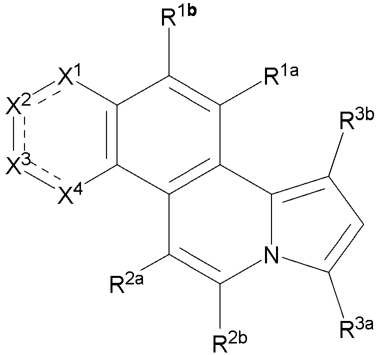
wherein, in Formula 2, X¹, X², X³, and X⁴ are each independently C(R⁴), nitrogen, oxygen, sulfur, or phosphorus;
R⁴ is hydrogen, deuterium, halogen, amino, hydroxy, substituted or unsubstituted C₁₋₁₀ alkyl, substituted or unsubstituted C₂₋₁₀ alkenyl, substituted or unsubstituted C₂₋₁₀ alkynyl, substituted or unsubstituted C₁₋₁₀ alkoxy, substituted or unsubstituted 6- to 10-membered aryl, or substituted or unsubstituted 5- to 10-membered heteroaryl;
R^{2a} and R^{2b} are each independently hydrogen, deuterium, halogen, amino, hydroxy, substituted or unsubstituted C₁₋₁₀ alkyl, substituted or unsubstituted C₂₋₁₀ alkenyl, substituted or unsubstituted C₂₋₁₀ alkynyl, substituted or unsubstituted C₁₋₁₀ alkoxy, substituted or unsubstituted 6- to 10-membered aryl, or substituted or unsubstituted 5- to 10-membered heteroaryl; and
R^{3a} and R^{3b} are each independently hydrogen, deuterium, halogen, amino, hydroxy, -CN, -C(O)NH₂, -C(O)NH₂, -C(O)O(C₁₋₁₀ alkyl), substituted or unsubstituted C₁₋₁₀ alkyl, substituted or unsubstituted 6- to 10-membered aryl, or substituted or unsubstituted 5- to 10-membered heteroaryl.
